(19) Europäisches Patentamt
European Patent Office
Office européen des brevets

(11) EP 4 603 546 A1

(12) EUROPEAN PATENT APPLICATION
published in accordance with Art. 153(4) EPC

(43) Date of publication:
**20.08.2025 Bulletin 2025/34**

(21) Application number: **23912278.1**

(22) Date of filing: **27.12.2023**

(51) International Patent Classification (IPC):
***C08L 101/00*** *(2006.01)* ***A61K 9/16*** *(2006.01)*
***A61K 38/08*** *(2019.01)* ***A61K 38/10*** *(2006.01)*
***A61K 38/12*** *(2006.01)* ***A61K 38/16*** *(2006.01)*
***C08L 89/00*** *(2006.01)*

(52) Cooperative Patent Classification (CPC):
**A61K 9/16; A61K 38/08; A61K 38/10; A61K 38/12; A61K 38/16; C08L 89/00; C08L 101/00**

(86) International application number:
**PCT/JP2023/047077**

(87) International publication number:
**WO 2024/143501 (04.07.2024 Gazette 2024/27)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **28.12.2022 JP 2022211372**

(71) Applicant: **CHUGAI SEIYAKU KABUSHIKI KAISHA**
**Tokyo 115-8543 (JP)**

(72) Inventors:
- **SHINKAI, Hiroki**
  **Tokyo 115-8543 (JP)**
- **KINOSHITA, Ryo**
  **Tokyo 115-8543 (JP)**

(74) Representative: **Vossius & Partner Patentanwälte Rechtsanwälte mbB**
**Siebertstrasse 3**
**81675 München (DE)**

# (54) SOLID DISPERSION CONTAINING SUBSTANCE TO BE DISPERSED, PHARMACEUTICAL COMPOSITION CONTAINING SAME, AND PRODUCTION METHODS THEREFOR

(57) The present invention relates to a solid dispersion comprising (1) a substance to be dispersed that has a molecular weight of 1,000 g/mol or more and 5,000 g/mol or less and is amorphous; and (2) a polymer, wherein a ratio of a total mass of the polymer to a total mass of the substance to be dispersed is less than 1.0, and the solid dispersion meets the following (I) or (II):
(I) a glass transition temperature of the substance to be dispersed and a glass transition temperature of the solid dispersion are different by 1°C or more;
(II) the glass transition temperature of the solid dispersion is 94.5% or more and 105.5% or less with respect to a value of Tga represented by the following formula (A);
[Expression 1]

$$Tga = \frac{Wc}{Wa} \times Tgc + \frac{Wp}{Wa} \times Tgp \quad \ldots \text{ Formula (A)}$$

in the formula (A), Tgc represents the glass transition temperature of the substance to be dispersed, Tgp represents the glass transition temperature of the polymer, Wa represents the total mass of the solid dispersion, Wc represents the total mass of the substance to be dispersed contained in the solid dispersion, and Wp represents the total mass of the polymer contained in the solid dispersion, respectively.

EP 4 603 546 A1

## Description

[Technical Field]

The present invention relates to a solid dispersion containing a substance to be dispersed, a pharmaceutical composition comprising the same, and production methods therefor.

[Background Art]

Solid dispersions containing a poorly water soluble compound and a polymeric compound (polymer) have been prepared to improve the solubility of a drug (Patent Literatures 1 to 5, and Non Patent Literature 1).

[Citation List]

[Patent Literature]

[Patent Literature 1] International Publication No. WO 2010/126030
[Patent Literature 2] International Publication No. WO 2014/092061
[Patent Literature 3] International Publication No. WO 2015/189901
[Patent Literature 4] International Publication No. WO 2008/047201
[Patent Literature 5] U.S. Patent No. 10004719

[Non Patent Literature]

[Non Patent Literature 1] International Journal of Pharmaceutics, 2010, vol. 399, pp. 94-101

[Summary of Invention]

[Technical Problem]

Conventionally, when preparing a solid dispersion containing a poorly water soluble compound (active ingredient), a certain amount of the polymer is formulated in the solid dispersion to ensure sufficient amorphous stability of the poorly water soluble compound. However, there has been the following problem: the higher the content of polymer, the longer the disintegration times of the solid dispersion and the pharmaceutical composition comprising the solid dispersion.
An object of the present invention is to provide a solid dispersion having excellent amorphous stability while reducing the content of polymer. Another object of the present invention is to provide a pharmaceutical composition comprising the solid dispersion.

[Solution to Problem]

The present invention relates to, for example, each of the following inventions.

[1] A solid dispersion comprising: (1) a substance to be dispersed that has a molecular weight of 1,000 g/mol or more and 5,000 g/mol or less and is amorphous; and (2) a polymer,
wherein a ratio of a total mass of the polymer to a total mass of the substance to be dispersed is less than 1.0, and the solid dispersion meets the following (I) or (II):

(I) a glass transition temperature of the substance to be dispersed and a glass transition temperature of the solid dispersion are different by 1°C or more;
(II) the glass transition temperature of the solid dispersion is 94.5% or more and 105.5% or less with respect to a value of Tga represented by the following formula (A);
[Expression 1]

$$Tga = \frac{Wc}{Wa} \times Tgc + \frac{Wp}{Wa} \times Tgp \quad \dots \text{ Formula (A)}$$

in the formula (A), Tgc represents the glass transition temperature of the substance to be dispersed, Tgp represents a glass transition temperature of the polymer, Wa represents the total mass of the solid dispersion, Wc represents the total mass of the substance to be dispersed contained in the solid dispersion, and Wp represents the total mass of the polymer contained in the solid dispersion, respectively.

[2] The solid dispersion according to [1], which meets the (I).
[3] The solid dispersion according to [1], which meets the (II).
[4] The solid dispersion according to [1], which meets the (I) and (II).
[5] The solid dispersion according to [1], wherein the glass transition temperature of the solid dispersion is 100°C or higher and 200°C or lower.
[6] The solid dispersion according to any one of [1] to [5], wherein the glass transition temperature of the substance to be dispersed exceeds the glass transition temperature of the polymer.
[7] The solid dispersion according to [6], wherein the glass transition temperature of the substance to be dispersed is 1°C or more higher than the glass transition temperature of the polymer.
[8] The solid dispersion according to any one of [1] to [7], wherein the glass transition temperature of the substance to be dispersed is 1°C or more higher than the glass transition temperature of the solid dispersion.
[9] The solid dispersion according to any one of [1] to [8], wherein the glass transition temperature of the substance to be dispersed is 90°C or higher and 180°C or lower.
[10] The solid dispersion according to any one of [1] to [9], wherein the substance to be dispersed is a peptide compound.
[11] The solid dispersion according to [10], wherein the number of amino acid residues constituting the peptide compound is 5 or more and 30 or less.
[12] The solid dispersion according to [10] or [11], wherein the number of non-natural amino acid residues contained in the peptide compound is 1 or more.
[13] The solid dispersion according to any one of [10] to [12], wherein the number of N-substituted amino acid residues contained in the peptide compound is 1 or more.
[14] The solid dispersion according to any one of [10] to [13], wherein the number of N-substituted amino acid residues contained in the peptide compound is 5 or more.
[15] The solid dispersion according to [13] or [14], wherein a substituent on a nitrogen atom of the N-substituted amino acid is a $C_1$-$C_6$ alkyl group.
[16] The solid dispersion according to any one of [10] to [15], wherein ClogP of the peptide compound is 4 or more and 25 or less.
[17] The solid dispersion according to any one of [10] to [16], wherein a ClogP/number of amino acid residues of the peptide compound is 1.0 or more.
[18] The solid dispersion according to any one of [10] to [17], wherein the peptide compound has a cyclic moiety.
[19] The solid dispersion according to [18], wherein the number of amino acid residues contained in the cyclic moiety is 5 or more and 15 or less.
[20] The solid dispersion according to any one of [1] to [19], wherein the glass transition temperature of the polymer is 30°C or higher and 170°C or lower.
[21] The solid dispersion according to any one of [1] to [20], wherein the polymer is a pharmaceutically acceptable polymer.
[22] The solid dispersion according to any one of [1] to [21], wherein a weight average molecular weight of the polymer is 3,000 or more and 400,000 or less.
[23] The solid dispersion according to any one of [1] to [22], wherein the polymer is at least one selected from the group consisting of a non-ionic polymer and an ionic polymer.
[24] The solid dispersion according to any one of [1] to [23], wherein the polymer is an ionic polymer.
[25] The solid dispersion according to any one of [1] to [24], wherein the polymer is an acidic polymer.
[26] The solid dispersion according to any one of [1] to [24], wherein the polymer is a basic polymer.
[27] The solid dispersion according to any one of [1] to [23], wherein the polymer is a non-ionic polymer.
[28] The solid dispersion according to [27], wherein the non-ionic polymer is a neutral non-cellulosic polymer.
[29] The solid dispersion according to [28], wherein the neutral non-cellulosic polymer is at least one selected from the group consisting of (i), (ii), (iii), and (iv):

(i) a vinyl polymer or copolymer having at least one substituent selected from the group consisting of hydroxyl, alkylacyloxy, and cyclic amide;
(ii) a vinyl copolymer having at least one hydrophilic hydroxyl-containing repeat unit and at least one hydrophobic alkyl or aryl-containing repeat unit, or a polyvinyl alcohol;
(iii) a polyvinyl alcohol having at least a portion of a repeat unit in a non-hydrolyzed (vinyl acetate) form, a polyvinyl

alcohol-polyvinyl acetate copolymer, a polyvinyl pyrrolidone, a copovidone, a copolymer of acrylate and methacrylic acid, or a polyethylene polyvinyl alcohol copolymer; and
(iv) a polyoxyethylene-polyoxypropylene block copolymer.

[30] The solid dispersion according to [27], wherein the non-ionic polymer is a neutral cellulosic polymer.
[31] The solid dispersion according to [30], wherein the neutral cellulosic polymer is at least one selected from the group consisting of hydroxypropyl methyl cellulose acetate (HPMCA), hydroxypropyl methyl cellulose (HPMC), hydroxypropyl cellulose (HPC), methyl cellulose, hydroxyethyl methyl cellulose, hydroxyethyl cellulose, hydroxyethyl cellulose acetate, and hydroxyethyl ethyl cellulose.
[32] The solid dispersion according to [27], wherein the non-ionic polymer is a neutralized acidic polymer.
[33] The solid dispersion according to [32], wherein the neutralized acidic polymer is at least one selected from the group consisting of cellulose acetate phthalate, cellulose acetate trimellitate, cellulose acetate succinate, methyl cellulose phthalate, hydroxymethyl cellulose ethyl phthalate, hydroxypropyl methyl cellulose phthalate, hydroxypropyl methyl cellulose acetate succinate (hypromellose acetate succinate: HPMCAS), hydroxypropyl methyl acetate maleate, hydroxypropyl methyl trimellitate, carboxymethyl ethyl cellulose, polyvinyl butyrate phthalate, polyvinyl alcohol acetate phthalate, a methacrylic acid/ethyl acrylate copolymer, a methacrylic acid/methyl methacrylate copolymer, and a methacrylic acid copolymer.
[34] The solid dispersion according to [24], wherein the ionic polymer is at least one selected from the group consisting of an acidic polymer and a basic polymer.
[35] The solid dispersion according to [25] or [34], wherein the acidic polymer is at least one selected from the group consisting of cellulose acetate phthalate, cellulose acetate trimellitate, cellulose acetate succinate, methyl cellulose phthalate, hydroxymethyl cellulose ethyl phthalate, hydroxypropyl methyl cellulose phthalate (HPMCP), hydroxypropyl methyl cellulose acetate succinate (HPMCAS), hydroxypropyl methyl acetate maleate, hydroxypropyl methyl trimellitate, carboxymethyl ethyl cellulose, polyvinyl butyrate phthalate, polyvinyl alcohol acetate phthalate, a methacrylic acid/ethyl acrylate copolymer, a methacrylic acid/methyl methacrylate copolymer, and a methacrylic acid copolymer.
[36] The solid dispersion according to [26] or [34], wherein the basic polymer is at least one selected from the group consisting of amino alkyl methacrylic acid copolymer E and polyvinyl acetal diethylaminoacetate.
[37] The solid dispersion according to any one of [1] to [22], wherein the polymer is at least one selected from the group consisting of a polymer having a hydroxypropyl cellulose backbone, a polymer having a povidone backbone, and a block copolymer polymer.
[38] The solid dispersion according to [37], wherein the polymer is at least one selected from the group consisting of hydroxypropyl methyl cellulose acetate succinate (HPMCAS), hydroxypropyl methyl cellulose (HPMC), polyvinyl pyrrolidone, a vinyl pyrrolidone/vinyl acetate copolymer, a methacrylic acid/methyl methacrylate copolymer, and a methacrylic acid/ethyl acrylate copolymer.
[39] The solid dispersion according to any one of [1] to [38], wherein a bulk density calculated from a volume of powder obtained by gently filling 0.5 g of the solid dispersion into a 5 mL measuring cylinder is 0.15 g/mL or more.
[40] The solid dispersion according to any one of [1] to [39], wherein, in a physical stability test under conditions of 40°C, 75%RH, a crystal is not observed until one month after starting the test.
[41] The solid dispersion according to any one of [1] to [40], wherein, in a physical stability test under conditions of 80°C, 75%RH, a crystal is not observed until one month after starting the test.
[42] The solid dispersion according to any one of [1] to [41], wherein a disintegration time in a disintegration test under conditions of using a second fluid for dissolution test as a test solution, a test solution temperature of 37.0°C and an amplitude-frequency of 30 times/minute is 1,000 seconds or less.
[43] The solid dispersion according to any one of [1] to [42], wherein a content of the substance to be dispersed in the solid dispersion is more than 50% by mass and less than 100% by mass based on a total amount of the solid dispersion.
[44] A pharmaceutical formulation comprising the solid dispersion according to any one of [1] to [43].
[45] The pharmaceutical composition according to [44], which is a molded solid.
[46] The pharmaceutical composition according to [44] or [45], wherein a content of the solid dispersion is 99% by mass or less based on a total amount of the pharmaceutical composition.
[47] The pharmaceutical composition according to any one of [44] to [46], wherein a solubility in water at pH 6.5, pressure of 1 atm, and temperature of 25°C is 10 mg/mL or less.
[48] The pharmaceutical composition according to any one of [44] to [47], wherein, in a physical stability test under conditions of 40°C, 75%RH, a crystal is not observed until one month after starting the test.
[49] The pharmaceutical composition according to any one of [44] to [48], wherein, in a physical stability test under conditions of 80°C, 75%RH, a crystal is not observed until one month after starting the test.
[50] The pharmaceutical composition according to any one of [44] to [49], wherein a disintegration time in a

disintegration test under conditions of using a second fluid for dissolution test as a test solution, a test solution temperature of 37.0°C and an amplitude-frequency of 30 times/minute is 1,000 seconds or less.
[51] The pharmaceutical composition according to any one of [44] to [50], further comprising a surfactant.
[52] The pharmaceutical composition according to [51], wherein the surfactant is a pharmaceutically acceptable surfactant.
[53] The pharmaceutical composition according to [51] or [52], wherein the surfactant has a linear hydrocarbon group.
[54] The pharmaceutical composition according to any one of [51] to [53], wherein the number of carbon atoms contained in the linear hydrocarbon group is 5 or more and 17 or less.
[55] The pharmaceutical composition according to [51] or [52], wherein the surfactant is represented by any one of the following general formulae (a1) to (a3):

[Formula 1]

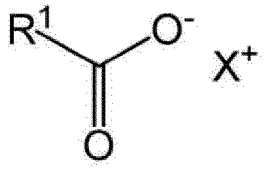

(a 1)

[Formula 2]

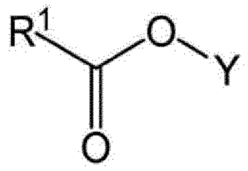

(a 2)

[Formula 3]

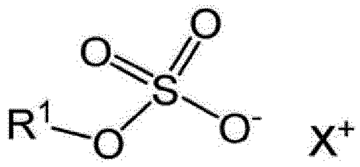

(a 3)

in the general formulae (a1) to (a3), $R^1$ represents an optionally substituted, saturated or unsaturated, linear hydrocarbon group having 5 or more and 17 or less carbon atoms, X represents sodium or potassium, and Y represents a group represented by the following formula (a4) or a stereoisomer thereof:

[Formula 4]

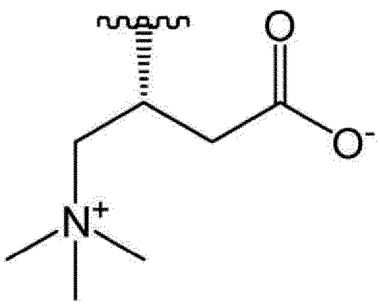

(a 4)

in the formula (a4),

[Formula 5]

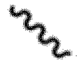

represents a bond.

[56] The pharmaceutical composition according to [51] or [52], wherein the surfactant is represented by the following general formula (a3):

[Formula 6]

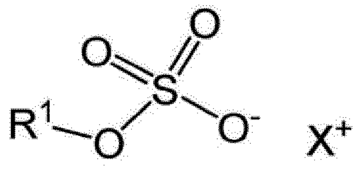

(a3)

in the general formula (a3), $R^1$ represents an optionally substituted, saturated or unsaturated, linear hydrocarbon group having 5 or more and 17 or less carbon atoms, and X represents sodium or potassium.

[57] The pharmaceutical composition according to [51] or [52], wherein the surfactant is sodium dodecyl sulfate.

[58] The pharmaceutical composition according to any one of [44] to [57], further comprising an excipient.

[59] The pharmaceutical composition according to any one of [44] to [58], further comprising a disintegrant.

[60] A method for producing a solid dispersion, comprising a step of evaporating a solvent in a mixture containing (1) a substance to be dispersed that has a molecular weight of 1,000 g/mol or more and 5,000 g/mol or less and is amorphous and (2) a polymer in an amount such that a ratio of a total mass of the polymer to a total mass of the substance to be dispersed is less than 1.0 by spray drying.

[61] The production method according to [60], wherein the solvent is capable of dissolving the substance to be dispersed and the polymer.

[62] The production method according to [60] or [61], wherein the solvent is one or more selected from the group consisting of water, an alcohol, a ketone, an ester, acetonitrile, methylene chloride, toluene, 1,1,1-trichloroethane, and tetrahydrofuran.

[63] The production method according to any one of [60] to [62], wherein the solvent is an alcohol.

[64] The production method according to any one of [60] to [62], wherein the solvent is a ketone.

[65] The production method according to any one of [60] to [62], wherein the solvent is an ester.

[66] The production method according to [62] or [63], wherein the alcohol is one or more selected from the group consisting of methanol, ethanol, n-propanol, isopropanol, and butanol.

[67] The production method according to [62] or [64], wherein the ketone is one or more selected from the group consisting of acetone, methyl ethyl ketone, and methyl isobutyl ketone.

[68] The production method according to [62] or [65], wherein the ester is one or more selected from the group consisting of ethyl acetate and propyl acetate.

[69] The production method according to any one of [60] to [68], wherein the solvent is a single solvent.

[70] The production method according to any one of [60] to [69], wherein the solvent is ethanol, acetone, or tetrahydrofuran.

[71] The production method according to any one of [60] to [70], wherein the spray drying is performed under the conditions of air volume of 100 L/h or more and 800 L/h or less, temperature of 40°C or higher and 120°C or lower, and 1 atm.

[72] The production method according to any one of [10] to [71], wherein the peptide compound is represented by the following structural formula (1):

[Formula 7]

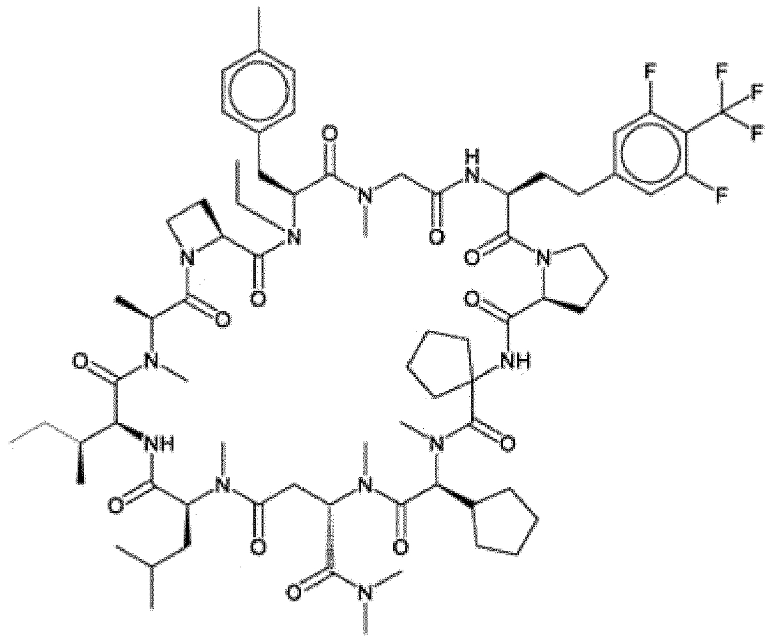

(1)

[Advantageous Effects of Invention]

According to the present invention, it is possible to provide a solid dispersion having excellent amorphous stability while reducing the content of polymer. According to the present invention, it is possible to provide a pharmaceutical composition comprising the solid dispersion.

[Brief Description of Drawings]

[Figure 1] Figure 1 is a graph showing XRD measurement results for the solid dispersion of Example I-A-1.
[Figure 2] Figure 2 is a graph showing XRD measurement results for the solid dispersion of Example I-A-4.
[Figure 3] Figure 3 is a graph showing XRD measurement results for the solid dispersion of Comparative Example I-A-ii.
[Figure 4] Figure 4 is a graph showing XRD measurement results for the solid dispersion of Example V-G-4.
[Figure 5] Figure 5 is a graph showing XRD measurement results for the solid dispersion of Example V-J-4.
[Figure 6] Figure 6 is a graph showing XRD measurement results for the solid dispersion of Comparative Example V-G-4.
[Figure 7] Figure 7 is a graph showing XRD measurement results for the solid dispersion of Comparative Example V-J-4.
[Figure 8] Figure 8 is a graph showing DSC measurement results for the solid dispersion of Example I-A-1.
[Figure 9] Figure 9 is a graph showing DSC measurement results for the solid dispersion of Example I-A-2.
[Figure 10] Figure 10 is a graph showing DSC measurement results for the solid dispersion of Example I-A-3.
[Figure 11] Figure 11 is a graph showing DSC measurement results for the solid dispersion of Example I-A-4.
[Figure 12] Figure 12 is a graph showing DSC measurement results for the solid dispersion of Example I-A-5.
[Figure 13] Figure 13 is a graph showing DSC measurement results for the solid dispersion of Example I-A-6.
[Figure 14] Figure 14 is a graph showing DSC measurement results for the solid dispersion of Comparative Example I-A-i.
[Figure 15] Figure 15 is a graph showing DSC measurement results for the solid dispersion of Comparative Example I-A-ii.
[Figure 16] Figure 16 is a graph showing DSC measurement results for the solid dispersion of Example I-B-4.
[Figure 17] Figure 17 is a graph showing DSC measurement results for the solid dispersion of Example I-C-4.
[Figure 18] Figure 18 is a graph showing DSC measurement results for the solid dispersion of Example I-D-4.
[Figure 19] Figure 19 is a graph showing DSC measurement results for the solid dispersion of Example I-E-4.

[Figure 20] Figure 20 is a graph showing DSC measurement results for the solid dispersion of Example I-F-4.
[Figure 21] Figure 21 is a graph showing DSC measurement results for the solid dispersion of Example I-G-4.
[Figure 22] Figure 22 is a graph showing DSC measurement results for the solid dispersion of Example I-H-4.
[Figure 23] Figure 23 is a graph showing DSC measurement results for the solid dispersion of Example I-I-4.
[Figure 24] Figure 24 is a graph showing DSC measurement results for the solid dispersion of Example I-J-4.
[Figure 25] Figure 25 is a graph showing DSC measurement results for the solid dispersion of Example I-L-4.
[Figure 26] Figure 26 is a graph showing DSC measurement results for the solid dispersion of Example V-G-4.
[Figure 27] Figure 27 is a graph showing DSC measurement results for the solid dispersion of Example V-J-4.
[Figure 28] Figure 28 is a graph showing DSC measurement results for the solid dispersion of Comparative Example V-G-4.
[Figure 29] Figure 29 is a graph showing DSC measurement results for the solid dispersion of Comparative Example V-J-4.
[Figure 30] Figure 30 is a graph showing DSC measurement results for the solid dispersion of Example I-K-4.
[Figure 31] Figure 31 is a graph showing DSC measurement results for the solid dispersion of Example V-A-4.
[Figure 32] Figure 32 is a graph showing DSC measurement results for the solid dispersion of Example VI-A-4.

[Description of Embodiments]

Hereinafter, the embodiments for carrying out the present invention are described in detail. However, the present invention is not limited by the embodiments given below.
The term "one or more" as used herein means the number of 1 or 2 or larger. When the term "one or more" is used in the context associated with a substituent of a group, the term means a number from 1 to the maximum number of substituents acceptable by the group. Specific examples of the term "one or more" include 1, 2, 3, 4, 5, 6, 7, 8, 9, 10, and/or larger numbers.
As used herein, the term "to" that indicates a range includes values of both ends thereof. For example, "A to B" means the range of A or more and B or less.
The term "about" as used herein, when used in combination with a numeric value, means the value range of + 10% and -10% of the numeric value.
In the present invention, the meaning of the term "and/or" includes any combination in which "and" and "or" are appropriately combined. Specifically, for example, the term "A, B, and/or C" includes the following seven variations: (i) A, (ii) B, (iii) C, (iv) A and B, (v) A and C, (vi) B and C, and (vii) A, B, and C.
The solid dispersion according to the present embodiments comprises (1) a substance to be dispersed that has a molecular weight of 1,000 g/mol or more and 5,000 g/mol or less and is amorphous; and (2) a polymer, wherein a ratio of a total mass of the polymer to a total mass of the substance to be dispersed is less than 1.0, and the solid dispersion meets the following (I) or (II):

(I) a glass transition temperature of the substance to be dispersed and a glass transition temperature of the solid dispersion are different;
(II) the glass transition temperature of the solid dispersion is 94.5% or more and 105.5% or less with respect to a value of Tga represented by the following formula (A);
[Expression 2]

$$Tga = \frac{Wc}{Wa} \times Tgc + \frac{Wp}{Wa} \times Tgp \quad \ldots \text{ Formula (A)}$$

in the formula (A), Tgc represents the glass transition temperature of the substance to be dispersed, Tgp represents the glass transition temperature of the polymer, Wa represents the total mass of the solid dispersion, Wc represents the total mass of the substance to be dispersed contained in the solid dispersion, and Wp represents the total mass of the polymer contained in the solid dispersion, respectively.

(Substance to be dispersed)

The molecular weight of the substance to be dispersed according to the present embodiments is 1,000 g/mol or more and 5,000 g/mol or less. The molecular weight as used herein means the sum of the atomic weights of the atoms constituting the compound molecule (unit: "g/mol"), and is obtained by calculating the sum of the atomic weights of the atoms included in the molecular structural formula (unit: "g/mol"). The unit of molecular weight is sometimes omitted herein. The molecular weight can be measured, for example, by liquid chromatography-mass spectrometry (LC/MS).
The molecular weight of the substance to be dispersed according to the present embodiments may be, for

example, 1,100 g/mol or more, 1,200 g/mol or more, 1,300 g/mol or more, or 1,400 g/mol or more. The molecular weight of the substance to be dispersed according to the present embodiments may be, for example, 4,000 g/mol or less, 3,000 g/mol or less, 2,500 g/mol or less, 2,000 g/mol or less, 1,800 g/mol or less, 1,600 g/mol or less, or 1,500 g/mol or less. Examples of the range of the molecular weight of the substance to be dispersed according to the present embodiments include 1,000 g/mol or more and 4,000 g/mol or less, 1,000 g/mol or more and 3,000 g/mol or less, 1,100 g/mol or more and 2,500 g/mol or less, 1,200 g/mol or more and 2,000 g/mol or less, 1,300 g/mol or more and 1,800 g/mol or less, 1,400 g/mol or more and 1,600 g/mol or less, or 1,400 g/mol or more and 1,500 g/mol or less. The molecular weight of the substance to be dispersed according to the present embodiments is, for example, 1,000 g/mol to 5,000 g/mol, preferably 1,200 g/mol to 2,000 g/mol or less, more preferably 1,300 g/mol to 1,800 g/mol, and most preferably 1,400 g/mol to 1,600 g/mol. When the molecular weight is in the above range, the substance to be dispersed is difficult to crystallize and has excellent amorphous stability.

The substance to be dispersed according to the present embodiments is present as amorphous in the solid dispersion according to the present embodiments. The "amorphous" as used herein refers to a substance that has no long-range order. It can be confirmed that the substance to be dispersed is amorphous, for example, by performing the X-ray diffraction (XRD) measurement described in Examples below on the solid dispersion, and checking the XRD spectrum showing a broad diffraction pattern or a halo pattern without diffraction peaks, in other words, no sharp diffraction pattern.

The "amorphous stability" as used herein refers to the length of time in which a substance to be dispersed present in a solid dispersion can remain in the amorphous state described above. Amorphous stability can be measured by storing the solid dispersion under certain conditions, such as the conditions of 40°C, 75%RH, the conditions of 80°C, 75%RH, or the conditions of 40°C, 85%RH, and then examining whether the substance to be dispersed in the solid dispersion remains in the amorphous state. Specifically, amorphous stability can be measured by the physical stability test described in Examples. Since the physical stability of a pharmaceutical composition is governed by the physical stability of a solid dispersion contained in the pharmaceutical composition, the physical stability of the solid dispersion contained in the pharmaceutical composition can be measured by subjecting the pharmaceutical composition to the above-described physical stability test. Conversely, it is possible to know the physical stability of a pharmaceutical composition containing a solid dispersion by measuring the physical stability of the solid dispersion.

The glass transition temperature of the substance to be dispersed (Tgc) according to the present embodiments may be, for example, 90°C or higher, 100°C or higher, 110°C or higher, 120°C or higher, 130°C or higher, 135°C or higher, or 140°C or higher. The glass transition temperature of the substance to be dispersed (Tgc) according to the present embodiments may be, for example, 180°C or lower, 175°C or lower, 170°C or lower, 165°C or lower, or 160°C or lower. Examples of the range of the glass transition temperature of the substance to be dispersed (Tgc) according to the present embodiments include 90°C or higher and 180°C or lower, 100°C or higher and 175°C or lower, 110°C or higher and 170°C or lower, 120°C or higher and 165°C or lower, 130°C or higher and 160°C or lower, 135°C or higher and 160°C or lower, or 140°C or higher and 160°C or lower. The glass transition temperature of the substance to be dispersed (Tgc) according to the present embodiments is, for example, 90°C to 180°C, preferably 100°C to 175°C, more preferably 120°C to 170°C, and most preferably 130°C to 165°C. When the glass transition temperature is in the above range, the substance to be dispersed is difficult to crystallize and has excellent amorphous stability. The glass transition temperature (°C) as used herein means a value measured by differential scanning calorimetry (DSC) as described in Examples below. Specifically, the glass transition temperature is the temperature corresponding to a point where the value obtained by primary differentiation of the Rev Heat Flow [mW] curve obtained by differential scanning calorimetry with temperatures [°C] becomes the smallest. It should be noted that when finding the smallest value among the values obtained by primary differentiation, the point at which the value becomes the smallest is determined after noises near the start of DSC measurement are excluded.

The substance to be dispersed according to the present embodiments may have a solubility of 10 mg/mL or less and more than 0 mg/mL in a 50 mM phosphate buffer solution (pH 6.5) at 25°C, 1 atm. The substance to be dispersed according to the present embodiments may have a solubility of, for example, 5 mg/mL or less, 2.5 mg/mL or less, 2 mg/mL or less, 1 mg/mL or less, 0.5 mg/mL or less, 0.25 mg/mL or less, 0.1 mg/mL or less, 0.05 mg/mL or less, 0.025 mg/mL or less, 0.01 mg/mL or less, 0.005 mg/mL or less, 0.0025 mg/mL or less, or 0.001 mg/mL or less in a 50 mM phosphate buffer solution (pH 6.5) at 25°C, 1 atm. The solubility may be 0.0001 mg/mL or more, 0.0005 mg/mL or more, or 0.001 mg/mL or more. The substance to be dispersed according to the present embodiments has a solubility of, for example, more than 0 mg/mL and 10 mg/mL or less, preferably more than 0 mg/mL and 1 mg/mL or less, more preferably more than 0 mg/mL and 0.1 mg/mL or less, and most preferably more than 0 mg/mL and 0.05 mg/mL or less in a 50 mM phosphate buffer solution (pH 6.5) at 25°C, 1 atm. The solubility of the substance to be dispersed in a 50 mM phosphate buffer solution (pH 6.5) at 25°C, 1 atm can be measured, for example, by the method described in Examples below.

The substance to be dispersed according to the present embodiments may be a compound classified into class II or class IV by the Biopharmaceutics Classification System (BCS). The BCS is a guideline for predicting the digestive absorption characteristics of a pharmaceutical by classifying the pharmaceutical into four classes (classes I to IV) based on its solubility and absorption rate.

The solubility in the BCS ($D_0$: Dose Number) is determined with $D_0 = 1$ as the boundary, and the solubility is determined to be high (high solubility) when $D_0 \leq 1$ and determined to be low (low solubility) when $D_0 \geq 1$. The absorption rate in the BCS ($F_a$: a rate of absorption from the digestive tract) is determined with an absorption rate of 90% as the boundary, and the absorption rate is determined to be low (low absorption rate) when $F_a \leq 0.9$, and determined to be high (high absorption rate) when $F_a \geq 0.9$. Classes I to IV of the BCS are defined as follows:

Class I: high solubility ($D_0 \leq 1$) and high absorption rate ($F_a \geq 0.9$);
Class II: low solubility ($D_0 \geq 1$) and high absorption rate ($F_a \geq 0.9$);
Class III: high solubility ($D_0 \leq 1$) and low absorption rate ($F_a \leq 0.9$); and
Class IV: low solubility ($D_0 \geq 1$) and low absorption rate ($F_a \leq 0.9$).

The substance to be dispersed according to the present embodiments may be a peptide compound. As used herein, the term "peptide compound" is not particularly limited as long as amino acid residues in the peptide compound are linked by an amide bond or an ester bond. The number of amino acid residues constituting the peptide compound may be, for example, 5 or more, 7 or more, 8 or more, 9 or more, or 10 or more. The number of amino acid residues constituting the peptide compound may be, for example, 30 or less, 25 or less, 20 or less, 15 or less, 14 or less, 13 or less, or 12 or less. The number of amino acid residues constituting the peptide compound may be, for example, 5 or more and 30 or less, 7 or more and 25 or less, 8 or more and 15 or less, 9 or more and 14 or less, 9 or more and 13 or less, or 11. The number of amino acid residues constituting the peptide compound is, for example, 5 to 30, preferably 9 to 15, more preferably 10 to 14, and most preferably 11 to 13. The peptide compound may have a branched structure.

The term "amino acid" as used herein includes a natural amino acid and a non-natural amino acid. Also, as used herein, the term "amino acid residue" includes a natural amino acid residue and a non-natural amino acid residue.

Natural amino acids refer to glycine (Gly), alanine (Ala), serine (Ser), threonine (Thr), valine (Val), leucine (Leu), isoleucine (Ile), phenylalanine (Phe), tyrosine (Tyr), tryptophan (Trp), histidine (His), glutamic acid (Glu), aspartic acid (Asp), glutamine (Gln), asparagine (Asn), cysteine (Cys), methionine (Met), lysine (Lys), arginine (Arg), and proline (Pro).

Examples of the non-natural amino acid include, but are not particularly limited to, β-amino acids, D-amino acids, N-substituted amino acids (excluding Pro), α,α-disubstituted amino acids, amino acids having a side chain different from that of a natural amino acid, and hydroxycarboxylic acids. As used herein, non-natural N-substituted amino acids mean N-substituted amino acids other than Pro.

For the amino acid as used herein, any steric configuration is acceptable. The selection of a side chain of the amino acid is not particularly restricted, and the side chain is freely selected from, in addition to a hydrogen atom, an alkyl group, an alkenyl group, an alkynyl group, an aryl group, a heteroaryl group, an aralkyl group, a heteroaralkyl group, a cycloalkyl group, and a spiro-bonded cycloalkyl group. Each of the side chains may have a substituent. The substituent is also not limited, and one or two or more substituents may be freely selected independently from any substituents including, for example, a halogen atom, an O atom, an S atom, an N atom, a B atom, a Si atom, or a P atom. That is, examples of the side chain include an alkyl group, an alkoxy group, an alkenyl group, an alkynyl group, an aryl group, a heteroaryl group, an aralkyl group, or a cycloalkyl group which may be substituted, oxo, aminocarbonyl, and a halogen atom. The amino acid according to one embodiment may be a compound having a carboxy group and an amino group in the same molecule, and even in this case, imino acids such as proline and hydroxyproline are also included in the amino acid.

The term "alkyl" as used herein is a monovalent group induced by the removal of any one hydrogen atom from aliphatic hydrocarbon, and has a subset of a hydrocarbyl or hydrocarbon group structure containing hydrogen and carbon atoms without containing a heteroatom (which refers to an atom other than carbon and hydrogen atoms) or an unsaturated carbon-carbon bond in the backbone. The alkyl includes not only a linear form but also a branched form. Preferred examples of the alkyl include alkyl having 1 to 20 carbon atoms ($C_1$-$C_{20}$; hereinafter, "$C_p$-$C_q$" means that the number of carbon atoms is p to q), preferably $C_1$-$C_{10}$ alkyl, and more preferably $C_1$-$C_6$ alkyl. Specific examples of the alkyl include methyl, ethyl, n-propyl, i-propyl, n-butyl, s-butyl, t-butyl, isobutyl (2-methylpropyl), n-pentyl, s-pentyl (1-methylbutyl), t-pentyl (1,1-dimethylpropyl), neopentyl (2,2-dimethylpropyl), isopentyl (3-methylbutyl), 3-pentyl (1-ethylpropyl), 1,2-dimethylpropyl, 2-methylbutyl, n-hexyl, 1,1,2-trimethylpropyl, 1,2,2-trimethylpropyl, 1,1,2,2-tetramethylpropyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, and 2-ethylbutyl.

Examples of halogen-derived substituents include fluoro (-F), chloro (-Cl), bromo (-Br), and iodo (-I).

Examples of O-atom-derived substituents include hydroxy (-OH), oxy (-OR), carbonyl (-C(=O)-R), carboxy ($-CO_2H$), oxycarbonyl(-C(=O)-OR), carbonyloxy (-O-C(=O)-R), thiocarbonyl (-C(=O)-SR), a carbonylthio group (-S-C(=O)-R), aminocarbonyl (-C(=O)-NHR), carbonylamino (-NH-C(=O)-R), oxycarbonylamino (-NH-C(=O)-OR), sulfonylamino ($-NH-SO_2-R$), aminosulfonyl ($-SO_2-NHR$), sulfamoylamino ($-NH-SO_2-NHR$), thiocarboxy (-C(=O)-SH), and carboxycarbonyl ($-C(=O)-CO_2H$).

Examples of oxy (-OR) include alkoxy, cycloalkoxy, alkenyloxy, alkynyloxy, aryloxy, heteroaryloxy, and aralkyloxy.

Examples of carbonyl (-C(=O)-R) include formyl (-C(=O)-H), alkylcarbonyl, cycloalkylcarbonyl, alkenylcarbonyl, alkynylcarbonyl, arylcarbonyl, heteroarylcarbonyl, and aralkylcarbonyl.

Examples of oxycarbonyl (-C(=O)-OR) include alkyloxycarbonyl, cycloalkyloxycarbonyl, alkenyloxycarbonyl, alkynyloxycarbonyl, aryloxycarbonyl, heteroaryloxycarbonyl, and aralkyloxycarbonyl.

Examples of carbonyloxy (-O-C(=O)-R) include alkylcarbonyloxy, cycloalkylcarbonyloxy, alkenylcarbonyloxy, alkynylcarbonyloxy, arylcarbonyloxy, heteroarylcarbonyloxy, and aralkylcarbonyloxy.

Examples of thiocarbonyl (-C(=O)-SR) include alkylthiocarbonyl, cycloalkylthiocarbonyl, alkenylthiocarbonyl, alkynylthiocarbonyl, arylthiocarbonyl, heteroarylthiocarbonyl, and aralkylthiocarbonyl.

Examples of carbonylthio (-S-C(=O)-R) include alkylcarbonylthio, cycloalkylcarbonylthio, alkenylcarbonylthio, alkynylcarbonylthio, arylcarbonylthio, heteroarylcarbonylthio, and aralkylcarbonylthio.

Examples of aminocarbonyl (-C(=O)-NHR) include alkylaminocarbonyl, cycloalkylaminocarbonyl, alkenylaminocarbonyl, alkynylaminocarbonyl, arylaminocarbonyl, heteroarylaminocarbonyl, and aralkylaminocarbonyl. In addition to these, examples include compounds in which an H atom bonded to the N atom in -C(=O)-NHR is further replaced with alkyl, cycloalkyl, alkenyl, alkynyl, aryl, heteroaryl, or aralkyl.

Examples of carbonylamino (-NH-C(=O)-R) include alkylcarbonylamino, cycloalkylcarbonylamino, alkenylcarbonylamino, alkynylcarbonylamino, arylcarbonylamino, heteroarylcarbonylamino, and aralkylcarbonylamino. In addition to these, examples include compounds in which an H atom bonded to the N atom in -NH-C(=O)-R is further replaced with alkyl, cycloalkyl, alkenyl, alkynyl, aryl, heteroaryl, or aralkyl.

Examples of oxycarbonylamino (-NH-C(=O)-OR) include alkoxycarbonylamino, cycloalkoxycarbonylamino, alkenyloxycarbonylamino, alkynyloxycarbonylamino, aryloxycarbonylamino, heteroaryloxycarbonylamino, and aralkyloxycarbonylamino. In addition to these, examples include compounds in which an H atom bonded to the N atom in - NH-C(=O)-OR is further replaced with alkyl, cycloalkyl, alkenyl, alkynyl, aryl, heteroaryl, or aralkyl.

Examples of sulfonylamino (-NH-$SO_2$-R) include alkylsulfonylamino, cycloalkylsulfonylamino, alkenylsulfonylamino, alkynylsulfonylamino, arylsulfonylamino, heteroarylsulfonylamino, and aralkylsulfonylamino. In addition to these, examples include compounds in which an H atom bonded to the N atom in -NH-$SO_2$-R is further replaced with alkyl, cycloalkyl, alkenyl, alkynyl, aryl, heteroaryl, or aralkyl.

Examples of aminosulfonyl (-$SO_2$-NHR) include alkylaminosulfonyl, cycloalkylaminosulfonyl, alkenylaminosulfonyl, alkynylaminosulfonyl, arylaminosulfonyl, heteroarylaminosulfonyl, and aralkylaminosulfonyl. In addition to these, examples include compounds in which an H atom bonded to the N atom in -$SO_2$-NHR is further replaced with alkyl, cycloalkyl, alkenyl, alkynyl, aryl, heteroaryl, or aralkyl.

Examples of sulfamoylamino (-NH-$SO_2$-NHR) include alkylsulfamoylamino, cycloalkylsulfamoylamino, alkenylsulfamoylamino, alkynylsulfamoylamino, arylsulfamoylamino, heteroarylsulfamoylamino, and aralkylsulfamoylamino. Furthermore, the two H atoms bonded to the N atoms in -$NHSO_2$-NHR may be substituted with substituents independently selected from the group consisting of alkyl, cycloalkyl, alkenyl, alkynyl, aryl, heteroaryl, and aralkyl, and the two substituents may form a ring.

Examples of S atom-derived substituents include thiol (-SH), thio (-S-R), sulfinyl (-S(=O)-R), sulfonyl (-$S(O)_2$-R), sulfo (-$SO_3H$), and pentafluorosulfanyl (-$SF_5$).

Examples of thio (-S-R) include alkylthio, cycloalkylthio, alkenylthio, alkynylthio, arylthio, heteroarylthio, and aralkylthio.

Examples of sulfinyl (-S(=O)-R) include alkylsulfinyl, cycloalkylsulfinyl, alkenylsulfinyl, alkynylsulfinyl, arylsulfinyl, heteroarylsulfinyl, and aralkylsulfinyl.

Examples of sulfonyl (-$S(O)_2$-R) include alkylsulfonyl, cycloalkylsulfonyl, alkenylsulfonyl, alkynylsulfonyl, arylsulfonyl, heteroarylsulfonyl, and aralkylsulfonyl.

Examples of N-atom-derived substituents include azido (-$N_3$, also referred to as "azido group"), cyano (-CN), primary amino (-$NH_2$), secondary amino (-NH-R), tertiary amino (-NR(R')), amidino (-C(=NH)-$NH_2$), substituted amidino (-C(=NR)-NR'R"), guanidino (-NH-C(=NH)-$NH_2$), substituted guanidino (-NR-C(=NR"')-NR'R"), and aminocarbonylamino (-NR-CO-NR'R").

Examples of secondary amino (-NH-R) include alkylamino, cycloalkylamino, alkenylamino, alkynylamino, arylamino, heteroarylamino, and aralkylamino.

Examples of tertiary amino (-NR(R')) include an amino group having any two substituents each independently selected from alkyl, cycloalkyl, alkenyl, alkynyl, aryl, heteroaryl, aralkyl, and the like, such as alkyl(aralkyl)amino, and the two substituents may form a ring.

Examples of substituted amidino (-C(=NR)-NR'R") include groups in which the three substituents R, R', and R" on the N atom are each independently selected from alkyl, cycloalkyl, alkenyl, alkynyl, aryl, heteroaryl, and aralkyl, such as alkyl(aralkyl)(aryl)amidino.

Examples of substituted guanidino (-NR-C(=NR"')-NR'R") include a group in which R, R', R", and R"' are each independently selected from alkyl, cycloalkyl, alkenyl, alkynyl, aryl, heteroaryl, and aralkyl, and a group in which they form a ring.

Examples of aminocarbonylamino (-NR-CO-NR'R") include a group in which R, R', and R" are each independently selected from a hydrogen atom, alkyl, cycloalkyl, alkenyl, alkynyl, aryl, heteroaryl, and aralkyl, and a group in which they form a ring.

Examples of B-atom-derived substituents include boryl (-BR(R')), and dioxyboryl (-B(OR)(OR')). The two substituents R and R' may be groups each independently selected from alkyl, cycloalkyl, alkenyl, alkynyl, aryl, heteroaryl, aralkyl, or the like, or may be a group in which they form a ring. Specific examples include a cyclic boryl group, and more specifically include a pinacolate boryl group, a neopentanediolate boryl group, and a catecholate boryl group.

The amino group of the main chain of the amino acid may be unsubstituted ($-NH_2$) or substituted (that is, -NHR). R represents, for example, an alkyl group, an alkenyl group, an alkynyl group, an aryl group, a heteroaryl group, an aralkyl group, or a cycloalkyl group, which is optionally substituted, or a carbon chain attached to the N atom and a carbon atom at position $\alpha$ may form a ring, like proline.

As used herein, an amino acid in which the amino group of the main chain is substituted is referred to as an "N-substituted amino acid". Examples of the "N-substituted amino acid" as used herein include preferably, but are not limited to, N-alkylamino acid, N-$C_1$-$C_6$ alkylamino acid, N-$C_1$-$C_5$ alkylamino acid, N-$C_1$-$C_4$ alkylamino acid, N-$C_1$-$C_3$ alkylamino acid, N-ethylamino acid, N-methylamino acid, N-$C_7$-$C_{14}$ aralkylamino acid, N-benzylamino acid, and N-phenethylamino acid.

Specific examples of the substituent on the nitrogen atom of the N-substituted amino acid (R of -NHR described above) herein include an alkyl group (preferably a $C_1$-$C_6$ alkyl group, more preferably a $C_1$-$C_4$ alkyl group, more preferably a $C_1$-$C_3$ alkyl group, and still more preferably an ethyl group or a methyl group), a $C_7$-$C_{14}$ aralkyl group, a benzyl group, and a phenethyl group. As the substituent on the nitrogen atom of the N-substituted amino acid, an ethyl group or a methyl group is more preferred, and a methyl group is particularly preferred (that is, N-methylamino acid is particularly preferred as the N-substituted amino acid).

The "hydrocarbon group" as used herein refers to a structure made of a carbon atom and a hydrogen atom.

The "amino acid" as used herein includes all isotopes corresponding to each. The isotope of the "amino acid" is a form in which at least one atom is replaced with an atom having the same atomic number (proton number) and a different mass number (total number of protons and neutrons). Examples of the isotope included in the "amino acid" as used herein include a hydrogen atom, a carbon atom, a nitrogen atom, an oxygen atom, a phosphorus atom, a sulfur atom, a fluorine atom, and a chlorine atom, which respectively include $^{2}H$, $^{3}H$, $^{13}C$, $^{14}C$, $^{15}N$, $^{17}O$, $^{18}O$, $^{32}P$, $^{35}S$, $^{18}F$, and $^{36}Cl$. For the compounds as used herein, all the compounds containing any proportions of radioactive or non-radioactive isotopic elements are encompassed within the scope of the present invention.

The number of non-natural amino acid residues contained in the peptide compound according to the present embodiments may be, for example, 1 or more, 2 or more, 3 or more, 4 or more, 5 or more, 6 or more, 7 or more, 8 or more, 9 or more, or 10 or more. The number of non-natural amino acid residues contained in the peptide compound according to the present embodiments may be, for example, 30 or less, 25 or less, 20 or less, 15 or less, 14 or less, 13 or less, or 12 or less. Examples of the range of the number of non-natural amino acid residues contained in the peptide compound according to the present embodiments include 1 or more and 30 or less, 2 or more and 25 or less, 3 or more and 20 or less, 4 or more and 15 or less, 5 or more and 14 or less, 6 or more and 13 or less, 7 or more and 12 or less, or 11. The number of non-natural amino acid residues contained in the peptide compound according to the present embodiments is, for example, 1 to 30, preferably 3 to 15, more preferably 5 to 12, and most preferably 7 to 10.

The number of N-substituted amino acid residues contained in the peptide compound according to the present embodiments may be, for example, 1 or more, 2 or more, 3 or more, 4 or more, or 5 or more. The number of N-substituted amino acid residues contained in the peptide compound according to the present embodiments is, for example, 1 or more, preferably 2 or more, more preferably 3 or more, and most preferably 5 or more.

The peptide compound according to the present embodiments may be a peptide compound having a cyclic moiety (sometimes referred to as "cyclic peptide compound" herein). As used herein, the term "cyclic moiety" of a peptide compound means a cyclic portion formed by linkage of 4 or more amino acid residues. The number of amino acid residues constituting the cyclic moiety of the cyclic peptide compound may be, for example, 5 or more, 6 or more, 7 or more, 8 or more, 9 or more, or 10 or more. The number of amino acid residues constituting the cyclic moiety of the cyclic peptide compound may be, for example, 15 or less, 14 or less, 13 or less, or 12 or less. Examples of the number of amino acid residues constituting the cyclic moiety of the cyclic peptide compound include 5 or more and 15 or less, 6 or more and 15 or less, 7 or more and 15 or less, 8 or more and 15 or less, 9 or more and 15 or less, 10 or more and 15 or less, 5 or more and 14 or less, 6 or more and 14 or less, 7 or more and 14 or less, 8 or more and 14 or less, 9 or more and 14 or less, 10 or more and 14 or less, 5 or more and 13 or less, 6 or more and 13 or less, 7 or more and 13 or less, 8 or more and 13 or less, 9 or more and 13 or less, 10 or more and 13 or less, 5 or more and 12 or less, 6 or more and 12 or less, 7 or more and 12 or less, 8 or more and 12 or less, 9 or more and 12 or less, 10 or more and 12 or less, 5 or more and 11 or less, 6 or more and 11 or less, 7 or more and 11 or less, 8 or more and 11 or less, 9 or more and 11 or less, 10 or more and 11 or less, or 11. The number of amino acid residues constituting the cyclic moiety of the cyclic peptide compound is, for example, 5 to 15, preferably 9 to 15, more preferably 10 to 14, and most preferably 11 to 13. The cyclic moiety is preferably formed via a covalent bond such as

an amide bond, a carbon-carbon bond forming reaction, an S-S bond, a thioether bond, and a triazole bond, and can be formed by allowing a group on the N-terminal side of a linear peptide compound in which amino acid residues are linked and a group on the C-terminal side to be bonded to each other. Specifically, for example, the cyclic moiety can be formed by allowing an amino group on the N-terminal side of a linear peptide compound in which amino acid residues are linked and a carboxy group on the C-terminal side to be bonded to each other. The cyclization may take any form, such as cyclization with a carbon-nitrogen bond such as an amide bond, cyclization with a carbon-oxygen bond such as an ester bond and ether bond, cyclization with a carbon-sulfur bond such as a thioether bond, cyclization with a carbon-carbon bond, cyclization with a sulfur-sulfur bond, or cyclization by heterocyclic construction. Among these, cyclization via a covalent bond such as an amide bond and a carbon-carbon bond is preferred, and cyclization via an amide bond by a carboxy group of a side chain and an amino group of the main chain is more preferred. The positions of the carboxy group, the amino group, and the like used for cyclization may be on the main chain or the side chain, and are not particularly restricted as long as the positions allow the groups to be cyclized.

Specific examples of the peptide compounds according to the present embodiments can include Compounds I to V described in Examples described below.

The structural formulae of Compounds I to V are as follows.

[Table 1]

| Compound No. | Structural formula |
|---|---|
| Compound I | 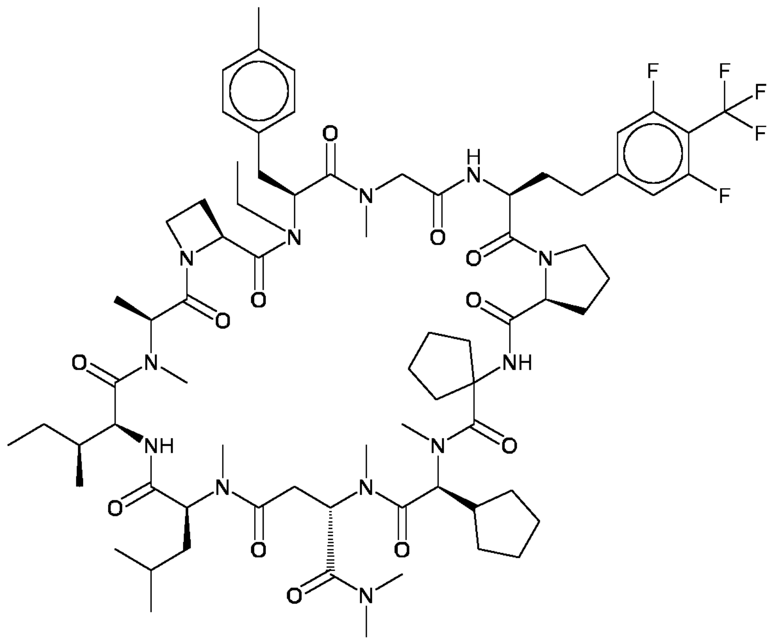 |

(continued)

| Compound No. | Structural formula |
|---|---|
| Compound II | 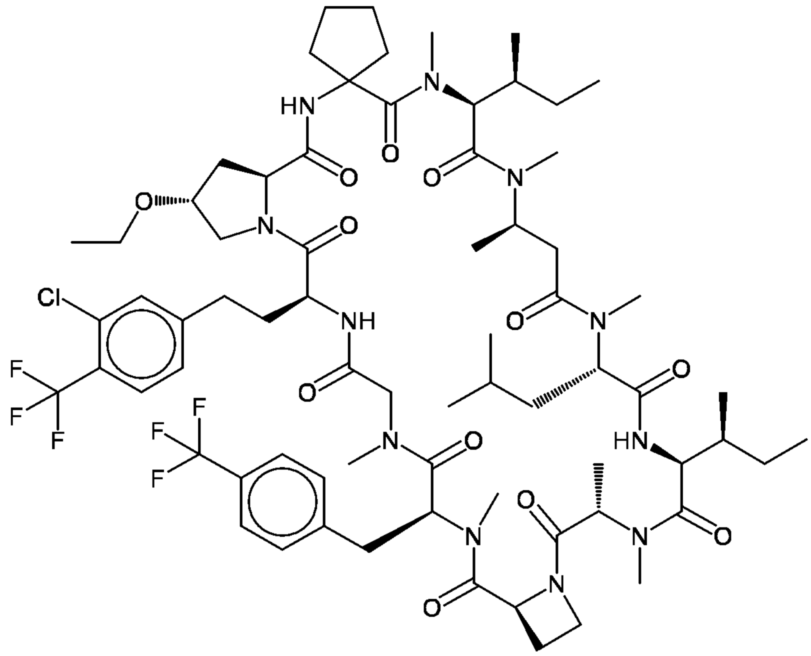 |

[Table 2]

| Compound No. | Structural formula |
|---|---|
| Compound III | 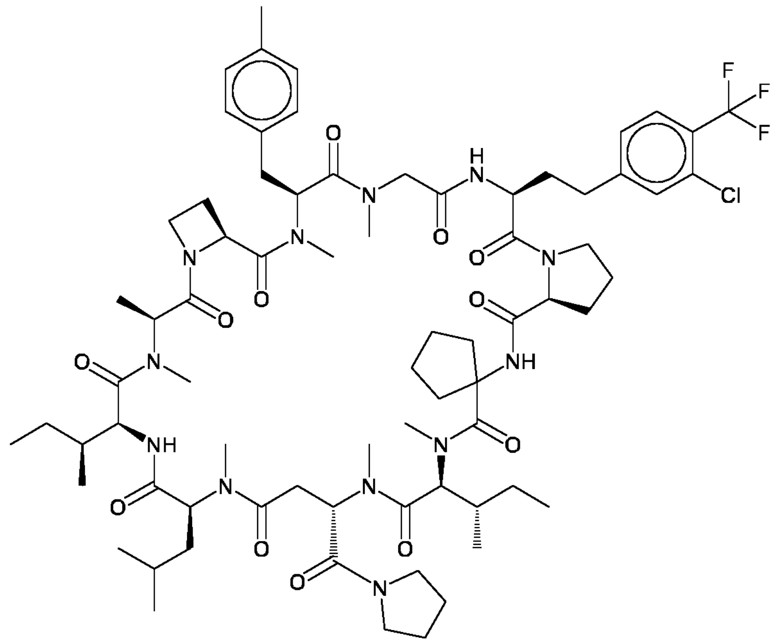 |

(continued)

| Compound No. | Structural formula |
|---|---|
| Compound IV | 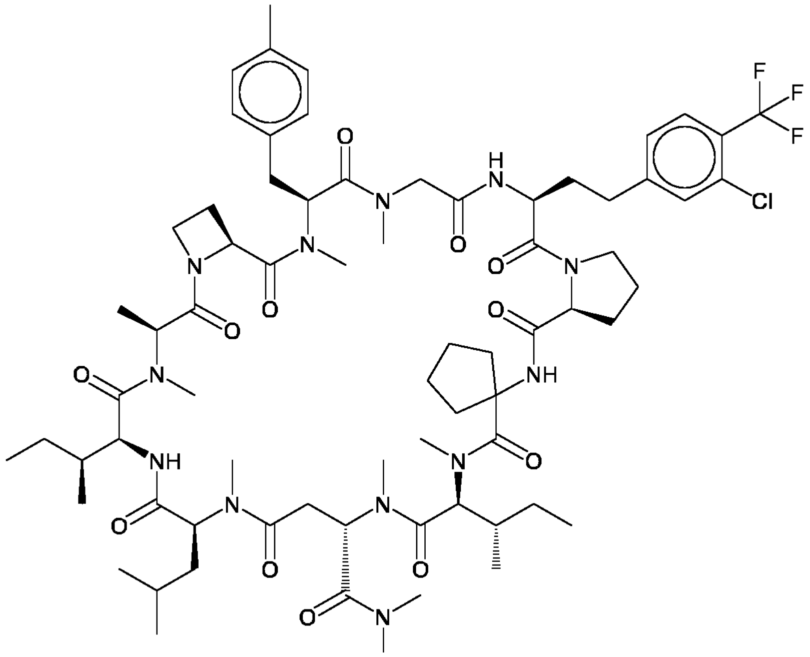 |

[Table 3]

| | |
|---|---|
| Compound V | 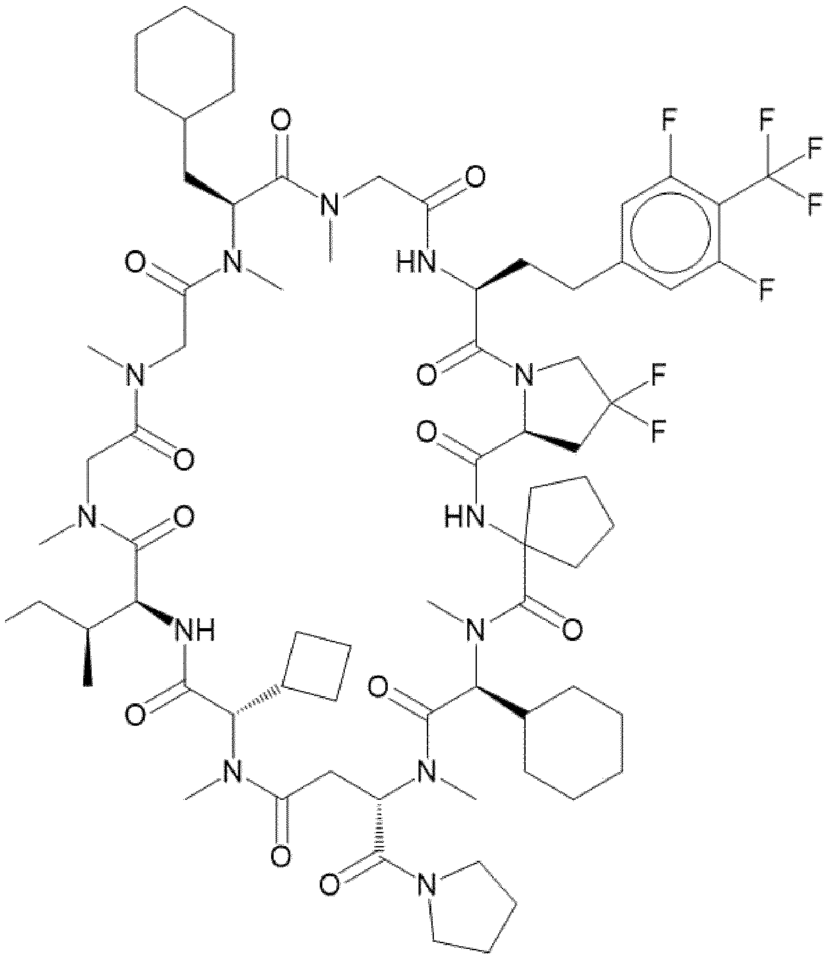 |

It is most preferred that the peptide compound according to the present embodiments is Compound I.

It is preferred that the peptide compound according to the present embodiments has ClogP of 4 or more and 25 or less. The ClogP is a partition coefficient calculated by a computer, and can be determined in accordance with the principle described in "CLOGP Reference Manual Daylight Version 4.9 (release date: August 1, 2011, https://www.daylight.com/dayhtml/doc/clogp/)". Examples of the method for calculating the ClogP include calculating using Daylight Version 4.95 (release date: August 1, 2011, ClogP algorithm version 5.4, database version 28, https://www.daylight.com/dayhtml/doc/release_notes/index.html) of Daylight Chemical Information Systems, Inc.

The ClogP of the peptide compound according to the present embodiments is more preferably 24 or less, further preferably 23 or less, still more preferably 22 or less, still even more preferably 21 or less, and particularly preferably 20 or less. The lower limit of the ClogP of the peptide compound according to the present embodiments is more preferably 5 or more, further preferably 6 or more, still more preferably 7 or more, still even more preferably 8 or more, and particularly preferably 10 or more. Examples of the range of the ClogP of the peptide compound according to the present embodiments include 5 or more and 24 or less, 6 or more and 23 or less, 7 or more and 22 or less, 8 or more and 21 or less, 9 or more and 20 or less, 10 or more and 20 or less, 11 or more and 18 or less, and 11.2 or more and 16.1 or less. The ClogP of the peptide compound according to the present embodiments is, for example, 4 to 25, preferably 6 to 23, more preferably 8 to 21, and most preferably 9 to 20.

It is preferred that the peptide compound according to the present embodiments has ClogP/number of amino acid residues of 1.0 or more. As used herein, the "number of amino acid residues" means the total number of amino acid residues constituting a peptide compound. For example, the number of amino acid residues of a cyclic peptide compound in which the cyclic moiety is composed of 10 amino acid residues and the linear moiety is composed of 1 amino acid residue is 11. The ClogP/number of amino acid residues is a value calculated by dividing the ClogP of a peptide compound by the number of amino acid residues contained in the peptide compound. For example, when the ClogP of a peptide compound is 14.0 and the number of amino acid residues contained in the peptide compound is 7, the ClogP/number of amino acid residues of the peptide compound is calculated to be 2.0.

The ClogP/number of amino acid residues of the peptide compound according to the present embodiments is more preferably 1.1 or more, and further preferably 1.2 or more. The upper limit of ClogP/number of amino acid residues of the peptide compound according to the present embodiments is preferably 1.8 or less, more preferably 1.7 or less, further preferably 1.6 or less, and still more preferably 1.5 or less. Examples of the range of ClogP/number of amino acid residues of the peptide compound according to the present embodiments include 1.0 or more and 1.8 or less, 1.0 or more and 1.7 or less, 1.1 or more and 1.6 or less, and 1.1 or more and 1.5 or less. The ClogP/number of amino acid residues of the peptide compound according to the present embodiments is, for example, 1.0 to 1.8, preferably 1.0 to 1.7, more preferably 1.1 to 1.6, and most preferably 1.1 to 1.5.

(Polymer)

The polymer according to the present embodiments is a pharmaceutically acceptable polymer.

The glass transition temperature of the polymer (Tgp) according to the present embodiments may be, for example, 30°C or higher, 50°C or higher, 90°C or higher, 95°C or higher, or 100°C or higher, 105°C or higher, 110°C or higher, or 115°C or higher. The glass transition temperature of the polymer (Tgp) according to the present embodiments may be, for example, 170°C or lower, 160°C or lower, 150°C or lower, 140°C or lower, 135°C or lower, 130°C or lower, 125 or lower, or 120°C or lower. Examples of the range of the glass transition temperature of the polymer (Tgp) according to the present embodiments include 30°C or higher and 170°C or lower, 90°C or higher and 140°C or lower, 100°C or higher and 135°C or lower, or 105°C or higher and 130°C or lower. The range of the glass transition temperature of the polymer (Tgp) according to the present embodiments is, for example, 30°C to 170°C, preferably 90°C to 140°C, more preferably 100°C to 135°C, and most preferably 105°C to 130°C.

The weight average molecular weight of the polymer according to the present embodiments may be, for example, 3,000 or more, 5,000 or more, 10,000 or more, 15,000 or more, or 20,000 or more. The weight average molecular weight of the polymer according to the present embodiments may be, for example, 400,000 or less, 200,000 or less, 150,000 or less, 100,000 or less, 80,000 or less, 70,000 or less, 60,000 or less, or 50,000 or less. Examples of the range of the weight average molecular weight of the polymer according to the present embodiments include 3,000 or more and 400,000 or less, 5,000 or more and 200,000 or less, 10,000 or more and 150,000 or less, 15,000 or more and 80,000 or less, or 20,000 or more and 50,000 or less. The weight average molecular weight of the polymer according to the present embodiments is, for example, 3,000 to 400,000, preferably 5,000 to 200,000 or less, more preferably 10,000 to 80,000 or less, and most preferably 15,000 or more and 50,000 or less. The "weight average molecular weight" as used herein means a value measured by size exclusion chromatography.

The polymer that can be used in the solid dispersion according to the present invention should be pharmaceutically acceptable and preferably have at least some solubility in an aqueous solution of a physiologically relevant pH (e.g., 1 to 8). Almost any polymer having a water solubility of at least about 0.1 mg/mL over at least a portion of the pH range of 1 to 8 may be suitable.

Examples of the polymer according to the present embodiments include a non-ionic polymer and an ionic polymer. The non-ionic polymer is a polymer that is substantially free of ionic functional groups. The "substantially free of ionic functional groups" means that the number of ionic groups covalently attached to the polymer is less than about 0.05 milliequivalents per gram of polymer. Preferably, the number is less than about 0.02 milliequivalents per gram of neutral polymer. The "ionic functional group" means a functional group that is ionized by at least about 10% over at least a part of a physiologically relevant pH range of 1 to 8. Such a group has a pKa value of about 0 to 9.

Examples of the non-ionic polymer include a neutral non-cellulosic polymer, a neutral cellulosic polymer, and a neutralized acidic polymer.

Examples of the neutral non-cellulosic polymer can include

(i) a vinyl polymer or copolymer having at least one substituent selected from the group consisting of hydroxyl, alkylacyloxy, and cyclic amide;
(ii) a vinyl copolymer having at least one hydrophilic hydroxyl-containing repeat unit and at least one hydrophobic alkyl or aryl-containing repeat unit, or a polyvinyl alcohol;
(iii) a polyvinyl alcohol having at least a portion of a repeat unit in a non-hydrolyzed (vinyl acetate) form, a polyvinyl alcohol-polyvinyl acetate copolymer, a polyvinyl pyrrolidone, a copovidone, a copolymer of acrylate and methacrylic acid, or a polyethylene polyvinyl alcohol copolymer; and
(iv) a polyoxyethylene-polyoxypropylene block copolymer.

Examples of the neutral cellulosic polymer can include hydroxypropyl methyl cellulose acetate (HPMCA), hydroxypropyl methyl cellulose (HPMC), hydroxypropyl cellulose (HPC), methyl cellulose, hydroxyethyl methyl cellulose, hydroxyethyl cellulose, hydroxyethyl cellulose acetate, and hydroxyethyl ethyl cellulose.

In another aspect, the polymers that can be used in the present invention include a neutralized acidic polymer.

The neutralized acidic polymer is described in more detail in U.S. Patent Application Publication No. 2003-0054038, entitled "Pharmaceutical Compositions of Drugs and Neutralized Acid Polymers" filed June 17, 2002. The relevant disclosures of the patent application are incorporated herein by reference. The "acidic polymer" means any polymer having a significant number of acidic moieties. In general, the significant number of acidic moieties is more than or equal to about 0.05 milliequivalents per gram of polymer. The "acidic moiety" includes any functional group that, when contacted or dissolved in water, is at least partially capable of imparting a hydrogen cation to the water and thus sufficiently acidic to increase the concentration of hydrogen ions. This definition includes any functional group or "substituent" (a functional group when covalently attached to a polymer) having less than about 10 pKa.

The "neutralized acidic polymer" means any acidic polymer in which a substantial portion of the "acidic moiety" or "acidic substituent" is "neutralized," i.e., present in a deprotonated form. A "degree of neutrality" $\alpha$ of a polymer substituted with a monoprotonic acid (e.g., carboxylic acid) is defined as a fraction of the acidic moiety on the polymer that is neutralized, i.e., deprotonated by a base. Typically, an acidic polymer is considered as a "neutralized acidic polymer," when $\alpha$ is at least about 0.01 (or 1%), more preferably at least about 0.1 (10%), further preferably at least about 0.5 (50%), and most preferably at least 0.9 (90%) (meaning that at least 90% of the acidic moieties are neutralized).

Examples of the neutralized acidic polymer can include cellulose acetate phthalate, cellulose acetate trimellitate, cellulose acetate succinate, methyl cellulose phthalate, hydroxymethyl cellulose ethyl phthalate, hydroxypropyl methyl cellulose phthalate, hydroxypropyl methyl cellulose acetate succinate (hypromellose acetate succinate: HPMCAS), hydroxypropyl methyl acetate maleate, hydroxypropyl methyl trimellitate, carboxymethyl ethyl cellulose, polyvinyl butyrate phthalate, polyvinyl alcohol acetate phthalate, a methacrylic acid/ethyl acrylate copolymer, a methacrylic acid/methyl methacrylate copolymer, and a methacrylic acid copolymer.

Examples of the ionic polymer include an acidic polymer and a basic polymer. The "ionic polymer" has a substantially ionic function, and is ionized by at least about 10% over at least a part of a physiologically relevant pH range of 1 to 8. The ionic polymer is generally classified into an acidic polymer and a basic polymer in the pH range to be ionized. The acidic polymer (or enteric polymer) is a polymer that has the property of dissolving in a neutral or alkaline solution, and the basic polymer is a polymer that has the property of dissolving in an acidic or neutral solution.

Examples of the acidic polymer can include cellulose acetate phthalate, cellulose acetate trimellitate, cellulose acetate succinate, methyl cellulose phthalate, hydroxymethyl cellulose ethyl phthalate, hydroxypropyl methyl cellulose phthalate, hydroxypropyl methyl cellulose acetate succinate (HPMCAS), hydroxypropyl methyl acetate maleate, hydroxypropyl methyl trimellitate, carboxymethyl ethyl cellulose, polyvinyl butyrate phthalate, polyvinyl alcohol acetate phthalate, a methacrylic acid/ethyl acrylate copolymer, a methacrylic acid/methyl methacrylate copolymer, and a methacrylic acid copolymer.

Examples of the basic polymer can include an amino alkyl methacrylic acid copolymer E, and polyvinyl acetal diethyl aminoacetate.

The polymer according to the present embodiments may be used alone, or used in combination of two or more.

The polymer used according to the present embodiments may be at least one selected from the group consisting of a polymer having a hydroxypropyl cellulose backbone, a polymer having a povidone backbone, and a block copolymer polymer, and may be at least one selected from the group consisting of hydroxypropyl methyl cellulose acetate succinate (HPMCAS), hydroxypropyl methyl cellulose (HPMC), polyvinyl pyrrolidone, a vinyl pyrrolidone/vinyl acetate copolymer, a methacrylic acid/methyl methacrylate copolymer, and a methacrylic acid/ethyl acrylate copolymer.

(Excipient)

The excipient according to the present embodiments should be pharmaceutically acceptable to the extent that the excipient does not impair the effect according to the present invention, and examples thereof include starches such as corn starch, lactose, glucose, and mannitol.

(Disintegrant)

The disintegrant according to the present embodiments should be pharmaceutically acceptable to the extent that the disintegrant does not impair the effect according to the present invention, and examples thereof include low-substituted hydroxypropyl cellulose, carmellose, carmellose calcium, croscarmellose sodium, carboxymethyl starch sodium, crospovidone, and polyvinylpyrrolidone.

(Solid dispersion)

The solid dispersion according to the present embodiments is composed of a substance to be dispersed and a polymer, and the substance to be dispersed is dispersed in the polymer. The substance to be dispersed is preferably uniformly dispersed in the polymer. **In** the solid dispersion, the substance to be dispersed is preferably dispersed as very

small units, and more preferably dispersed at a molecular level in the polymer.

The solid dispersion according to the present embodiments meets the following (I) or (II):

(I) a glass transition temperature of the substance to be dispersed (Tgc) and a glass transition temperature of the solid dispersion are different;
(II) the glass transition temperature of the solid dispersion is 94.5% or more and 105.5% or less with respect to a value of Tga represented by the following formula (A);
[Expression 3]

$$Tga = \frac{Wc}{Wa} \times Tgc + \frac{Wp}{Wa} \times Tgp \quad \text{... Formula (A)}$$

in the formula (A), Tgc represents the glass transition temperature of the substance to be dispersed, Tgp represents the glass transition temperature of the polymer, Wa represents the total mass of the solid dispersion, Wc represents the total mass of the substance to be dispersed contained in the solid dispersion, and Wp represents the total mass of the polymer contained in the solid dispersion, respectively.

The glass transition temperature of the substance to be dispersed (Tgc) and the glass transition temperature of the solid dispersion are usually different, and may be different by 1°C or more, 2°C or more, 3°C or more, 4°C or more, 5°C or more, 8°C or more, 10°C or more, 15°C or more, 20°C or more, 25°C or more, or 30°C or more. The glass transition temperature of the substance to be dispersed (Tgc) and the glass transition temperature of the solid dispersion are different, for example, by 1°C or more, preferably 2°C or more, more preferably 3°C or more, and most preferably 5°C or more. When the glass transition temperature of the substance to be dispersed (Tgc) and the glass transition temperature of the solid dispersion are different, it can be inferred that the substance to be dispersed is dispersed as uniform and very small units in the solid dispersion.

The glass transition temperature of the substance to be dispersed (Tgc) may be higher than the glass transition temperature of the solid dispersion, and may be higher by 1°C or more, 2°C or more, 3°C or more, 4°C or more, 5°C or more, 8°C or more, 10°C or more, 15°C or more, 20°C or more, 25°C or more, or 30°C or more. The glass transition temperature of the substance to be dispersed (Tgc) is higher than the glass transition temperature of the solid dispersion, for example, by 1°C or more, preferably 2°C or more, more preferably 3°C or more, and most preferably 5°C or more.

The glass transition temperature of the solid dispersion may be, for example, 94.5% or more and 105.5% or less, 95% or more and 105% or less, 96% or more and 104% or less, 97% or more and 103% or less, 98% or more and 102% or less, 99% or more and 101% or less, and 100% with respect to the value of Tga represented by the formula (A). The glass transition temperature of the solid dispersion is, for example, 94.5% to 105.5%, preferably 95% to 105%, more preferably 96% to 104%, and most preferably 97% to 103%, with respect to the value of Tga represented by the formula (A).

The glass transition temperature of the solid dispersion may be, for example, 94.5% or more and 105.5% or less, 95.0% or more and 105.0% or less, 96.0% or more and 104.0% or less, 97.0% or more and 103.0% or less, 98.0% or more and 102.0% or less, 99.0% or more and 101.0% or less, and 100.0% with respect to the value of Tga represented by the formula (A). The glass transition temperature of the solid dispersion is, for example, 94.5% to 105.5%, preferably 95.0% to 105.0%, more preferably 96.0% to 104.0%, and most preferably 97.0% to 103.0%, with respect to the value of Tga represented by the formula (A).

The glass transition temperature of the solid dispersion according to the present embodiments may be, for example, 100°C or higher, 110°C or higher, 120°C or higher, 125°C or higher, 130°C or higher, 135°C or higher, or 140°C or higher. The glass transition temperature of the solid dispersion according to the present embodiments may be, for example, 200°C or lower, 190°C or lower, 180°C or lower, 170°C or lower, or 165°C or lower. Examples of the range of the glass transition temperature of the solid dispersion according to the present embodiments include 100°C or higher and 200°C or lower, 120°C or higher and 180°C or lower, 130°C or higher and 170°C or lower, or 140°C or higher and 165°C or lower. The glass transition temperature of the solid dispersion according to the present embodiments is, for example, 100°C to 200°C, preferably 120°C to 180°C, more preferably 130°C to 170°C, and most preferably 140°C to 165°C.

The solid dispersion according to the present embodiments may be one in which the glass transition temperature of the substance to be dispersed (Tgc) exceeds the glass transition temperature of the polymer (Tgp). Specifically, for example, the glass transition temperature of the substance to be dispersed (Tgc) may be higher than the glass transition temperature of the polymer (Tgp) by 1°C or more, 3°C or more, 10°C or more, 15°C or more, 20°C or more, 25°C or more, 30°C or more, 35°C or more, 40°C or more, 45°C or more, 50°C or more, 80°C or more, or 100°C or more. The glass transition temperature of the substance to be dispersed (Tgc) is higher than the glass transition temperature of the polymer (Tgp), for example, by 10°C or more, preferably 20°C or more, more preferably 30°C or more, and most preferably 40°C or more.

In the present embodiments, it is preferred that the Tgc is higher than the glass transition temperature of the solid

dispersion and the glass transition temperature of the solid dispersion is higher than the Tgp.
Also, for example, the Tgc may be 110°C to 180°C, the glass transition temperature of the solid dispersion may be 100°C to 170°C, and the Tgp may be 30°C to 170°C, preferably the Tgc may be 120°C to 175°C, the glass transition temperature of the solid dispersion may be 110°C to 170°C, and the Tgp may be 90°C to 140°C, more preferably the Tgc may be 130°C to 170°C, the glass transition temperature of the solid dispersion may be 120°C to 165°C, and the Tgp may be 100°C to 135°C, and most preferably the Tgc may be 140°C to 165°C, the glass transition temperature of the solid dispersion may be 130°C to 165°C, and the Tgp may be 105°C to 130°C.

The solid dispersion according to the present embodiments has a ratio of the total mass of the polymer to the total mass of the substance to be dispersed of less than 1.0. The ratio may be, for example, 0.90 or less, 0.80 or less, 0.70 or less, 0.60 or less, 0.50 or less, 0.40 or less, 0.30 or less, 0.20 or less, 0.10 or less, 0.010 or less, or 0.0010 or less. The lower limit of the ratio is not particularly limited, but for example, 0.00010 or more. The ratio of the total mass of the polymer to the total mass of the substance to be dispersed is, for example, less than 1.0, preferably 0.8 or less, more preferably 0.5 or less, and most preferably 0.4 or less. The ratio is, for example, more than 0. The ratio of the total mass of the polymer to the total mass of the substance to be dispersed is, for example, more than 0 and less than 1.0, preferably more than 0 and 0.8 or less, more preferably more than 0 and 0.5 or less, and most preferably more than 0 and 0.4 or less.

The content of the substance to be dispersed in the solid dispersion according to the present embodiments may be, for example, more than 50% by mass, 55% by mass or more, 60% by mass or more, 62.5% by mass or more, 65% by mass or more, 70% by mass or more, or 75% by mass or more based on the total amount of the solid dispersion. The content of the substance to be dispersed in the solid dispersion according to the present embodiments may be, for example, less than 100% by mass, 99% by mass or less, 97.5% by mass or less, 95% by mass or less, 92.5% by mass or less, 90% by mass or less, 87.5% by mass or less, 85% by mass or less, 82.5% by mass or less, or 80% by mass or less based on the total amount of the solid dispersion. Examples of the range of the content of the substance to be dispersed in the solid dispersion according to the present embodiments include more than 50% by mass and less than 100% by mass, 60% by mass or more and 99% by mass or less, 65% by mass or more and 95% by mass or less, 70% by mass or more and 90% by mass or less, or 75% by mass or more and 85% by mass or less based on the total amount of the solid dispersion. The content of the substance to be dispersed in the solid dispersion according to the present embodiments is, for example, more than 50% by mass or less than 100% by mass, preferably 60% by mass or more and 99% by mass or less, more preferably 65% by mass or more and 90% by mass or less, and most preferably 70% by mass or more and 85% by mass or less based on the total amount of the solid dispersion. The content of the substance to be dispersed in the solid dispersion can be measured, for example, by a method using high-performance liquid chromatography (HPLC) as described in Examples below.

The content of the polymer in the solid dispersion according to the present embodiments may be, for example, less than 50% by mass, 45% by mass or less, 40% by mass or less, 38.5% by mass or less, 35% by mass or less, 30% by mass or less, or 25% by mass or less based on the total amount of the solid dispersion. The content of the polymer in the solid dispersion according to the present embodiments may be, for example, more than 0% by mass, 1% by mass or more, 2.5% by mass or more, 5% by mass or more, 7.5% by mass or more, 10% by mass or more, 12.5% by mass or more, 15% by mass or more, 17.5% by mass or more, or 20% by mass or more based on the total amount of the solid dispersion. Examples of the range of the content of the polymer in the solid dispersion according to the present embodiments include more than 0% by mass and less than 50% by mass, 1% by mass or more and 40% by mass or less, 5% by mass or more and 35% by mass or less, 10% by mass or more and 30% by mass or less, or 15% by mass or more and 25% by mass or less based on the total amount of the solid dispersion. The content of the polymer in the solid dispersion according to the present embodiments is, for example, more than 0% by mass or less than 50% by mass, preferably 1% by mass or more and 40% by mass or less, more preferably 10% by mass or more and 35% by mass or less, and most preferably 15% by mass or more and 30% by mass or less based on the total amount of the solid dispersion. The content of the polymer in the solid dispersion can be determined by subtracting the content (% by mass) of the substance to be dispersed contained in the solid dispersion measured by a method using high-performance liquid chromatography (HPLC) from 100% by mass.

The solid dispersion according to the present embodiments may have a bulk density of 0.15 g/mL or more calculated from a volume of powder obtained by gently filling 0.5 g of the solid dispersion into a 5 mL measuring cylinder.

The solid dispersion according to the present embodiments may be one in which, in a physical stability test under conditions of 40°C, 75%RH, a crystal is not observed until one month after starting the test. The physical stability test is a test performed by the method described in Examples below. Specifically, the physical stability test is a test in which the solid dispersion is stored for a predetermined period of time under an environment maintained at a specific temperature and humidity, and after a predetermined period of time, the presence or absence of a halo pattern is checked, for example, by XRD measurement. Since the solid dispersion according to the present embodiments has excellent amorphous stability, the solid dispersion according to the present embodiments can maintain the amorphous state until one month after starting the test under conditions of 40°C, 75%RH.

Similarly, the solid dispersion according to the present embodiments may be one in which, in a physical stability test under conditions of 80°C, 75%RH, a crystal is not observed until one month after starting the test.

The solid dispersion according to the present embodiments may have a disintegration time of 1,000 seconds or less in a disintegration test under conditions using a second fluid for dissolution test (a fluid obtained by adding 118 mL of 0.2 M sodium hydroxide test solution and water to 250 mL of 0.2 M potassium dihydrogen phosphate test solution to be 1,000 mL, the pH of which is 6.7 or more and 6.9 or less) as a test solution, a test solution temperature of 37.0°C and an amplitude-frequency of 30 times/minute. The disintegration time may be, for example, 950 seconds or less, 900 seconds or less, 850 seconds or less, or 800 seconds or less. The solid dispersion to be subjected to the disintegration test is preferably in the dosage form of a tablet.

(Production method of solid dispersion)

The solid dispersion according to the present embodiments can be produced, for example, by a production method including a step of mixing a substance to be dispersed and a polymer in a solvent to prepare a mixed solution (a mixing step), and a step of removing the solvent from the mixed solution obtained in the mixing step to obtain a solid dispersion containing the substance to be dispersed and the polymer (an obtaining step).

As the substance to be dispersed and the polymer, those same as the substance to be dispersed and the polymer described above can be used, respectively.

The solvent used in the mixing step is only required to be capable of dissolving the substance to be dispersed and the polymer, and examples thereof include water, alcohol (for example, methanol, ethanol, n-propanol, iso-propanol and butanol), ketone (for example, acetone, methyl ethyl ketone and methyl iso-butyl ketone), an ester (for example, ethyl acetate and propyl acetate), acetonitrile, methylene chloride, toluene, 1,1,1-trichloroethane, and tetrahydrofuran. The solvent may be used alone, or used as a mixed solvent obtained by mixing two or more.

The obtaining step may be performed, for example, by a spray drying method using a spray drying device (e.g., a small spray dryer B-290, manufactured by BUCHI). Examples of the conditions for spray drying include the conditions described in Examples described below. Specifically, for example, a solution containing the substance to be dispersed and the polymer can be spray-dried in a spray dryer at an inlet temperature of 50-90°C (outlet temperature of 45-55°C), an aspirator output of 100%, a peristaltic pump output of 20-25%, and a spray air flow meter height of 25-30 mm, and the obtained powder can be dried in a vacuum dryer at 60°C, 0 Pa overnight to obtain a powder of a solid dispersion.

The term "spray drying" has been conventionally used and is broadly refers to a method including crushing (spraying) a liquid mixture into small droplets in a spray drying device, and removing a solvent fast from the mixture. In the spray drying device, a strong propulsive force works to evaporate the solvent from the droplets. Spray drying methods and spray drying devices are generally described in Perry's Chemical Engineers' Handbook, pages 20-54 to 20-57 (6th edition, 1984). Further details regarding spray drying methods and devices are outlined in Marshall, "Atomization and Spray-Drying," 50 Chem. Eng. Prog. Monogr. Series 2 (1954) and Masters, Spray-Drying Handbook (4th edition, 1985). The strong propulsive force for solvent evaporation is generally provided by maintaining a partial pressure of the solvent in the spray drying device much lower than the vapor pressure of the solvent at the temperature of the droplets to be dried. This is accomplished by (1) maintaining the pressure in the spray drying device at a partial vacuum (e.g., 0.01 to 0.50 atm); (2) mixing the droplets with a warm dry gas; or (3) both (1) and (2). Furthermore, at least a portion of the heat required for solvent evaporation can be provided by heating of the spray solution.

The solvent-containing feedstock can be spray-dried under very varied conditions to obtain a solid dispersion that still has acceptable properties. For example, various types of nozzles can be used to spray the spray solution. In doing so, the spray solution can be introduced into a spray drying chamber as a collection of droplets. Essentially any type of nozzle can be used to spray a solution as long as the formed droplets are small enough and dried enough (by evaporation of the solvent) not to adhere to or not cover the spray dry chamber wall.

The maximum droplet size varies widely depending on the size, shape and flow pattern in the spray dryer, but in general, droplets should have a diameter of less than about 500 μm when discharged from the nozzle. Examples of the type of nozzles that can be used to form a solid dispersion include a two-fluid nozzle, a fountain-type nozzle, a flat-fan-type nozzle, a pressure nozzle, and a rotary atomizer. In a preferred aspect, a pressure nozzle is used. Details are disclosed in U.S. Patent Application Publication No. 2003-0185893, filed January 24, 2003, which is incorporated herein by reference.

The spray solution can be supplied to the spray nozzle(s) at a wide range of temperatures and flow rates. In general, the temperature of the spray solution can be in any range from just above the freezing point of the solvent to about 20°C above the ambient pressure boiling point thereof (by pressurizing the solution). In some cases, the temperature may be higher than that. The flow rate of the spray solution into the spray nozzle can vary widely depending on the type of nozzle, the size of the spray dryer, and the spray drying conditions (e.g., the inlet temperature and flow rate of dry gas). In general, the energy for evaporating the solvent from the spray solution by the spray drying method is mainly derived from the dry gas.

The dry gas may be essentially any gas, but inert gases such as nitrogen, nitrogen-enriched air, or argon are utilized for safety reasons and to minimize undesirable oxidation of drugs or other substances in the solid dispersion. The dry gas is typically introduced into a drying chamber at a temperature of about 60°C to about 240°C.

Since the surface area to volume ratio of the liquid droplets is large, and the propulsive force for solvent evaporation is also large, the solidification time of the liquid droplets is fast. The solidification time should be less than about 20 seconds, preferably less than about 10 seconds, and even more preferably less than 1 second. This fast solidification is often important for the particles to maintain a uniform and homogeneous dispersion instead of separating into a drug-rich phase and a polymer-rich phase.

After solidification, the solid powder typically remains in the spray drying chamber for about 5 to 60 seconds to further evaporate the solvent from the solid powder. The final solvent content of the solid dispersion when exiting the dryer should be low. Because the low content reduces the mobility of the drug molecule in the solid dispersion and improves its stability. In general, the solvent content of the solid dispersion when exiting the spray drying chamber should be less than 10% by weight, preferably less than 2% by weight, and further preferably less than 1% by weight.

After formation, the solid dispersion can be dried using a suitable drying method to remove the residual solvent. Examples of the drying method include a tray drying, a vacuum drying, a fluidized bed drying, a microwave drying, a belt drying, a rotary drying, and other drying methods known in the art. Suitable secondary drying methods are a vacuum drying, a tray drying, or the like. To minimize chemical degradation during drying, the drying is preferably performed under an inert gas such as nitrogen or under vacuum.

Solid dispersions are usually in the form of small particles. The volume average diameter of the particles may be less than 500 μm, less than 100 μm in diameter, less than 50 μm in diameter, or less than 25 μm in diameter. When the solid dispersion is formed by spray drying, the resulting dispersion takes such a form of small particles.

Examples of an embodiment of a method for producing a solid dispersion utilizing spray drying can include a production method including a step of evaporating a solvent in a mixture containing (1) a substance to be dispersed that has a molecular weight of 1,000 g/mol or more and 5,000 g/mol or less and is amorphous and (2) a polymer in an amount such that a ratio of a total mass of the polymer to a total mass of the substance to be dispersed is less than 1.0 by spray drying (corresponding to a removing step). The production method according to the embodiment may further include a step of mixing the substance to be dispersed and the polymer in a solvent to obtain a mixture containing the substance to be dispersed and the polymer (corresponding to a mixing step) prior to the step of evaporating the solvent by spray drying.

[Pharmaceutical Composition]

The pharmaceutical composition according to the present embodiments is one containing the solid dispersion as described above. The pharmaceutical composition according to the present embodiments may contain only the above-described solid dispersion or may contain, in addition to the above-described solid dispersion, other pharmaceutically acceptable components. The pharmaceutical composition may use the above-described solid dispersion as is, and may contain the above-described solid dispersion as is or a mixture of the solid dispersion and other pharmaceutically acceptable components. The pharmaceutical composition may take the form of a powder, a granule, a tablet, a capsule filled therewith, and the like.

The content of the solid dispersion contained in the pharmaceutical composition according to the present embodiments may be, for example, 100% by mass based on the total amount of the pharmaceutical composition, and may be 99% by mass or less, 90% by mass or less, 80% by mass or less, 70% by mass or less, 60% by mass or less, 50% by mass or less, 40% by mass or less, or 30% by mass or less.

The other components are not particularly limited as long as they are pharmaceutically acceptable, and examples include an excipient, a disintegrant, a glidant or lubricant, a taste and odor masking agent, a stabilizer, a pH adjusting agent, a preservative, an antioxidant, and a surfactant.

Examples of the excipient include lactose, corn starch, white sugar, glucose, mannitol, sorbit, starch, crystalline cellulose, silicon dioxide, and magnesium aluminate metasilicate.

Examples of the disintegrant include sodium starch, pregelatinized starch, agar, gelatin powder, crystalline cellulose, calcium carbonate, sodium chloride, sodium bicarbonate, calcium citrate, anhydrous silicic acid, dextrin, pectin, carmellose, carmellose calcium, croscarmellose sodium, low-substituted hydroxypropyl cellulose, and sodium starch glycolate.

Examples of the glidant or lubricant include light anhydrous silicic acid, aqueous silicic acid dioxide, magnesium stearate, and talc.

Examples of the taste and odor masking agent include cocoa powder, menthol, aromatic powder, peppermint oil, borneol, and cinnamon powder.

Examples of the stabilizer include phosphatidic acid, ascorbic acid, glycerin, and cetanol.

Examples of the pH adjusting agent include lactic acid, succinic acid, gluconic acid, citric acid, citric acid hydrate, trisodium citrate, phosphoric acid, potassium carbonate, sodium hydrogen carbonate, tartaric acid, malic acid, ascorbic acid, fumaric acid, aspartic acid, glutamic acid, glutamic acid hydrochloride, malonic acid, maleic acid, meglumine, arginine, lysine, glycine, sodium carbonate, and sodium hydrogen phosphate.

Examples of the preservative include ethyl paraoxybenzoate and propyl paraoxybenzoate.

Examples of the antioxidant include butylated hydroxytoluene, butylated hydroxyanisole, propyl gallate, and gallic acid propyl ester.

The pharmaceutical composition according to the present embodiments may further contain a surfactant. The surfactant is not particularly limited as long as it is pharmaceutically acceptable.

The "surfactant" in the present invention means a substance having both a hydrophilic group and a hydrophobic group in a molecule, and includes an ionic surfactant and a non-ionic surfactant.

An ionic surfactant means an ionic surfactant that, when dissolved in water, is ionized to become an ion (an atom or a group of atoms having a charge). The ionic surfactant is further classified into an anionic surfactant, a cationic surfactant, and an amphoteric surfactant, depending on the charge of the ion generated.

Examples of the non-ionic surfactant include a sugar ester type surfactant such as sorbitan fatty acid ester (C12-18), polyoxyethylene (POE) sorbitan fatty acid ester (C12-18), or sucrose fatty acid ester; a fatty acid ester type surfactant such as POE fatty acid ester (C12-18), POE resin acid ester, or POE fatty acid diester (C12-18); an alcohol type surfactant such as POE alkyl ether (C12-18); an alkyl phenol type surfactant such as POE alkyl (C8-12) phenyl ether, POE dialkyl (C8-12) phenyl ether, or POE alkyl (C8-12) phenyl ether formalin condensate; a polyoxyethylene polyoxypropylene block polymer type surfactant such as a polyoxyethylene polyoxypropylene block polymer, or alkyl (C12-18) polyoxyethylene polyoxypropylene block polymer ether; an alkylamine type surfactant such as POE alkylamine (C12-18) or POE fatty acid amide (C12-18); a bisphenol-type surfactant such as POE fatty acid bisphenyl ether; a polyaromatic ring-type surfactant such as polyoxyalkylene (POA) benzylphenyl (or phenylphenyl) ether and POA styrylphenyl (or phenylphenyl) ether; a POE ether and ester-type silicon and fluoro-based surfactant; and a vegetable oil-type surfactant such as POE castor oil and POE cured castor oil.

Preferred examples of the non-ionic surfactant include polyoxyl 40 stearate, sorbitan trioleate, polyoxyethylene (105) polyoxypropylene (5) glycol, polyoxyethylene cured castor oil 60, polyoxyl 35 castor oil, lauromacrogol, and tocopherol polyethylene glycol succinate (TPGS).

Examples of the anionic surfactant include a sulfate-type surfactant such as alkyl sulfate (C12-18), POE alkylether sulfate (C12-18), POE alkylphenylether sulfate (C12-18), POE benzyl(or stylyl)phenyl (or phenylphenyl) ether sulfate, polyoxyethylene, or polyoxypropylene block polymer sulfate; a sulfonate-type surfactant such as paraffin (alkane) sulfonate (C12-22), alpha olefin sulfonate (C14-16), dialkylsulfosuccinate (C8-12), alkylbenzene sulfonate (C12), mono or dialkyl (C3-6) naphthalenesulfonate, naphthalenesulfonate-formaline condensate, alkyl (C8-12) diphenylether disulfonate, lignin sulfonate, POE alkyl (C8-12) phenylether sulfonate, or half-ester of POE alkyl (C12-18) ether sulfosuccinic acid; a carboxylic acid salt-type surfactant such as a fatty acid salt (C12-18), N-acyl amino acid salt (C12-18), or a resin acid salt; and a phosphate-type surfactant such as POE alkyl (C12-18) ether phosphate, POE mono or dialkyl (C8-12) phenyl ether phosphate, POE benzyl (or stylyl) phenyl (or phenylphenyl) ether phosphate, polyoxyethylene-polyoxypropylene block polymer, or alkyl (C8-12) phosphate.

In the present invention, the surfactant may be used alone, or used in combination of two or more in appropriate proportions.

The surfactant according to one embodiment may be a surfactant having a linear hydrocarbon group. The number of carbon atoms contained in the linear hydrocarbon group may be 5 or more, 6 or more, 8 or more, 10 or more, or 12. Examples of the range of the number of carbon atoms contained in the linear hydrocarbon group include 5 or more and 17 or less, 6 or more and 15 or less, 8 or more and 13 or less, 10 or more and 12 or less, or 12.

It is also preferred that the surfactant according to one embodiment is a compound represented by any of the following general formulae (a1) to (a3):

[Formula 8]

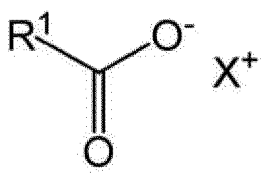

(a 1)

[Formula 9]

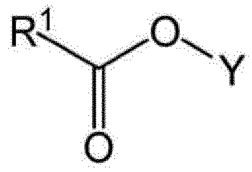

(a 2)

[Formula 10]

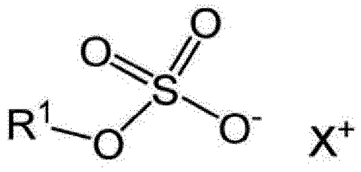

(a 3)

In the general formulae (a1) to (a3), $R^1$ represents an optionally substituted, saturated or unsaturated, linear hydrocarbon group having 5 or more and 17 or less carbon atoms, X represents sodium or potassium, and Y represents a group represented by the following formula (a4) or a stereoisomer thereof.

[Formula 11]

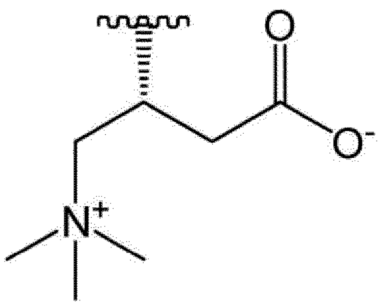

(a 4)

In the general formulae (a1) to (a3), $R^1$ is preferably an alkyl group having 5 or more and 17 or less carbon atoms, more preferably an alkyl group having 7 or more and 12 or less carbon atoms, further preferably an alkyl group having 8 or more and 12 or less carbon atoms, still more preferably an alkyl group having 10 or more and 12 or less carbon atoms, and particularly preferably an alkyl group having 11 carbon atoms. It is also preferred that the alkyl group is a linear alkyl group. It is also preferred that the alkyl group is a saturated alkyl group. It is also preferred that the alkyl group is an unsubstituted alkyl group. In the formula (a4),

[Formula 12]

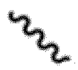

represents a bond.

It is more preferred that the surfactant according to one embodiment is a compound represented by the following general formula (a3):

[Formula 13]

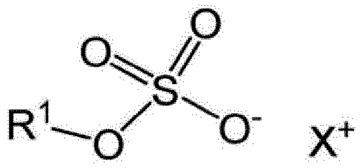

(a 3)

in the general formula (a3), $R^1$ represents an optionally substituted, saturated or unsaturated, linear hydrocarbon group having 5 or more and 17 or less carbon atoms, and X represents sodium or potassium.

In the surfactant according to one embodiment, it is preferred that $R^1$ in the above general formula (a3) is a saturated and linear hydrocarbon group having 5 or more and 17 or less carbon atoms.

The surfactant according to one embodiment may be an anionic surfactant, a cationic surfactant, an amphoteric surfactant, or a non-ionic surfactant. The surfactant is preferably an anionic surfactant or a cationic surfactant, more preferably a cationic surfactant. Examples of the anionic surfactant include a carboxylate, a sulfonate, and a sulfate, preferably a sulfonate, and further preferably sodium lauryl sulfate (sodium dodecyl sulfate).

The form of the pharmaceutical composition according to the present embodiments is not particularly limited, but is typically a solid. The pharmaceutical composition according to the present embodiments is molded into, for example, a powder, a subtle granule, a granule, a tablet, a coated tablet, a capsule, and the like, and used.

The pharmaceutical composition according to the present embodiments may be orally administered or may be parenterally administered. The pharmaceutical composition according to the present embodiments is preferably administered orally since the solubility of the substance to be dispersed (active ingredient) is improved.

There are no particular restrictions on the subject to which the pharmaceutical composition according to the present embodiments is administered, and it may be a human or a non-human animal. Examples of the non-human animal include a dog, a monkey, a mini-pig, a rabbit, a rat, and a mouse.

There are no particular restrictions on the dosage of the pharmaceutical composition according to the present embodiments, and it may be administered, for example, such that the dosage of the substance to be dispersed (active ingredient) per body weight (kg) of the subject is 0.1 mg/kg or more and 1,000 mg/kg or less. The dosage of the substance to be dispersed (active ingredient) may be, for example, 1 mg/kg or more and 500 mg/kg or less, 1 mg/kg or more and 100 mg/kg or less, 1 mg/kg or more and 50 mg/kg or less, 3 mg/kg or more and 30 mg/kg or less, 10 mg/kg or more and 30 mg/kg or less, 0.1 mg/kg or more and 10 mg/kg or less, 1 mg/kg or more and 5 mg/kg or less, 10 mg/kg or more and 100 mg/kg or less, 15 mg/kg or more and 50 mg/kg or less, 20 mg/kg or more and 40 mg/kg or less, 25 mg/kg or more and 35 mg/kg or less, 3 mg/kg, or 30 mg/kg.

The pharmaceutical composition according to the present embodiments can be produced by being molded into an arbitrary dosage form by a method that is commonly used.

The pharmaceutical composition according to the present embodiments may have a solubility of 10 mg/mL or less and more than 0 mg/mL in a 50 mM phosphate buffer solution (pH 6.5) at 25°C, 1 atm. The pharmaceutical composition according to the present embodiments may have a solubility of, for example, 5 mg/mL or less, 2.5 mg/mL or less, 2 mg/mL or less, 1 mg/mL or less, 0.5 mg/mL or less, 0.25 mg/mL or less, 0.1 mg/mL or less, 0.05 mg/mL or less, 0.025 mg/mL or less, 0.01 mg/mL or less, 0.005 mg/mL or less, 0.0025 mg/mL or less, or 0.001 mg/mL or less in a 50 mM phosphate buffer solution (pH 6.5) at 25°C, 1 atm. The solubility may be 0.0001 mg/mL or more, 0.0005 mg/mL or more, 0.001 mg/mL or more. The pharmaceutical composition according to the present embodiments has a solubility of, for example, more than 0 mg/mL and 10 mg/mL or less, preferably more than 0 mg/mL and 1 mg/mL or less, more preferably more than 0 mg/mL and 0.1 mg/mL or less, and most preferably more than 0 mg/mL and 0.05 mg/mL or less in a 50 mM phosphate buffer solution (pH 6.5) at 25°C, 1 atm. The solubility of the pharmaceutical composition in a 50 mM phosphate buffer solution (pH 6.5) at 25°C, 1 atm can be measured, for example, by the methods described in Examples below.

The pharmaceutical composition according to the present embodiments may be one in which, in a physical stability test under conditions of 40°C, 75%RH, a crystal is not observed until one month after starting the test. The physical stability test is a test performed by the method described in Examples below. Specifically, the physical stability test is a test in which the pharmaceutical composition is stored for a predetermined period of time under an environment maintained at a specific temperature and humidity, and after a predetermined period of time, the presence or absence of a halo pattern is checked, for example, by XRD measurement. Since the pharmaceutical composition according to the present embodiments contains the solid dispersion according to the present invention having excellent amorphous stability, the pharmaceutical composition according to the present embodiments can maintain the amorphous state until one month after starting the test under conditions of 40°C, 75%RH.

Similarly, the pharmaceutical composition according to the present embodiments may be one in which, in a physical stability test under conditions of 80°C, 75%RH, a crystal is not observed until one month after starting the test.

The pharmaceutical composition according to the present embodiments may have a disintegration time of 1,000 seconds or less in a disintegration test under conditions of using a second fluid for dissolution test (a fluid obtained by adding 118 mL of 0.2 M sodium hydroxide test solution and water to 250 mL of 0.2 M potassium dihydrogen phosphate test solution to be 1,000 mL, the pH of which is 6.7 or more and 6.9 or less) as a test solution, a test solution temperature of 37.0°C and an amplitude-frequency of 30 times/minute. The disintegration time may be, for example, 950 seconds or less, 900 seconds or less, 850 seconds or less, or 800 seconds or less. The pharmaceutical composition to be subjected to the disintegration test is preferably in the dosage form of a tablet.

[Examples]

Hereinafter, preferred specific aspects of the present invention are described by way of Examples, but the present invention is not limited thereto.

[Synthesis Example] Synthesis of cyclic peptide compounds

Cyclic peptide compounds I to V used in the present Examples are shown in Tables 4, 5, and 6 below. These compounds were synthesized by the method described in International Publication No. WO 2021/090855.

[Table 4]

| Compound No. | Structural formula |
|---|---|
| Compound I | 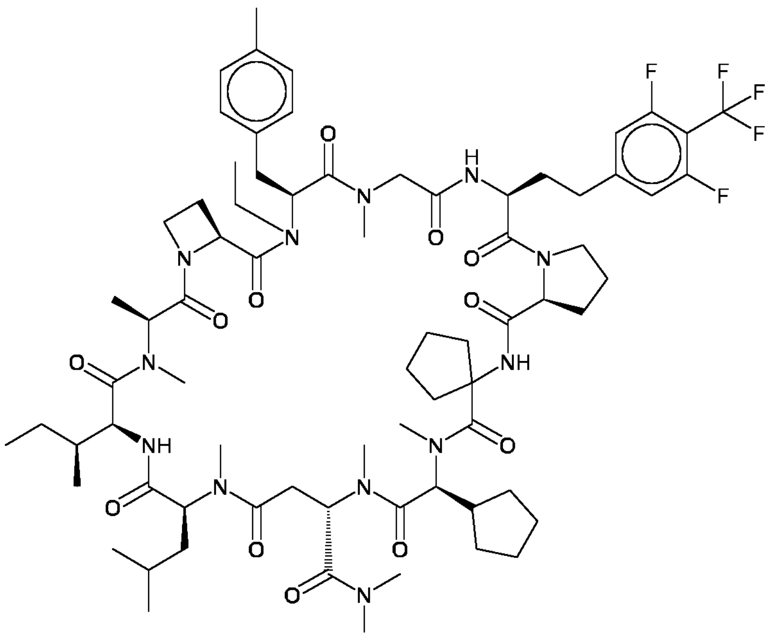 |

(continued)

| Compound No. | Structural formula |
|---|---|
| Compound II | 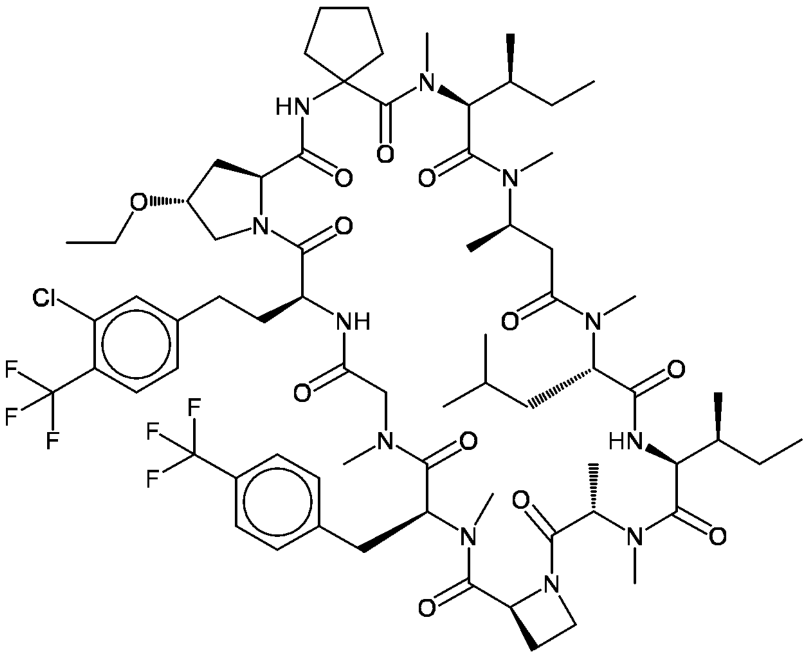 |

[Table 5]

| Compound No. | Structural formula |
|---|---|
| Compound III | 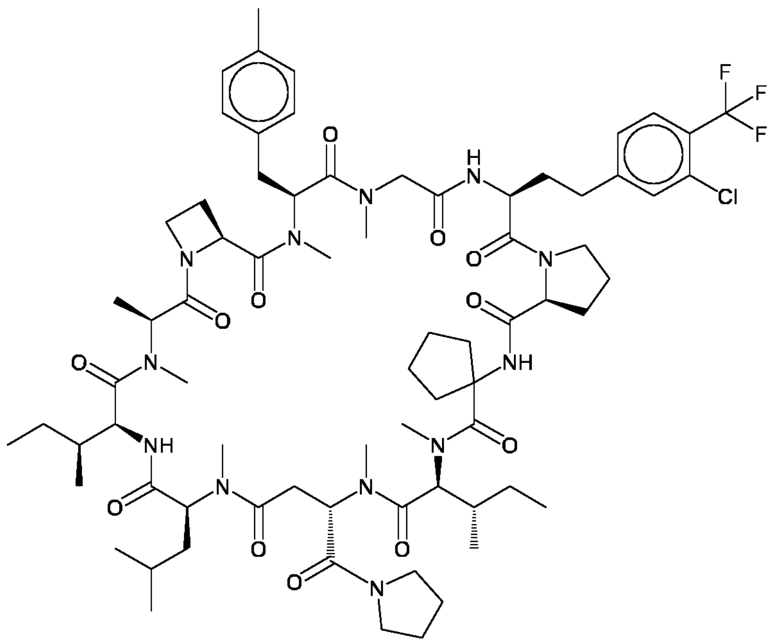 |

(continued)

| Compound No. | Structural formula |
|---|---|
| Compound IV | 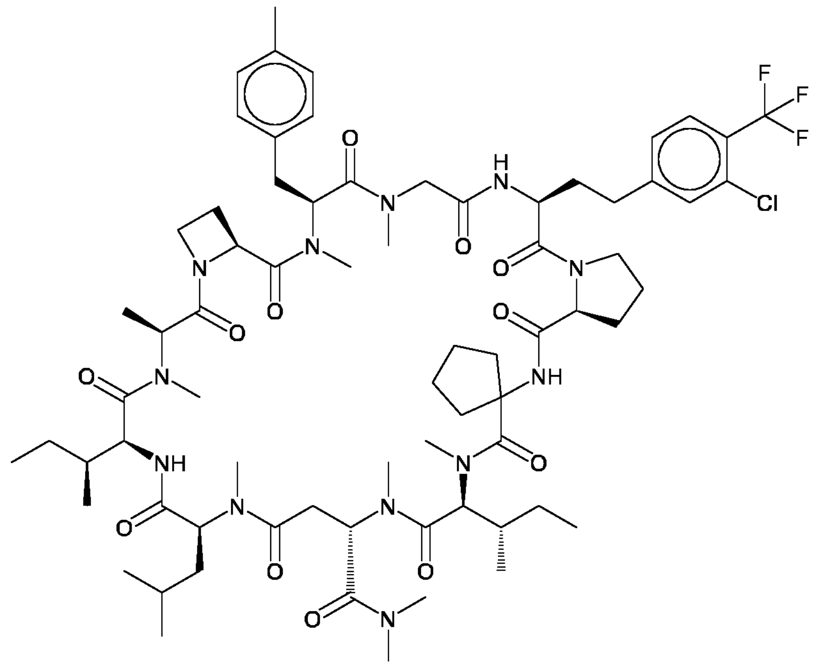 |

[Table 6]

| | |
|---|---|
| Compound V | 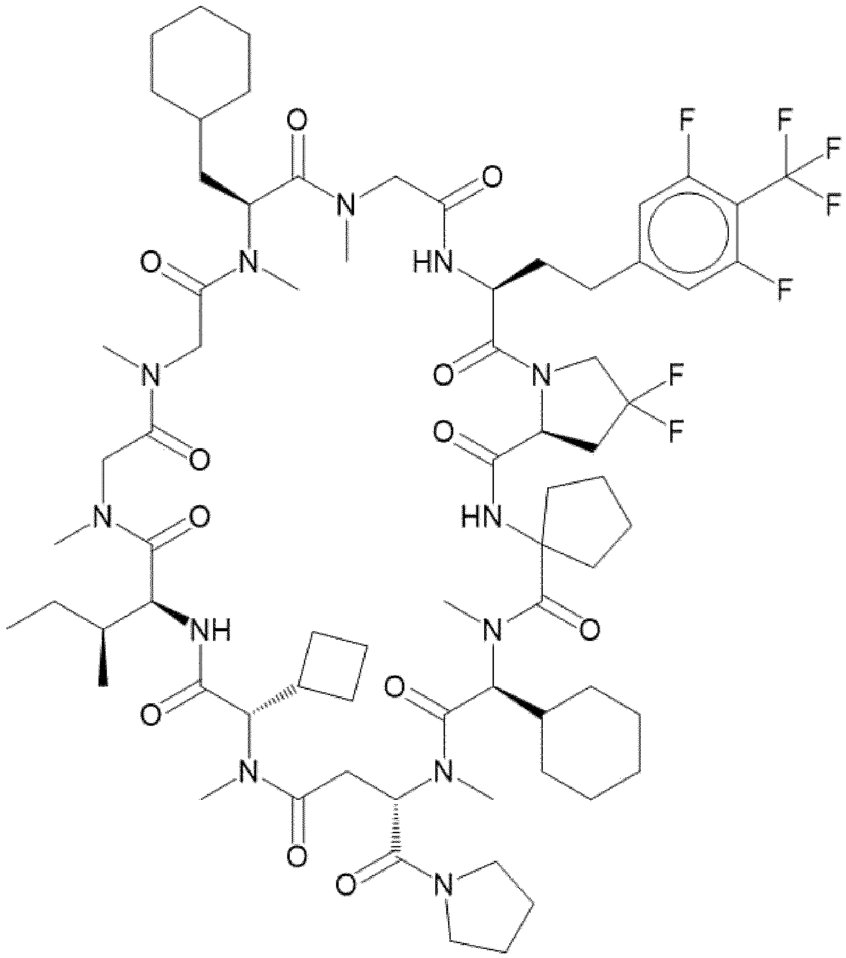 |

The reagents used in the present Examples are summarized in Table 7.

[Table 7]

| Abbreviation | Substance name | Grade, Source |
|---|---|---|
| HPMCAS | Hypromellose acetate succinate | ShinEtsu AQOAT LF, manufactured by Shin-Etsu Chemical Co., Ltd. |
| HPMC | Hydroxypropyl methyl cellulose | TC-5E, manufactured by Shin-Etsu Chemical Co., Ltd. |
| HPMCP55S | Hypromellose phthalate | 55S, manufactured by Shin-Etsu Chemical Co., Ltd. |
| Eudragit L100 | Methacrylic acid copolymer L | L100, manufactured by Evonik Industries AG |
| Eudragit L100-55 | Dried methacrylic acid copolymer LD | L100-55, manufactured by Evonik Industries AG |
| Eudragit EPO | Amino alkyl methacrylate copolymer E | EPO, manufactured by Evonik Industries AG |
| PVP K30 | Povidone | Kollidon 30, manufactured by BASF SE |
| PVP VA64 | Copolyvidone | Kollidon VA64, manufactured by BASF SE |
| HPC-L | Hydroxypropyl cellulose | L, manufactured by Nippon Soda Co., Ltd. |
| HPC-SSL | Hydroxypropyl cellulose | SSL, manufactured by Nippon Soda Co., Ltd. |

(continued)

| Abbreviation | Substance name | Grade, Source |
|---|---|---|
| Soluplus | Polyvinyl caprolactam-polyvinyl acetate-polyethylene glycol graft copolymer | manufactured by BASF SE |
| PVAP | Polyvinyl acetate phthalate | manufactured by Colorcon |
| - | Acetone | manufactured by JUNSEI CHEMICAL CO., LTD. |
| SLS | Sodium lauryl sulfate | KOLLIPHOR SLS FINE, manufactured by BASF SE |
| - | Mannitol | PARTECK M200, manufactured by MERCK MILLIPORE |
| - | Sodium croscarmellose | Ac-Di-Sol, manufactured by DuPont |
| - | Silicon dioxide | Adsolider101, manufactured by Freund Corporation |
| - | Magnesium stearate | manufactured by Mallinckrodt Pharma K.K. |

The molecular weights (unit: g/mol) of Compounds I to V and cyclosporine A are as follows:

Compound I: 1437.7

Compound II: 1451.1

Compound III: 1436.1

Compound IV: 1410.1

Compound V: 1463.7

Cyclosporine A: 1202.6

Tgc (unit: °C) of Compounds I to V and cyclosporine A are as follows:

Compound I: 164
Compound II: 140
Compound III: 157
Compound IV: 156
Compound V: 161
Cyclosporine A: 125

ClogP of Compounds I to V and cyclosporine A are as follows:

Compound I: 14.5
Compound II: 15.1
Compound III: 14.6
Compound IV: 14.2
Compound V: 15.1
Cyclosporine A: 14.4

The solubility (unit: mg/mL) of Compounds I to V and cyclosporine A in a 50 mM phosphate buffer solution (pH 6.5) at 25°C, 1 atm was measured by the following method.

To a powder of the compound, a 50 mM phosphate buffer solution (PB: Phosphate buffer, pH 6.5) was added, and after shaking under normal pressure (at 25°C, 2,000 rpm, 16 to 24 hours), the mixture was filtered through a filter, and the compound concentration of the filtrate was measured by HPLC. Solubility (unit: mg/mL) was calculated from the measured compound concentration. The HPLC conditions are as follows.

Detector: ultraviolet absorption photometer (measurement wavelength: 230 nm)
Column: stainless steel tube of 2.1 mm inner diameter, 5 cm long filled with 1.7 μm ethylene cross-linked hybrid particles for liquid chromatography
Column temperature: constant temperature near 70°C
Mobile phase A: water/trifluoroacetic acid mixed solution (2000/1)
Mobile phase B: acetonitrile/trifluoroacetic acid mixed solution (2000/1)
Liquid delivery of mobile phase: concentration gradient control by changing the mixing ratio of mobile phase A and mobile phase B as shown in Table 8 below

[Table 8]

| Time after injection (min) | Mobile phase A (vol%) | Mobile phase B (vol%) | Flow rate (mL/min) |
|---|---|---|---|
| 0→0.75 | 90→0 | 10→100 | 0.8 |
| 0.75→1.1 | 0 | 100 | 0.8 |
| 1.1→1.11 | 0→90 | 100→10 | 0.8 |
| 1.11→3 | 90 | 10 | 0.8 |

Injection volume: 10 μL
Sample temperature: constant temperature near 25°C.
The solubility (unit: mg/mL) of Compounds I to V and cyclosporine A in a 50 mM phosphate buffer solution (pH 6.5) at 25°C, 1 atm is as follows:

Compound I: less than 0.001
Compound II: 0.001
Compound III: 0.010
Compound IV: 0.020
Compound V: less than 0.001
Cyclosporine A: 0.011.

The weight average molecular weight of each polymer is as follows:

HPMCAS: 21,000-26,000
HPMC: 16,000-60,000
HPMCP55S: 37,500-60,500
Eudragit L100: 125,000
Eudragit L100-55: 320,000
Eydragit EPO: 47,000
PVP K30: 44,000-54,000
PVP VA 64: 45,000-70,000
HPC-L: 140,000
HPC-SSL: 40,000
Soluplus: 90,000-140,000
PVAP: 45,000-68,000.

Tgp (unit:°C) of each polymer is as follows:

HPMCAS: 118
HPMC: 128
HPMCP55S: 129
Eudragit L100: non available
Eudragit L100-55: 123
EudragitEPO: 49
PVP K30: 161
PVP VA 64: 108
HPC-L: non available
HPC-SSL: non available

Soluplus: 79
PVAP: 128.

(Example I-A-4)

5.0 g of Compound I was added to 108 mL of acetone with stirring at 25°C, 1 atm, and dissolved to a solid concentration of 6 wt/vol% (4.6 wt/vol % as Compound I concentration). Then 1.5 g of HPMCAS was added with stirring, and the resulting solution was spray-dried at an inlet temperature of 50-90°C (outlet temperature of 45-55°C), aspirator output of 100%, peristaltic pump output of 20-25%, and spray air flow meter height of 25-30 mm with a small spray dryer B-290 (manufactured by BUCHI), and the powder recovered after spray drying was dried at 60°C, 0 Pa overnight in a vacuum dryer to obtain 6.5 g of solid dispersion.

(Comparative Example V-J-4)

30 mg of HPC-SSL as a polymer was dissolved in 10 mL of pure water to a solid concentration of 0.3 wt/vol% at 25°C, 1 atm. 100 mg of amorphous cyclosporine A was then weighed and placed together with 30 g of zirconia beads (Zirconia oxide, 0.5 mm, NIKKATO CORPORATION) in a mixer vessel (150 mL volume, ARE-310, THINKY CORPORATION), and 0.3 mL of the polymer solution obtained above was added and mixed at 1,000 rpm for 2 minutes to obtain a suspension. Subsequently, 9.7 mL of the polymer solution was added to the mixer vessel and mixed at 2,000 rpm for 2 minutes. Finally, the mixture was mixed at 400 rpm for 1 minute to obtain a suspension. The obtained suspension was freeze-dried to obtain a solid dispersion.
It was mixed.

(Comparative Example V-G-4)

A solid dispersion was produced in the same manner as in Comparative Example V-J-4 except that PVP K30 was used instead of HPC-SSL as the polymer.
For other Examples, Reference Examples, and Comparative Examples, a solid dispersion was performed in the same manner as in Example I-A-4 except that the conditions were changed as shown in Tables 9 and 10 below (however, the contents of substance to be dispersed and polymer in Reference Examples are set values).

[Table 9]

| | Substance to be dispersed | Polymer | Production method | Solid concentration (wt/vol%) | Prepared batch amount (g) | Solvent type | Solvent amount (mL) | Substance to be dispersed content (wt%) | Polymer content (wt%) |
|---|---|---|---|---|---|---|---|---|---|
| Reference Example I-0 | Compound I | - | Spray drying | 6 | 1.0 | Acetone | 16.7 | 100 | 0 |
| Example I-A-1 | Compound I | HPMCAS | Spray drying | 6 | 5.0 | Acetone | 83.4 | 98.4 | 1.6 |
| Example I-A-2 | Compound I | HPMCAS | Spray drying | 6 | 5.1 | Acetone | 84.2 | 98 | 2 |
| Example I-A-3 | Compound I | HPMCAS | Spray drying | 6 | 5.5 | Acetone | 91.7 | 91.7 | 8.3 |
| Example I-A-4 | Compound I | HPMCAS | Spray drying | 6 | 6.5 | Acetone | 108 | 78.6 | 21.4 |
| Example I-A-5 | Compound I | HPMCAS | Spray drying | 6 | 7.5 | Acetone | 125 | 68.5 | 31.5 |
| Example I-A-6 | Compound I | HPMCAS | Spray drying | 6 | 8.0 | Acetone | 133 | 62.7 | 37.3 |
| Comparative Example I-A-i | Compound I | HPMCAS | Spray drying | 6 | 10.0 | Acetone | 167 | 48 | 52 |
| Comparative Example I-A-ii | Compound I | HPMCAS | Spray drying | 6 | 15.0 | Acetone | 250 | 33.2 | 66.8 |
| Example I-B-1 | Compound I | HPMC | Spray drying | 6 | 2.5 | Acetone | 41.7 | N.A. | N.A. |
| Example I-B-3 | Compound I | HPMC | Spray drying | 6 | 2.0 | 5 v/v% water-containing methanol | 36.7 | 93.7 | 6.3 |
| Example I-B-4 | Compound I | HPMC | Spray drying | 6 | 6.2 | Acetone | 103 | 81.5 | 18.5 |
| Example I-B-6 | Compound I | HPMC | Spray drying | 6 | 4.0 | Acetone | 66.7 | N.A. | N.A. |
| Example I-C-4 | Compound I | HPMCP55S | Spray drying | 6 | 3.9 | Acetone | 65 | 76 | 24 |

(continued)

| | Substance to be dispersed | Polymer | Production method | Solid concentration (wt/vol%) | Prepared batch amount (g) | Solvent type | Solvent amount (mL) | Substance to be dispersed content (wt%) | Polymer content (wt%) |
|---|---|---|---|---|---|---|---|---|---|
| Example I-D-3 | Compound I | EudragitL100 | Spray drying | 6 | 11.0 | Ethanol | 183 | 92.2 | 7.8 |
| Example I-D-4 | Compound I | EudragitL100 | Spray drying | 6 | 6.5 | Ethanol | 108 | 79 | 21 |
| Example I-D-5 | Compound I | EudragitL100 | Spray drying | 6 | 7.5 | Ethanol | 125 | 66.7 | 33.3 |
| Example I-D-6 | Compound I | EudragitL100 | Spray drying | 6 | 4.8 | Ethanol | 80 | N.A. | N.A. |
| Example I-E-1 | Compound I | EudragitL100-55 | Spray drying | 6 | 2.5 | Acetone | 41.7 | N.A. | N.A. |
| Example I-E-4 | Compound I | EudragitL100-55 | Spray drying | 6 | 6.2 | Acetone | 103 | 76.6 | 23.4 |
| Example I-E-6 | Compound I | EudragitL100-55 | Spray drying | 6 | 4.0 | Acetone | 66.7 | N.A. | N.A. |
| Example I-F-4 | Compound I | EudragitEPO | Spray drying | 6 | 3.9 | Ethanol | 65 | 74.7 | 25.3 |
| Example I-G-3 | Compound I | PVPK30 | Spray drying | 6 | 2.0 | Ethanol | 36.7 | 94.0 | 6.0 |
| Example I-G-4 | Compound I | PVPK30 | Spray drying | 6 | 6.2 | Ethanol | 103 | 74.2 | 25.8 |

[Table 10]

| | Substance to be dispersed | Polymer | Production method | Solid concentration (wt/vol%) | Prepared batch amount (g) | Solvent type | Solvent amount (mL) | Substance to be dispersed content (wt%) | Polymer content (wt%) |
|---|---|---|---|---|---|---|---|---|---|
| Example I-H-1 | Compound I | PVPVA64 | Spray drying | 6 | 2.5 | Acetone | 41.7 | N.A. | N.A. |
| Example I-H-3 | Compound I | PVPVA64 | Spray drying | 6 | 2.5 | Acetone | 45.8 | 93.3 | 6.7 |
| Example I-H-4 | Compound I | PVPVA64 | Spray drying | 6 | 6.5 | Acetone | 108 | 78.4 | 21.6 |
| Example I-H-6 | Compound I | PVPVA64 | Spray drying | 6 | 4.0 | Acetone | 66.7 | N.A. | N.A. |
| Example I-I-4 | Compound I | HPC-L | Spray drying | 6 | 3.9 | Ethanol | 65 | 74.7 | 25.3 |
| Example I-J-4 | Compound I | HPC-SSL | Spray drying | 6 | 3.9 | Ethanol | 65 | 75.4 | 24.6 |
| Example I-K-4 | Compound I | Soluplus | Spray drying | 6 | 2.0 | Ethanol | 43.3 | 79.1 | 20.9 |
| Example I-L-4 | Compound I | PVAP | Spray drying | 6 | 3.9 | Tetrahydrofuran | 65 | 74.5 | 25.5 |
| Reference Example II-0 | Compound II | - | Spray drying | 6 | 0.8 | Acetone | 12.5 | 100 | 0 |
| Example II-A-1 | Compound II | HPMCAS | Spray drying | 6 | 1.0 | Acetone | 16.7 | N.A. | N.A. |
| Example II-D-4 | Compound II | EudragitL100 | Spray drying | 6 | 1.3 | Ethanol | 21.7 | N.A. | N.A. |
| Reference Example III-0 | Compound III | - | Spray drying | 6 | 0.8 | Acetone | 12.5 | 100 | 0 |
| Example III-A-1 | Compound III | HPMCAS | Spray drying | 6 | 1.0 | Acetone | 16.7 | N.A. | N.A. |
| Example III-A-4 | Compound III | HPMCAS | Spray drying | 6 | 1.3 | Acetone | 21.7 | N.A. | N.A. |

(continued)

| | Substance to be dispersed | Polymer | Production method | Solid concentration (wt/vol%) | Prepared batch amount (g) | Solvent type | Solvent amount (mL) | Substance to be dispersed content (wt%) | Polymer content (wt%) |
|---|---|---|---|---|---|---|---|---|---|
| Reference Example IV-0 | Compound IV | - | Spray drying | 6 | 0.8 | Acetone | 12.5 | 100 | 0 |
| Example IV-A-4 | Compound IV | HPMCAS | Spray drying | 6 | 1.3 | Acetone | 21.7 | N.A. | N.A. |
| Reference Example V-0 | Cyclosporine A | - | Spray drying | 6 | 10.0 | Acetone | 167 | 100 | 0 |
| Example V-A-4 | Cyclosporine A | HPMCAS | Spray drying | 6 | 13.0 | Acetone | 217 | N.A. | N.A. |
| Example V-G-4 | Cyclosporine A | PVP K30 | Spray drying | 6 | 2.6 | Ethanol | 43.3 | 77.3 | 22.7 |
| Example V-J-4 | Cyclosporine A | HPC-SSL | Spray drying | 6 | 2.6 | Ethanol | 43.3 | 77.7 | 22.3 |
| Comparative Example V-G-4 | Cyclosporine A | PVP K30 | Freeze drying | N.A. | 0.1 | Water | 10 | 79.2 | 20.8 |
| Comparative Example V-J-4 | Cyclosporine A | HPC-SSL | Freeze drying | N.A. | 0.1 | Water | 10 | 70.9 | 29.1 |
| Example VI-A-4 | Compound V | HPMCAS | Spray drying | 6 | 1.2 | Acetone | 26 | 76.2 | 23.8 |

(Production Example I-A-4)

34.24 wt.% of the solid dispersion obtained in Example I-A-4 as the main drug, 26.34 wt.% of SLS as the surfactant, 27.29 wt.% of mannitol as the excipient, 10.54 wt.% of croscarmellose sodium as the disintegrant, 1 wt.% of silicon dioxide as the glidant, and 0.6 wt.% of magnesium stearate as the lubricant were mixed. The obtained mixed powder was then dry-granulated with a compression simulator HB100 (manufactured by Huxley Bertram Engineering Ltd.), dry-milled with a small scale hand mill (manufactured by Gerteis Maschinen + Process-engineering AG), and tableted with a compression simulator HB100 (manufactured by Huxley Bertram Engineering Ltd.) to obtain a tablet.

Specifically, when preparing a 2.67 g batch, 0.91 g of the solid dispersion obtained in Example I-A-4, 0.7 g of sodium lauryl sulfate, 0.73 g of mannitol, 0.28 g of sodium croscarmellose, 0.03 g of silicon dioxide, and 0.02 g of magnesium stearate were mixed. The obtained mixed powder was dry-granulated with a compression simulator HB100 at an initial pounder height of -2.0 mm, an upper pounder descent depth of 3.0 mm, a compression speed of 2.0 rpm, and a compression pressure of 4.0 kN. The granules obtained by dry-granulation were dry-milled with a small-scale hand mill (mesh size 1.00). After adding 0.002 g of magnesium stearate to the milled granule obtained by dry-sizing and mixing, a tablet was obtained by tableting with a compression simulator HB100 at an initial pounder height of -2.0 mm, an upper pounder descent depth of 3.0 mm, a compression speed of 30.0 rpm, a pre-compression pressure of 9.0 kN, and a compression pressure of 10.0 kN.

For the tablets using the solid dispersion obtained in other Examples and Comparative Examples as the main drug, the tablets were molded in the same manner as in Production Example I-A-4 except that the conditions were changed as shown in Tables 11 to 13 below.

[Table 11]

| | Main drug | | Main drug amount | Surfactant amount | Excipient amount | Disintegrant amount | Glidant amount | Lubricant amount | Prepared batch amount |
|---|---|---|---|---|---|---|---|---|---|
| | | | | SLS | Mannitol | Sodium croscarmellose | Silicon dioxide | Magnesium stearate | |
| Production Example I-A-1 | Solid dispersion obtained by Example I-A-1 | wt.% | 28.66 | 28.63 | 29.66 | 11.45 | 1 | 0.6 | |
| | | g | 0.76 | 0.76 | 0.79 | 0.31 | 0.03 | 0.02 | 2.67 |
| Production Example I-A-2 | Solid dispersion obtained by Example I-A-2 | wt.% | 28.84 | 28.55 | 29.58 | 11.42 | 1 | 0.6 | |
| | | g | 1.08 | 1.07 | 1.11 | 0.43 | 0.04 | 0.02 | 3.74 |
| Production Example I-A-3 | Solid dispersion obtained by Example I-A-3 | wt.% | 30.61 | 27.83 | 28.83 | 11.13 | 1 | 0.6 | |
| | | g | 0.82 | 0.74 | 0.77 | 0.3 | 0.03 | 0.02 | 2.67 |
| Production Example I-A-4 | Solid dispersion obtained by Example I-A-4 | wt.% | 34.24 | 26.34 | 27.29 | 10.54 | 1 | 0.6 | |
| | | g | 0.91 | 0.7 | 0.73 | 0.28 | 0.03 | 0.02 | 2.67 |
| Production Example I-A-5 | Solid dispersion obtained by Example I-A-5 | wt.% | 37.5 | 25 | 25.9 | 10 | 1 | 0.6 | |
| | | g | 1 | 0.67 | 0.69 | 0.27 | 0.03 | 0.02 | 2.67 |
| Production Example I-A-6 | Solid dispersion obtained by Example I-A-6 | wt.% | 39.01 | 24.38 | 25.26 | 9.75 | 1 | 0.6 | |
| | | g | 1.04 | 0.65 | 0.67 | 0.26 | 0.03 | 0.02 | 2.67 |
| Production Example I-A-i | Solid dispersion obtained by Comparative Example I-A-i | wt.% | 44.36 | 22.18 | 22.98 | 8.87 | 1 | 0.6 | |
| | | g | 1.58 | 0.79 | 0.82 | 0.32 | 0.04 | 0.02 | 3.56 |
| Production Example I-A-ii | Solid dispersion obtained by Comparative Example I-A-ii | wt.% | 54.3 | 18.1 | 18.75 | 7.24 | 1 | 0.6 | |
| | | g | 1.93 | 0.64 | 0.67 | 0.26 | 0.04 | 0.02 | 3.56 |
| Production Example I-B-3 | Solid dispersion obtained by Example I-B-3 | wt.% | 30.61 | 27.83 | 28.83 | 11.13 | 1 | 0.6 | |
| | | g | 0.54 | 0.50 | 0.51 | 0.20 | 0.02 | 0.01 | 1.78 |

(continued)

| | Main drug | | Main drug amount | Surfactant amount | Excipient amount | Disintegrant amount | Glidant amount | Lubricant amount | Prepared batch amount |
|---|---|---|---|---|---|---|---|---|---|
| | | | | SLS | Mannitol | Sodium croscarmellose | Silicon dioxide | Magnesium stearate | |
| Production Example I-B-4 | Solid dispersion obtained by Example I-B-4 | wt.% | 34.24 | 26.34 | 27.29 | 10.54 | 1 | 0.6 | |
| | | g | 0.91 | 0.7 | 0.73 | 0.28 | 0.03 | 0.02 | 2.67 |
| Production Example I-C-4 | Solid dispersion obtained by Example I-C-4 | wt.% | 34.24 | 26.34 | 27.29 | 10.54 | 1 | 0.6 | |
| | | g | 0.91 | 0.7 | 0.73 | 0.28 | 0.03 | 0.02 | 2.67 |

[Table 12]

| | Main drug | | Main drug amount | Surfactant amount | Excipient amount | Disintegrant amount | Glidant amount | Lubricant amount | Prepared batch amount |
|---|---|---|---|---|---|---|---|---|---|
| | | | | SLS | Mannitol | Sodium croscarmellose | Silicon dioxide | Magnesium stearate | |
| Production Example I-D-3 | Solid dispersion obtained by Example I-D-3 | wt.% | 30.61 | 27.83 | 28.83 | 11.13 | 1 | 0.6 | |
| | | g | 0.82 | 0.74 | 0.77 | 0.3 | 0.03 | 0.02 | 2.67 |
| Production Example I-D-4 | Solid dispersion obtained by Example I-D-4 | wt.% | 34.24 | 26.34 | 27.29 | 10.54 | 1 | 0.6 | |
| | | g | 0.91 | 0.7 | 0.73 | 0.28 | 0.03 | 0.02 | 2.67 |
| Production Example I-E-4 | Solid dispersion obtained by Example I-E-4 | wt.% | 34.24 | 26.34 | 27.29 | 10.54 | 1 | 0.6 | |
| | | 9 | 0.91 | 0.7 | 0.73 | 0.28 | 0.03 | 0.02 | 2.67 |
| Production Example I-F-4 | Solid dispersion obtained by Example I-F-4 | wt.% | 34.24 | 26.34 | 27.29 | 10.54 | 1 | 0.6 | |
| | | g | 0.91 | 0.7 | 0.73 | 0.28 | 0.03 | 0.02 | 2.67 |
| Production Example I-G-3 | Solid dispersion obtained by Example I-G-3 | wt.% | 30.61 | 27.83 | 28.83 | 11.13 | 1 | 0.6 | |
| | | g | 0.54 | 0.50 | 0.51 | 0.20 | 0.02 | 0.01 | 1.78 |
| Production Example I-G-4 | Solid dispersion obtained by Example I-G-4 | wt.% | 34.24 | 26.34 | 27.29 | 10.54 | 1 | 0.6 | |
| | | g | 0.91 | 0.7 | 0.73 | 0.28 | 0.03 | 0.02 | 2.67 |

[Table 13]

| | Main drug | | Main drug amount | Surfactant amount | Excipient amount | Disintegrant amount | Glidant amount | Lubricant amount | Prepared batch amount |
|---|---|---|---|---|---|---|---|---|---|
| | | | | SLS | Mannitol | Sodium croscarmellose | Silicon dioxide | Magnesium stearate | |
| Production Example I-H-3 | Solid dispersion obtained by Example I-H-3 | wt.% | 30.61 | 27.83 | 28.83 | 11.13 | 1 | 0.6 | |
| | | g | 0.54 | 0.50 | 0.51 | 0.20 | 0.02 | 0.01 | 1.78 |
| Production Example I-H-4 | Solid dispersion obtained by Example I-H-4 | wt.% | 34.24 | 26.34 | 27.29 | 10.54 | 1 | 0.6 | |
| | | g | 0.91 | 0.7 | 0.73 | 0.28 | 0.03 | 0.02 | 2.67 |
| Production Example I-I-4 | Solid dispersion obtained by Example I-I-4 | wt.% | 34.24 | 26.34 | 27.29 | 10.54 | 1 | 0.6 | |
| | | g | 0.91 | 0.7 | 0.73 | 0.28 | 0.03 | 0.02 | 2.67 |
| Production Example I-J-4 | Solid dispersion obtained by Example I-J-4 | wt.% | 34.24 | 26.34 | 27.29 | 10.54 | 1 | 0.6 | |
| | | g | 0.91 | 0.7 | 0.73 | 0.28 | 0.03 | 0.02 | 2.67 |
| Production Example I-K-4 | Solid dispersion obtained by Example I-K-4 | wt.% | 34.24 | 26.34 | 27.29 | 10.54 | 1 | 0.6 | |
| | | g | 0.61 | 0.47 | 0.49 | 0.19 | 0.02 | 0.01 | 1.78 |
| Production Example I-L-4 | Solid dispersion obtained by Example I-L-4 | wt.% | 34.24 | 26.34 | 27.29 | 10.54 | 1 | 0.6 | |
| | | g | 0.91 | 0.7 | 0.73 | 0.28 | 0.03 | 0.02 | 2.67 |
| Production Example II-D-4 | Solid dispersion obtained by Example II-D-4 | wt.% | 34.24 | 26.34 | 27.29 | 10.54 | 1 | 0.6 | |
| | | g | 0.48 | 0.37 | 0.38 | 0.15 | 0.01 | 0.01 | 1.39 |
| Production Example III-A-1 | Solid dispersion obtained by Example III-A-1 | wt.% | 28.66 | 28.63 | 29.66 | 11.45 | 1 | 0.6 | |
| | | g | 0.37 | 0.37 | 0.39 | 0.15 | 0.01 | 0.01 | 1.30 |
| Production Example III-A-4 | Solid dispersion obtained by Example III-A-4 | wt.% | 34.24 | 26.34 | 27.29 | 10.54 | 1 | 0.6 | |
| | | g | 0.48 | 0.37 | 0.38 | 0.15 | 0.01 | 0.01 | 1.39 |
| Production Example IV-A-4 | Solid dispersion obtained by Example IV-A-4 | wt.% | 34.24 | 26.34 | 27.29 | 10.54 | 1 | 0.6 | |
| | | g | 0.48 | 0.37 | 0.38 | 0.15 | 0.01 | 0.01 | 1.39 |

(continued)

| | Main drug | | Main drug amount | Surfactant amount | Excipient amount | Disintegrant amount | Glidant amount | Lubricant amount | Prepared batch amount |
|---|---|---|---|---|---|---|---|---|---|
| | | | | SLS | Mannitol | Sodium croscarmellose | Silicon dioxide | Magnesium stearate | |
| Production Example V-A-4 | Solid dispersion obtained by Example V-A-4 | wt.% | 34.24 | 26.34 | 27.29 | 10.54 | 1 | 0.6 | |
| | | g | 0.61 | 0.47 | 0.49 | 0.19 | 0.02 | 0.01 | 1.78 |
| Production Example VI-A-4 | Solid dispersion obtained by Example VI-A-4 | wt.% | 34.24 | 26.34 | 27.29 | 10.54 | 1 | 0.6 | |
| | | g | 0.61 | 0.47 | 0.49 | 0.19 | 0.02 | 0.01 | 1.78 |

(Measurement Example 1: Measurement of Crystalline State by X-ray Diffraction (XRD) Measuring Device)

The crystalline state of the solid dispersions obtained in the above Examples and Comparative Examples was measured by XRD under the following conditions.

Measuring device (model): Empyrean (manufactured by Malvern Panalytical Ltd.)
Counter cathode: Cu
Tube voltage: 45 kV
Tube current: 40 mA
Step width: 0.026°
scanning axis: 2θ
Scanning speed: 1.313°/s
Scanning range: 3 to 25°

The results are shown in Table 14 and Figures 1 to 7. Figures 1 to 7 are graphs showing examples of XRD measurement results. The XRD results showed that each of these solid dispersions exhibited a halo pattern characteristic of amorphous, indicating that the substances to be dispersed were present as amorphous in the solid dispersions.

[Table 14]

| Solid dispersion | Measurement result |
|---|---|
| Example I-A-1 | Halo pattern |
| Example I-A-2 | Halo pattern |
| Example I-A-3 | Halo pattern |
| Example I-A-4 | Halo pattern |
| Example I-A-5 | Halo pattern |
| Example I-A-6 | Halo pattern |
| Comparative Example I-A-i | Halo pattern |
| Comparative Example I-A-ii | Halo pattern |

(Measurement Example 2: Measurement of Content of Substance to be Dispersed by High-Performance Liquid Chromatography (HPLC))

The content of substance to be dispersed in the solid dispersions obtained in the above Examples and Comparative Examples was measured by HPLC under the following conditions.
Detector: ultraviolet absorption photometer (measurement wavelength: 230 nm)
Column: stainless steel tube of 2.1 mm inner diameter, 5 cm long filled with 1.7 μm ethylene cross-linked hybrid particles for liquid chromatography
Column temperature: constant temperature near 70°C
Mobile phase A: water/trifluoroacetic acid mixed solution (2000/1)
Mobile phase B: acetonitrile/trifluoroacetic acid mixed solution (2000/1)
Liquid delivery of mobile phase: concentration gradient control by changing the mixing ratio of mobile phase A and mobile phase B as shown in Table 15 below

[Table 15]

| Time after injection (min) | Mobile phase A (vol%) | Mobile phase B (vol%) | Flow rate (mL/min) |
|---|---|---|---|
| 0→0.75 | 90→0 | 10→100 | 0.8 |
| 0.75→1.1 | 0 | 100 | 0.8 |
| 1.1→1.11 | 0→90 | 100→10 | 0.8 |
| 1.11→3 | 90 | 10 | 0.8 |

Injection volume: 1-3 μL

Sample temperature: constant temperature near 25°C

The results are shown in Table 16. From the HPLC results, it was confirmed that the substances to be dispersed were contained in the solid dispersions in respective proportions.

[Table 16]

| | Substance to be dispersed | Polymer | Production method | Substance to be dispersed content (wt%) | Polymer content (wt%) |
|---|---|---|---|---|---|
| Example I-A-1 | Compound I | HPMCAS | Spray drying | 98.4 | 1.6 |
| Example I-A-2 | Compound I | HPMCAS | Spray drying | 98 | 2 |
| Example I-A-3 | Compound I | HPMCAS | Spray drying | 91.7 | 8.3 |
| Example I-A-4 | Compound I | HPMCAS | Spray drying | 78.6 | 21.4 |
| Example I-A-5 | Compound I | HPMCAS | Spray drying | 68.5 | 31.5 |
| Example I-A-6 | Compound I | HPMCAS | Spray drying | 62.7 | 37.3 |
| Comparative Example I-A-i | Compound I | HPMCAS | Spray drying | 48 | 52 |
| Comparative Example I-A-ii | Compound I | HPMCAS | Spray drying | 33.2 | 66.8 |
| Example I-B-3 | Compound I | HPMC | Spray drying | 93.7 | 6.3 |
| Example I-B-4 | Compound I | HPMC | Spray drying | 81.5 | 18.5 |
| Example I-C-4 | Compound I | HPMCP55S | Spray drying | 76 | 24 |
| Example I-D-3 | Compound I | EudragitL100 | Spray drying | 92.2 | 7.8 |
| Example I-D-4 | Compound I | EudragitL100 | Spray drying | 79 | 21 |
| Example I-D-5 | Compound I | EudragitL100 | Spray drying | 66.7 | 33.3 |
| Example I-E-4 | Compound I | EudragitL1 00-55 | Spray drying | 76.6 | 23.4 |
| Example I-F-4 | Compound I | EudragitEPO | Spray drying | 74.7 | 25.3 |
| Example I-G-3 | Compound I | PVPK30 | Spray drying | 94.0 | 6.0 |
| Example I-G-4 | Compound I | PVPK30 | Spray drying | 74.2 | 25.8 |
| Example I-H-3 | Compound I | PVPVA64 | Spray drying | 93.3 | 6.7 |
| Example I-H-4 | Compound I | PVPVA64 | Spray drying | 78.4 | 21.6 |
| Example I-I-4 | Compound I | HPC-L | Spray drying | 74.7 | 25.3 |
| Example I-J-4 | Compound I | HPC-SSL | Spray drying | 75.4 | 24.6 |
| Example I-K-4 | Compound I | Soluplus | Spray drying | 79.1 | 20.9 |
| Example I-L-4 | Compound I | PVAP | Spray drying | 74.5 | 25.5 |

(Measurement Example 3: Measurement of Glass Transition Temperature by Differential Scanning Calorimetry (DSC) Device)

The glass transition temperature of the solid dispersions obtained in the above Examples, Reference Examples, and Comparative Examples was measured by mDSC under the following conditions.

Measuring device (model): Q200 (manufactured by TA Instruments)
Heating rate: 5°C/min
Measurement temperature range: 35-200°C (220°C for Examples I-D-4, I-D-5, and I-D-6)
Ambient gas: dry nitrogen
Ambient gas flow rate: 50 mL/min
Cell: aluminum pan
Sample volume: 2-10 mg
Standard: empty container

The results are shown in Tables 17 to 18 and Figures 8 to 32. Figures 8 to 32 are graphs showing examples of DSC measurement results. From the results of mDSC, it was shown that each of the solid dispersions of Examples, Reference Examples and Comparative Examples obtained by spray drying has a unique and single glass transition temperature, respectively. In the solid dispersions obtained by spray drying in Examples, the glass transition temperature of Compounds I-V and cyclosporine A and the glass transition temperature of the obtained solid dispersion were different by 1°C or more, or the glass transition temperature of the obtained solid dispersion was 94.5% or more and 105.5% or less with respect to the value of Tga. From this, it was inferred that Compounds I to V and cyclosporine A were dispersed as uniform and very small units in the solid dispersion in a state mixed with the polymer. On the other hand, in the solid dispersions obtained by freeze-drying in Comparative Examples, the glass transition temperature of cyclosporine A and the glass transition temperature of the obtained solid dispersion were not different by 1°C or more, and the glass transition temperature of the obtained solid dispersion was not 94.5% or more and 105.5% or less with respect to the value of Tga. From this, it was inferred that cyclosporine A was not able to be dispersed as uniform and very small units in the solid dispersion.

[Table 17]

| | Substance to be dispersed | Polymer | Production method | Tg (°C) |
|---|---|---|---|---|
| Reference Example I-0 | Compound I | - | Spray drying | 164 |
| Example I-A-1 | Compound I | HPMCAS | Spray drying | 163 |
| Example I-A-2 | Compound I | HPMCAS | Spray drying | 163 |
| Example I-A-3 | Compound I | HPMCAS | Spray drying | 160 |
| Example I-A-4 | Compound I | HPMCAS | Spray drying | 154 |
| Example I-A-5 | Compound I | HPMCAS | Spray drying | 151 |
| Example I-A-6 | Compound I | HPMCAS | Spray drying | 149 |
| Comparative Example I-A-i | Compound I | HPMCAS | Spray drying | 145 |
| Comparative Example I-A-ii | Compound I | HPMCAS | Spray drying | 137 |
| Example I-B-1 | Compound I | HPMC | Spray drying | 164 |
| Example I-B-3 | Compound I | HPMC | Spray drying | 161 |
| Example I-B-4 | Compound I | HPMC | Spray drying | 159 |
| Example I-B-6 | Compound I | HPMC | Spray drying | 155 |
| Example I-C-4 | Compound I | HPMCP55S | Spray drying | 155 |
| Example I-D-3 | Compound I | EudragitL100 | Spray drying | 171 |
| Example I-D-4 | Compound I | EudragitL100 | Spray drying | 184 |
| Example I-D-5 | Compound I | EudragitL100 | Spray drying | 188 |
| Example I-D-6 | Compound I | EudragitL100 | Spray drying | 192 |
| Example I-E-1 | Compound I | EudragitL100-55 | Spray drying | 164 |
| Example I-E-4 | Compound I | EudragitL100-55 | Spray drying | 159 |
| Example I-E-6 | Compound I | EudragitL100-55 | Spray drying | 155 |
| Example I-F-4 | Compound I | EudragitEPO | Spray drying | 131 |
| Example I-G-3 | Compound I | PVPK30 | Spray drying | 162 |
| Example I-G-4 | Compound I | PVPK30 | Spray drying | 166 |

[Table 18]

| | Substance to be dispersed | Polymer | Production method | Tg (°C) |
|---|---|---|---|---|
| Example I-H-3 | Compound I | PVPVA64 | Spray drying | 159 |
| Example I-H-1 | Compound I | PVPVA64 | Spray drying | 164 |

(continued)

| | Substance to be dispersed | Polymer | Production method | Tg (°C) |
|---|---|---|---|---|
| Example I-H-4 | Compound I | PVPVA64 | Spray drying | 148 |
| Example I-H-6 | Compound I | PVPVA64 | Spray drying | 139 |
| Example I-I-4 | Compound I | HPC-L | Spray drying | 142 |
| Example I-J-4 | Compound I | HPC-SSL | Spray drying | 142 |
| Example I-K-4 | Compound I | Soluplus | Spray drying | 139 |
| Example I-L-4 | Compound I | PVAP | Spray drying | 149 |
| Reference Example II-0 | Compound II | - | Spray drying | 140 |
| Example II-A-1 | Compound II | HPMCAS | Spray drying | 140 |
| Example II-D-4 | Compound II | EudragitL100 | Spray drying | 167 |
| Reference Example III-0 | Compound III | - | Spray drying | 157 |
| Example III-A-1 | Compound III | HPMCAS | Spray drying | 156 |
| Example III-A-4 | Compound III | HPMCAS | Spray drying | 149 |
| Reference Example IV-0 | Compound IV | - | Spray drying | 156 |
| Example IV-A-4 | Compound IV | HPMCAS | Spray drying | 148 |
| Reference Example V-0 | Cyclosporine A | - | Spray drying | 125 |
| Example V-A-4 | Cyclosporine A | HPMCAS | Spray drying | 124 |
| Example V-G-4 | Cyclosporine A | PVP K30 | Spray drying | 137 |
| Example V-J-4 | Cyclosporine A | HPC-SSL | Spray drying | 113 |
| Comparative Example V-G-4 | Cyclosporine A | PVP K30 | Freeze drying | 125 |
| Comparative Example V-J-4 | Cyclosporine A | HPC-SSL | Freeze drying | 125 |
| Example VI-A-4 | Compound V | HPMCAS | Spray drying | 152 |

(Measurement Example 4: Measurement of Moisture Content by Thermogravimetric (TG-DTA) Device)

The results of measuring the moisture content of the solid dispersion obtained in the above Examples by TG-DTA are shown in Table 19. The results of TG-DTA showed that the solid dispersion obtained in each Example contained 1-2 wt.% moisture.

[Table 19]

| Solid dispersion | Measurement result |
|---|---|
| Example I-A-1 | 1.9 wt.% |
| Example I-A-2 | 1.8 wt.% |
| Example I-A-3 | 1.7 wt.% |
| Example I-A-4 | 1.3 wt.% |
| Example I-A-5 | 1.2 wt.% |
| Example I-A-6 | 1.9 wt.% |

(Evaluation Example 1: Physical Stability Test)

Table 20 shows the results of the physical stability test performed on the solid dispersions prepared in the above Examples and Comparative Examples. Physical stability was evaluated by XRD measurements after 1-month storage under the conditions of 40°C, 75%RH for the solid dispersions obtained in Examples and Comparative Examples other than Examples V-G-4 and V-J-4 and Comparative Examples V-G-4 and V-J-4, and after 1-month storage under the

conditions of 80°C, 75%RH for the solid dispersions obtained in Examples V-G-4 and V-J-4 and Comparative Examples V-G-4 and V-J-4. In the results of the XRD measurement after the physical stability test, the halo pattern characteristic of amorphous was shown in any of the solid dispersions, and in particular, the solid dispersion having a very small polymer content also showed sufficient physical stability. These examples show that, with the substance to be dispersed according to the present invention, a solid dispersion that has sufficient physical stability can be obtained even when the polymer content is greatly reduced.

[Table 20]

| | Substance to be dispersed | Polymer | Production method | Measurement result |
|---|---|---|---|---|
| Example I-A-1 | Compound I | HPMCAS | Spray drying | Halo pattern |
| Example I-A-2 | Compound I | HPMCAS | Spray drying | Halo pattern |
| Example I-A-3 | Compound I | HPMCAS | Spray drying | Halo pattern |
| Example I-A-4 | Compound I | HPMCAS | Spray drying | Halo pattern |
| Example I-A-5 | Compound I | HPMCAS | Spray drying | Halo pattern |
| Example I-A-6 | Compound I | HPMCAS | Spray drying | Halo pattern |
| Comparative Example I-A-i | Compound I | HPMCAS | Spray drying | Halo pattern |
| Comparative Example I-A-ii | Compound I | HPMCAS | Spray drying | Halo pattern |
| Example I-B-4 | Compound I | HPMC | Spray drying | Halo pattern |
| Example I-C-4 | Compound I | HPMCP55S | Spray drying | Halo pattern |
| Example I-D-4 | Compound I | EudragitL100 | Spray drying | Halo pattern |
| Example I-E-4 | Compound I | EudragitL100-55 | Spray drying | Halo pattern |
| Example I-F-4 | Compound I | EudragitEPO | Spray drying | Halo pattern |
| Example I-G-4 | Compound I | PVPK30 | Spray drying | Halo pattern |
| Example I-H-4 | Compound I | PVPVA64 | Spray drying | Halo pattern |
| Example I-I-4 | Compound I | HPC-L | Spray drying | Halo pattern |
| Example I-J-4 | Compound I | HPC-SSL | Spray drying | Halo pattern |
| Example I-K-4 | Compound I | Soluplus | Spray drying | Halo pattern |
| Example I-L-4 | Compound I | PVAP | Spray drying | Halo pattern |
| Example V-G-4 | Cyclosporine A | PVP K30 | Spray drying | Halo pattern |
| Example V-J-4 | Cyclosporine A | HPC-SSL | Spray drying | Halo pattern |
| Comparative Example V-G-4 | Cyclosporine A | PVP K30 | Freeze drying | Halo pattern |
| Comparative Example V-J-4 | Cyclosporine A | HPC-SSL | Freeze drying | Halo pattern |

(Evaluation Example 2: Disintegration Test)

Table 21 shows the evaluation results of the disintegration test for the tablets prepared in the above Production Examples. The disintegration test was performed in the conditions in which a second fluid for dissolution test was used as a test solution, a test solution temperature was 37.0°C, an amplitude-frequency was 30 times/minute, and each sample was n = 3 tablets, and the time when the tablet disappeared from the basket was taken as the measurement endpoint. As a result of the disintegration test, it was observed that the lower the polymer content in the solid dispersion, the shorter the disintegration time of the tablet. These examples show that the disintegration of the tablet can be greatly improved by reducing the polymer content in the solid dispersion.

[Table 21]

| | Disintegration (s) |
|---|---|
| Production Example I-A-1 | 457 |

(continued)

| | Disintegration (s) |
|---|---|
| Production Example I-A-2 | 536 |
| Production Example I-A-3 | 549 |
| Production Example I-A-4 | 603 |
| Production Example I-A-5 | 706 |
| Production Example I-A-6 | 797 |
| Production Example I-A-i | 1034 |
| Production Example I-A-ii | 1373 |
| Production Example I-B-3 | 492 |
| Production Example I-B-4 | 681 |
| Production Example I-C-4 | 579 |
| Production Example I-D-3 | 440 |
| Production Example I-D-4 | 495 |
| Production Example I-E-4 | 565 |
| Production Example I-F-4 | 547 |
| Production Example I-G-3 | 478 |
| Production Example I-G-4 | 595 |
| Production Example I-H-3 | 599 |
| Production Example I-H-4 | 764 |
| Production Example I-I-4 | 697 |
| Production Example I-J-4 | 643 |
| Production Example I-K-4 | 558 |
| Production Example I-L-4 | 492 |
| Production Example II-D-4 | 528 |
| Production Example III-A-1 | 473 |
| Production Example III-A-4 | 700 |
| Production Example IV-A-4 | 743 |
| Production Example V-A-4 | 699 |
| Production Example VI-A-4 | 536 |

(Evaluation Example 3: Bulk Density)

Table 22 shows the results of bulk density measured on the solid dispersions prepared in the above Examples and Comparative Examples. The test was performed by measuring the volume of powder obtained by gently filling 0.5 g of the measurement sample into a 5 mL measuring cylinder, and calculating a bulk density from the obtained volume. As a result of measuring the bulk density, it was observed that the bulk density of the solid dispersion prepared by spray drying was higher than that of the solid dispersion prepared by freeze-drying. This result shows that obtaining a solid dispersion by spray drying improves the ease of handling as a powder.

[Table 22]

| | Substance to be dispersed | Polymer | Production method | Bulk density (g/mL) |
|---|---|---|---|---|
| Example I-A-1 | Compound I | HPMCAS | Spray drying | 0.21 |

(continued)

| | Substance to be dispersed | Polymer | Production method | Bulk density (g/mL) |
|---|---|---|---|---|
| Example I-A-3 | Compound I | HPMCAS | Spray drying | 0.22 |
| Example I-A-4 | Compound I | HPMCAS | Spray drying | 0.19 |
| Example I-B-4 | Compound I | HPMC | Spray drying | 0.25 |
| Example I-E-4 | Compound I | EudragitL100-55 | Spray drying | 0.19 |
| Example VI-A-4 | Compound V | HPMCAS | Spray drying | 0.20 |
| Example V-A-4 | Cyclosporine A | HPMCAS | Spray drying | 0.23 |
| Example V-G-4 | Cyclosporine A | PVP K30 | Spray drying | 0.23 |
| Example V-J-4 | Cyclosporine A | HPC-SSL | Spray drying | 0.22 |
| Comparative Example V-G-4 | Cyclosporine A | PVP K30 | Freeze drying | 0.08 |
| Comparative Example V-J-4 | Cyclosporine A | HPC-SSL | Freeze drying | 0.12 |

**Claims**

1. A solid dispersion comprising: (1) a substance to be dispersed that has a molecular weight of 1,000 g/mol or more and 5,000 g/mol or less and is amorphous; and (2) a polymer,
wherein a ratio of a total mass of the polymer to a total mass of the substance to be dispersed is less than 1.0, and the solid dispersion meets the following (I) or (II):

(I) a glass transition temperature of the substance to be dispersed and a glass transition temperature of the solid dispersion are different by 1°C or more;
(II) the glass transition temperature of the solid dispersion is 94.5% or more and 105.5% or less with respect to a value of Tga represented by the following formula (A);
[Expression 1]

$$Tga = \frac{Wc}{Wa} \times Tgc + \frac{Wp}{Wa} \times Tgp \quad \ldots \text{Formula (A)}$$

in the formula (A), Tgc represents the glass transition temperature of the substance to be dispersed, Tgp represents a glass transition temperature of the polymer, Wa represents the total mass of the solid dispersion, Wc represents the total mass of the substance to be dispersed contained in the solid dispersion, and Wp represents the total mass of the polymer contained in the solid dispersion, respectively.

2. The solid dispersion according to claim 1, wherein the glass transition temperature of the solid dispersion is 100°C or higher and 200°C or lower.

3. The solid dispersion according to claim 1 or 2, wherein a content of the substance to be dispersed in the solid dispersion is more than 50% by mass and less than 100% by mass based on a total amount of the solid dispersion.

4. The solid dispersion according to any one of claims 1 to 3, wherein the glass transition temperature of the substance to be dispersed exceeds the glass transition temperature of the polymer.

5. The solid dispersion according to any one of claims 1 to 4, wherein the glass transition temperature of the substance to be dispersed is 90°C or higher and 180°C or lower.

6. The solid dispersion according to any one of claims 1 to 5, wherein the substance to be dispersed is a peptide compound.

7. The solid dispersion according to claim 6, wherein the number of amino acid residues constituting the peptide compound is 5 or more and 30 or less.

8. The solid dispersion according to claim 6 or 7, wherein the number of N-substituted amino acid residues contained in the peptide compound is 1 or more.

9. The solid dispersion according to any one of claims 1 to 8, wherein the glass transition temperature of the polymer is 30°C or higher and 170°C or lower.

10. The solid dispersion according to any one of claims 1 to 9, wherein a weight average molecular weight of the polymer is 3,000 or more and 400,000 or less.

11. The solid dispersion according to any one of claims 1 to 10, wherein a bulk density calculated from a volume of powder obtained by gently filling 0.5 g of the solid dispersion into a 5 mL measuring cylinder is 0.15 g/mL or more.

12. A pharmaceutical composition comprising the solid dispersion according to any one of claims 1 to 11, wherein a content of the solid dispersion is 99% by mass or less based on a total amount of the pharmaceutical composition.

13. The pharmaceutical composition according to claim 12, wherein, in a physical stability test under conditions of 40°C, 75%RH, a crystal is not observed until one month after starting the test.

14. The pharmaceutical composition according to claim 12 or 13, wherein a disintegration time in a disintegration test under conditions of using a second fluid for dissolution test as a test solution, a test solution temperature of 37.0°C and an amplitude-frequency of 30 times/minute is 1,000 seconds or less.

15. A method for producing a solid dispersion, comprising a step of evaporating a solvent in a mixture containing (1) a substance to be dispersed that has a molecular weight of 1,000 g/mol or more and 5,000 g/mol or less and is amorphous and (2) a polymer in an amount such that a ratio of a total mass of the polymer to a total mass of the substance to be dispersed is less than 1.0 by spray drying.

*Fig.1*

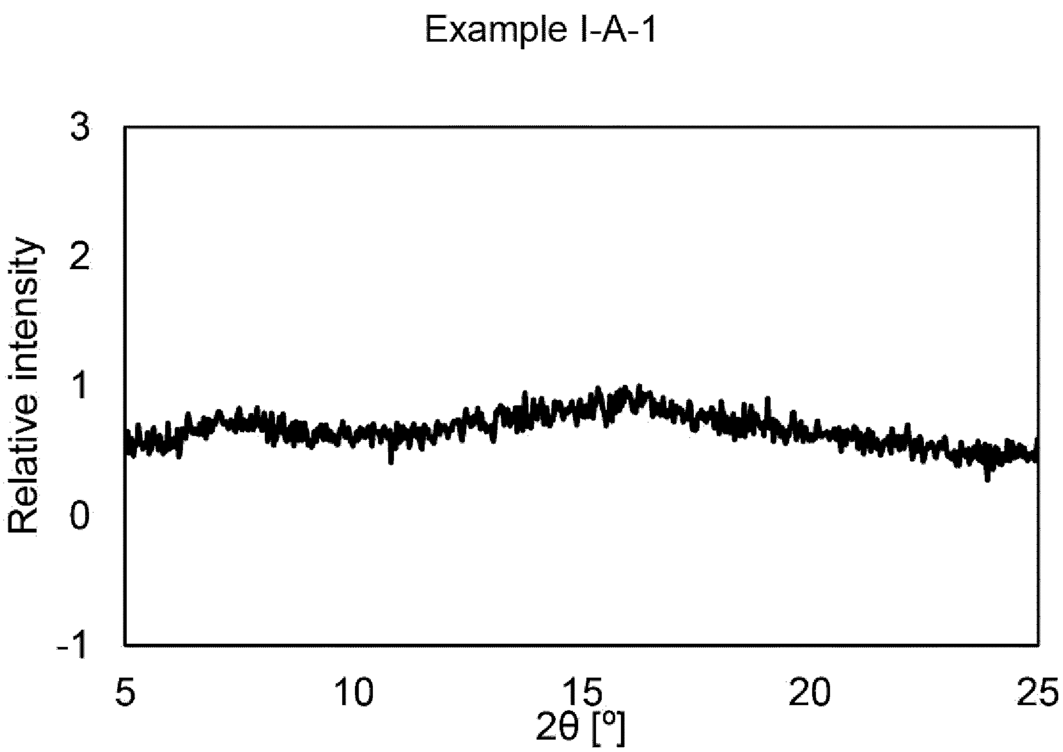

# Fig.2

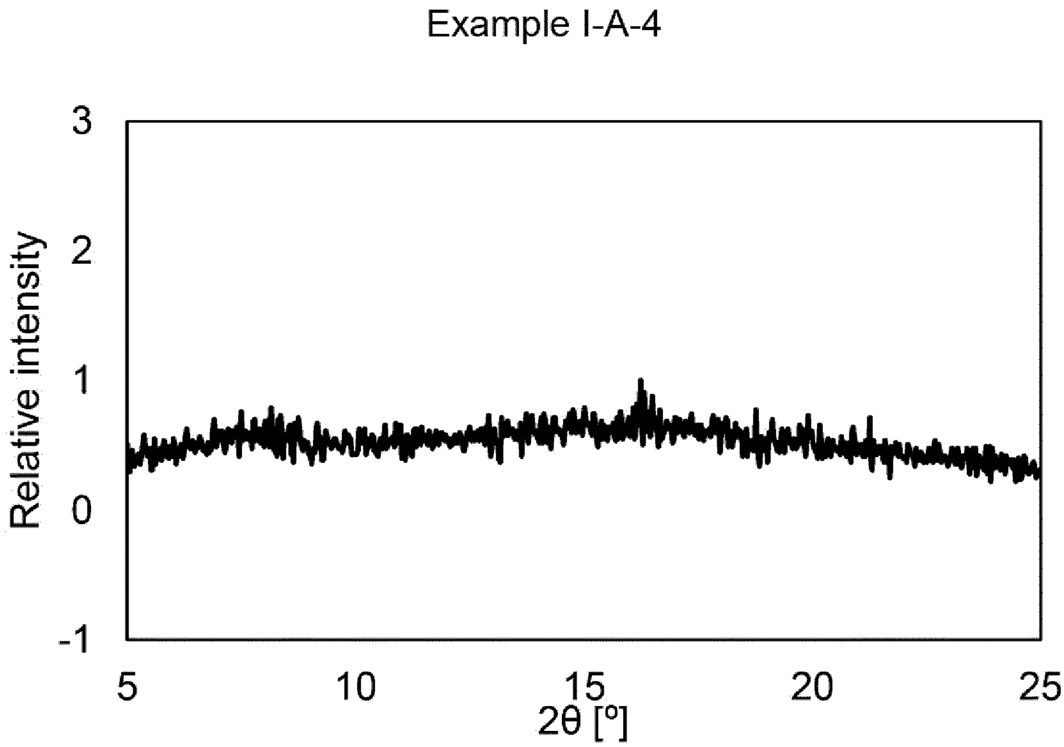

*Fig.3*

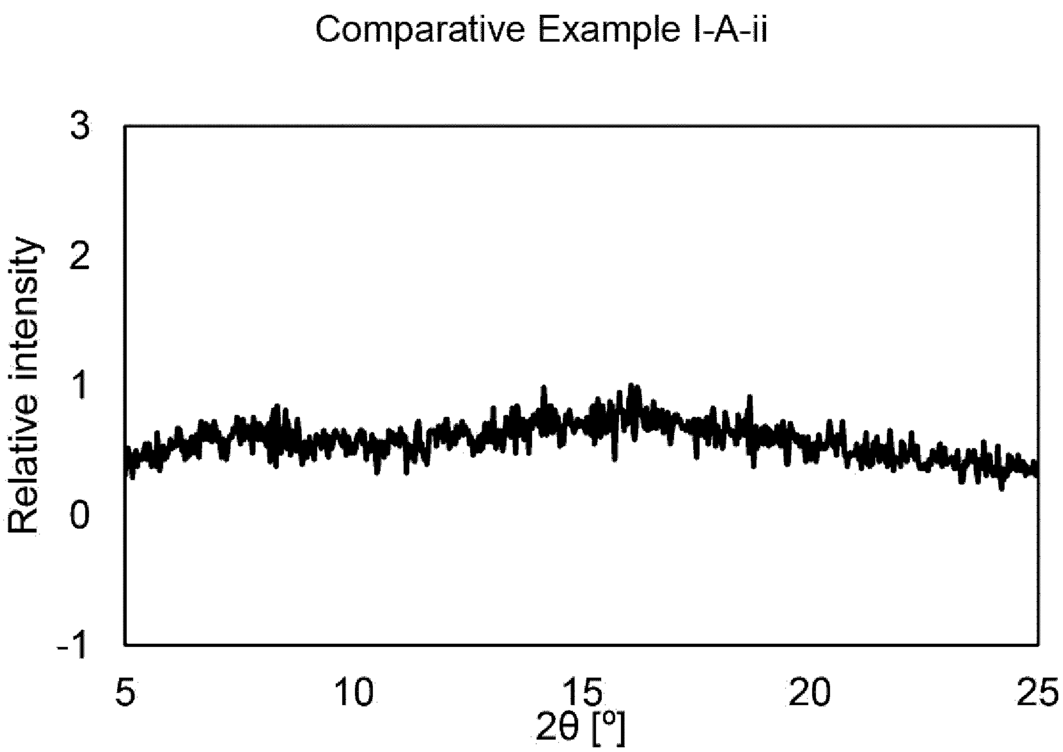
Comparative Example I-A-ii
3
2
1
0
-1
Relative intensity
5
10
15
20
25
2θ [°]

*Fig.4*

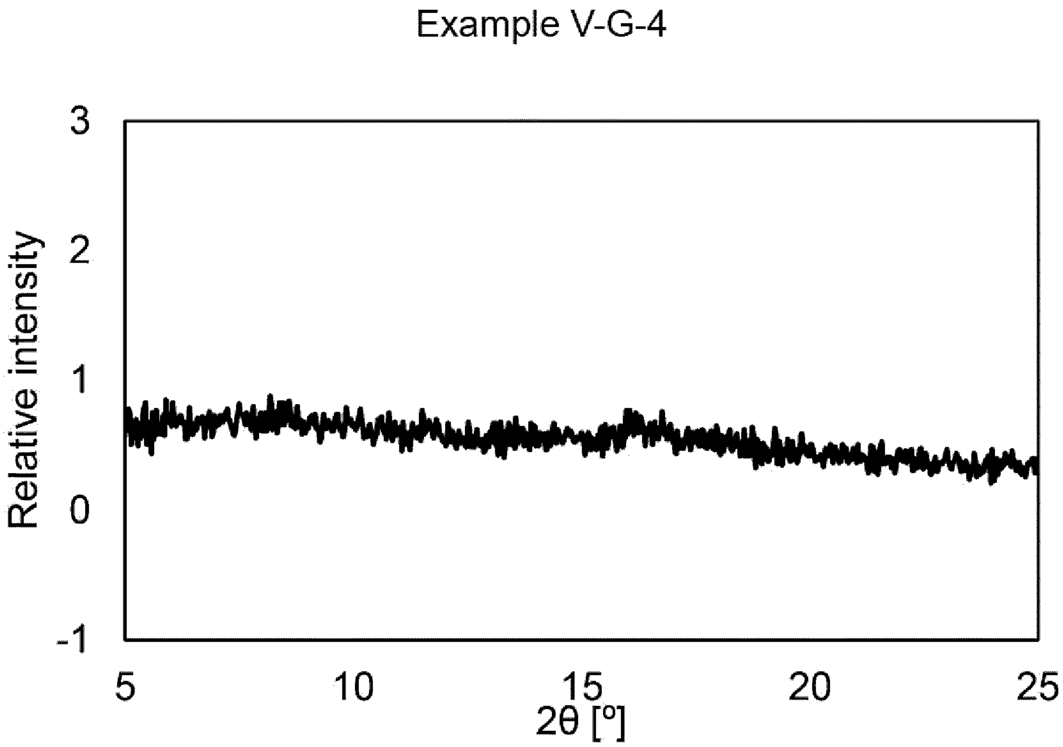

*Fig.5*

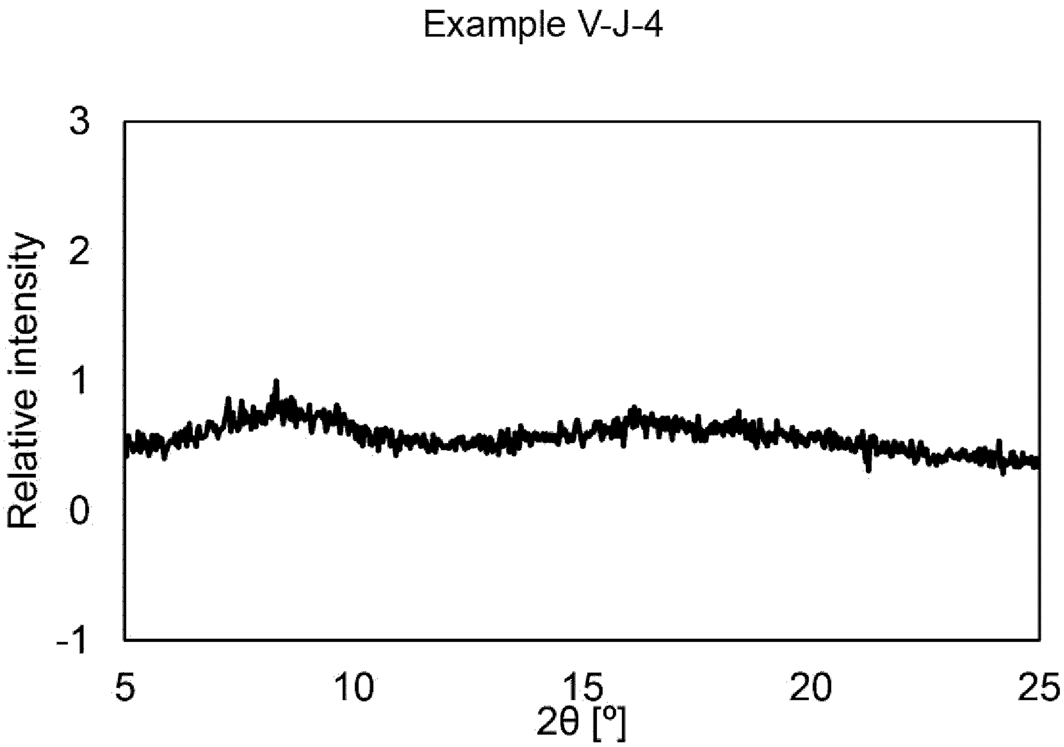

*Fig.6*

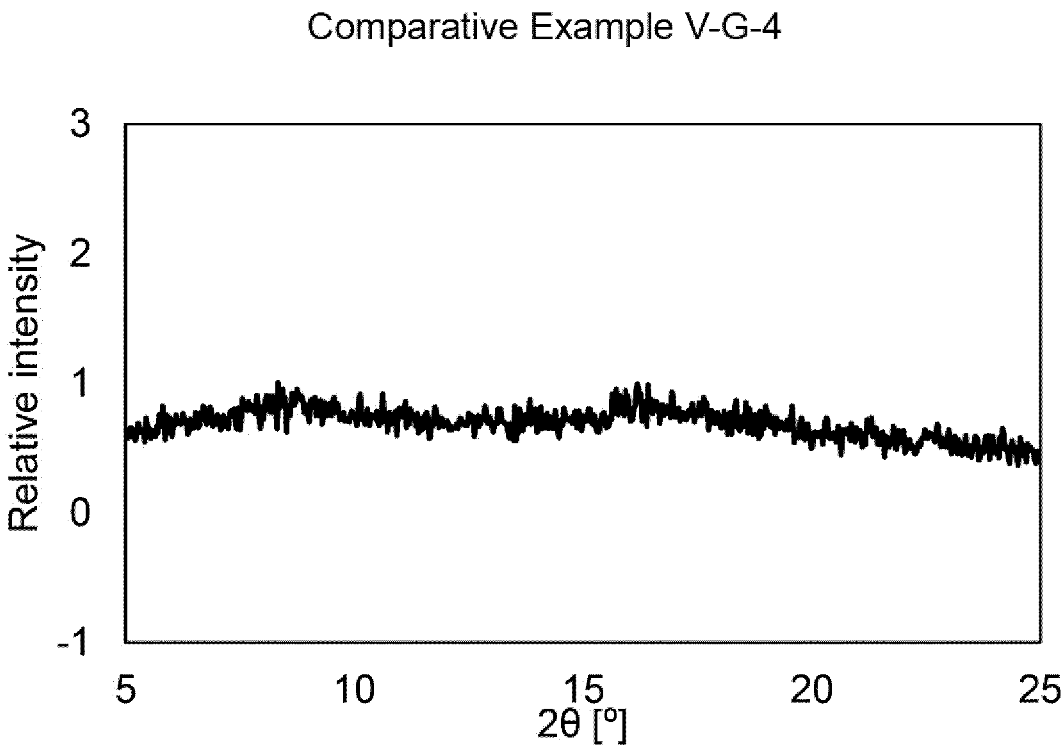
Comparative Example V-G-4
Relative intensity
3
2
1
0
-1
5
10
15
20
25
2θ [°]

Fig.7

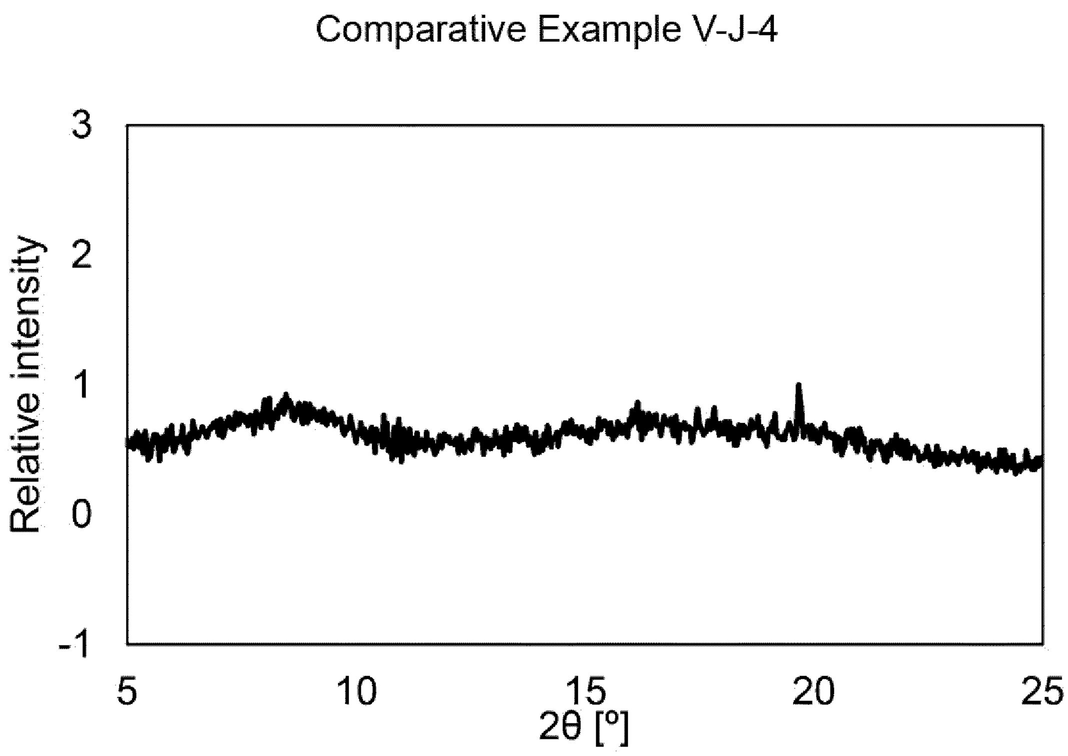
Comparative Example V-J-4
Relative intensity
3
2
1
0
-1
5
10
15
20
25
2θ [°]

# Fig.8

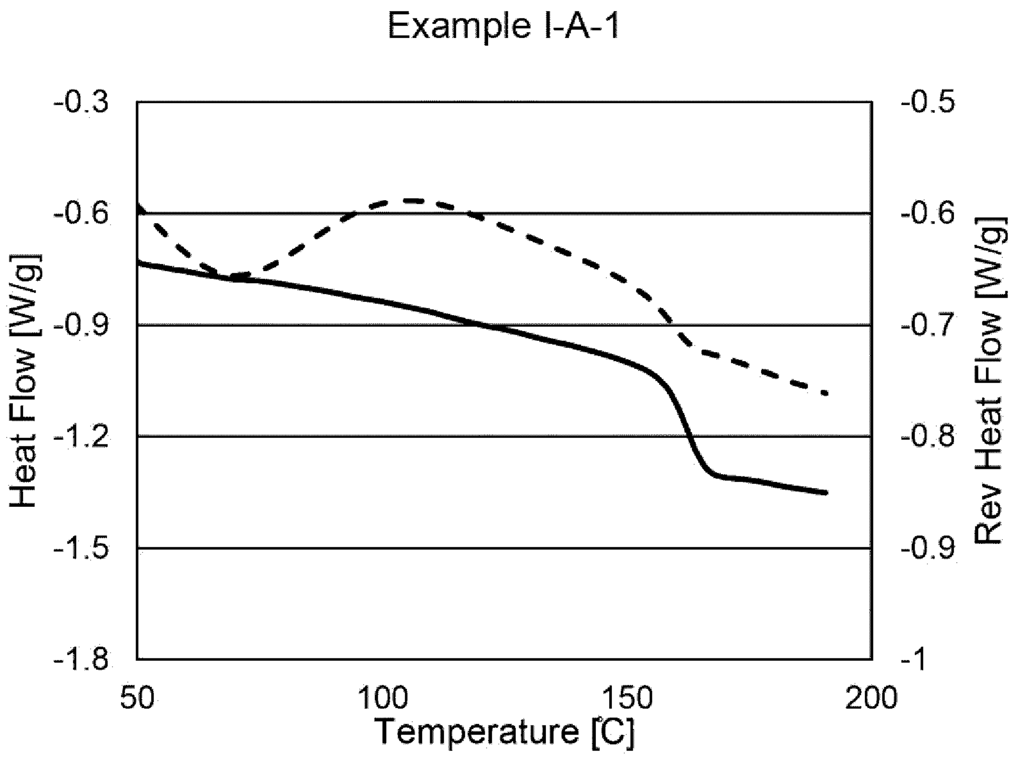
Example I-A-1
-0.3
-0.6
-0.9
-1.2
-1.5
-1.8
Heat Flow [W/g]
-0.5
-0.6
-0.7
-0.8
-0.9
-1
Rev Heat Flow [W/g]
50
100
150
200
Temperature [℃]

*Fig.9*

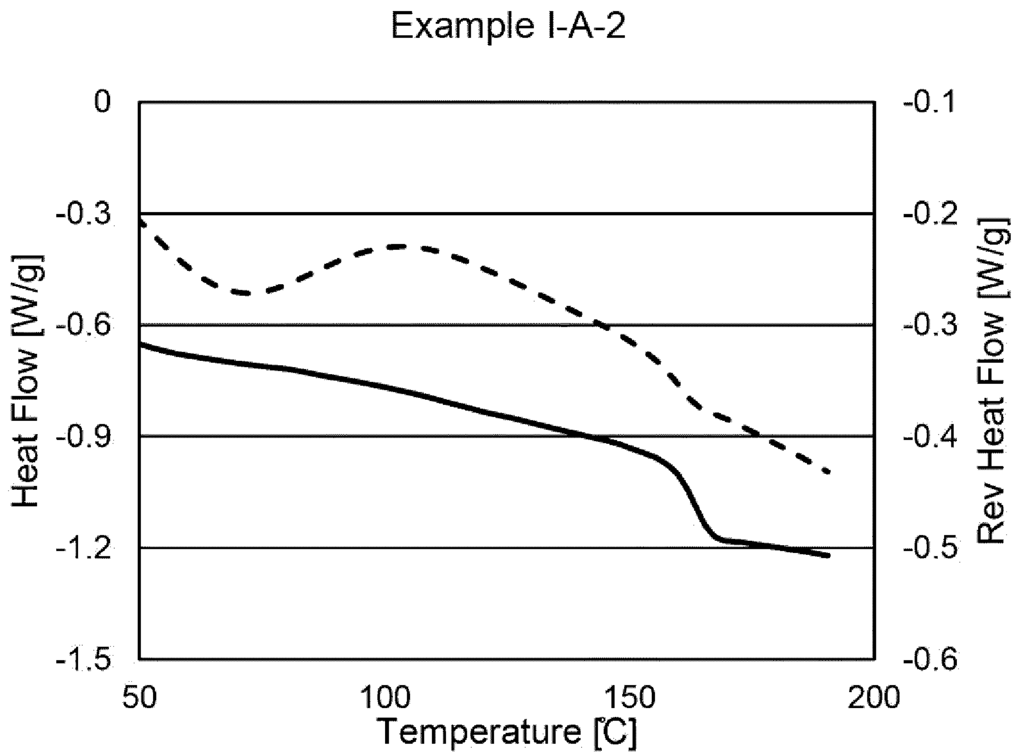
Example I-A-2
0
-0.3
-0.6
-0.9
-1.2
-1.5
-0.1
-0.2
-0.3
-0.4
-0.5
-0.6
50
100
150
200
Temperature [℃]
Heat Flow [W/g]
Rev Heat Flow [W/g]

# Fig.10

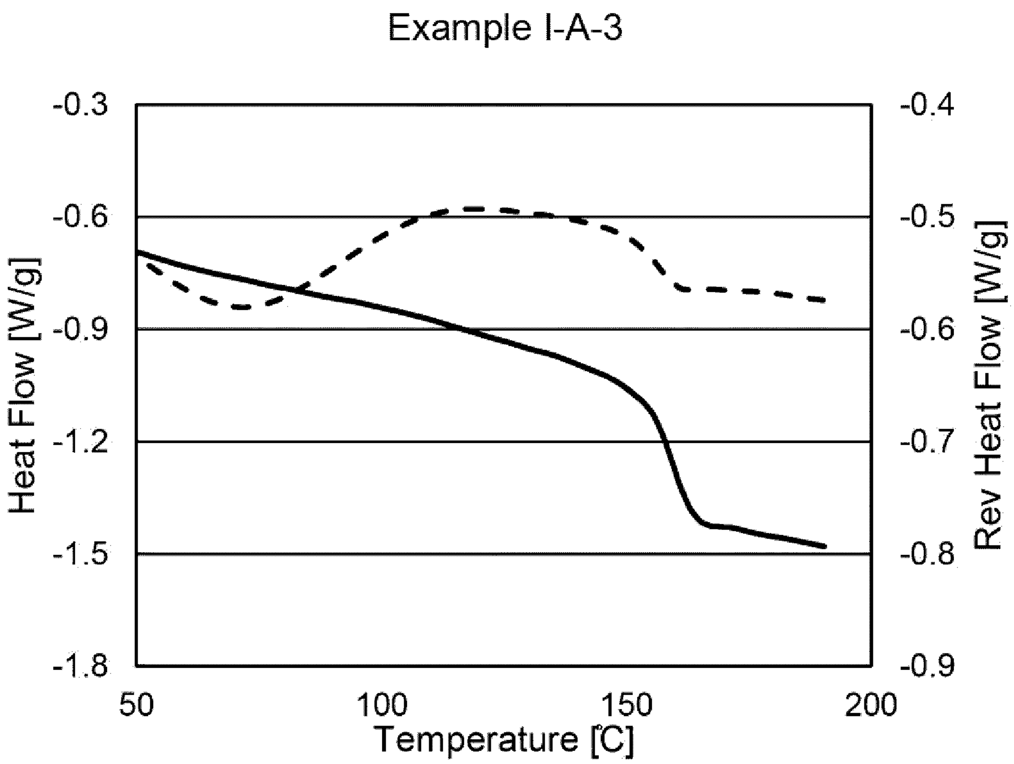
Example I-A-3
-0.3
-0.6
-0.9
-1.2
-1.5
-1.8
Heat Flow [W/g]
-0.4
-0.5
-0.6
-0.7
-0.8
-0.9
Rev Heat Flow [W/g]
50
100
150
200
Temperature [℃]

# Fig.11

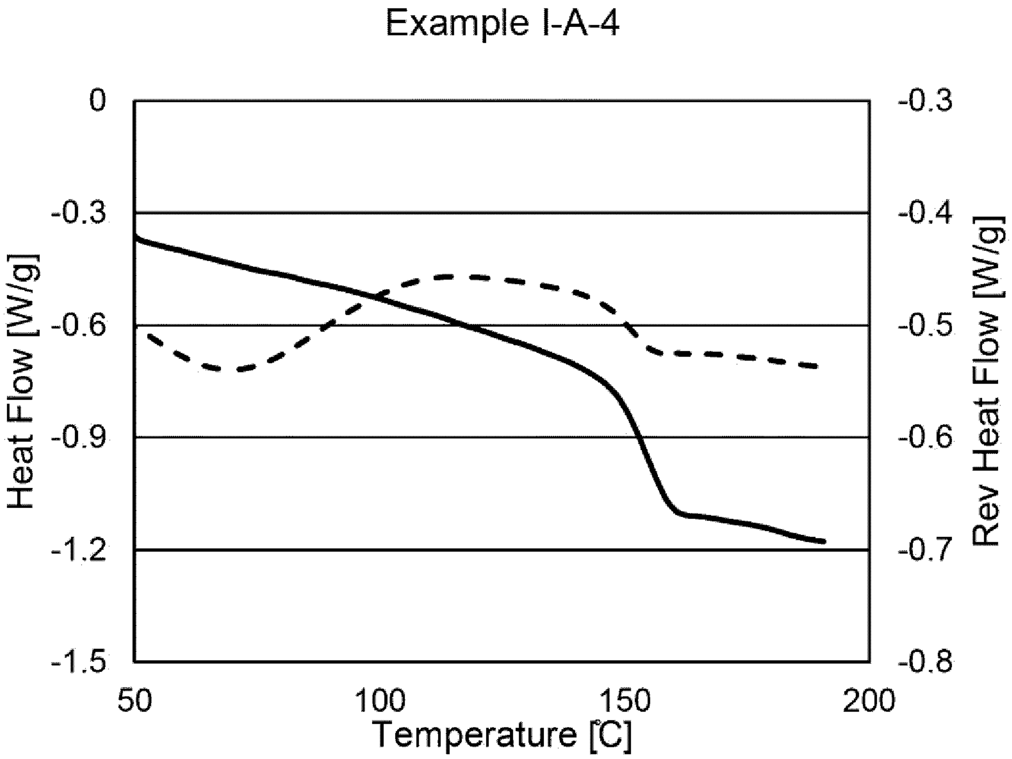
Example I-A-4
0
-0.3
-0.6
-0.9
-1.2
-1.5
Heat Flow [W/g]
-0.3
-0.4
-0.5
-0.6
-0.7
-0.8
Rev Heat Flow [W/g]
50
100
150
200
Temperature [℃]

# Fig.12

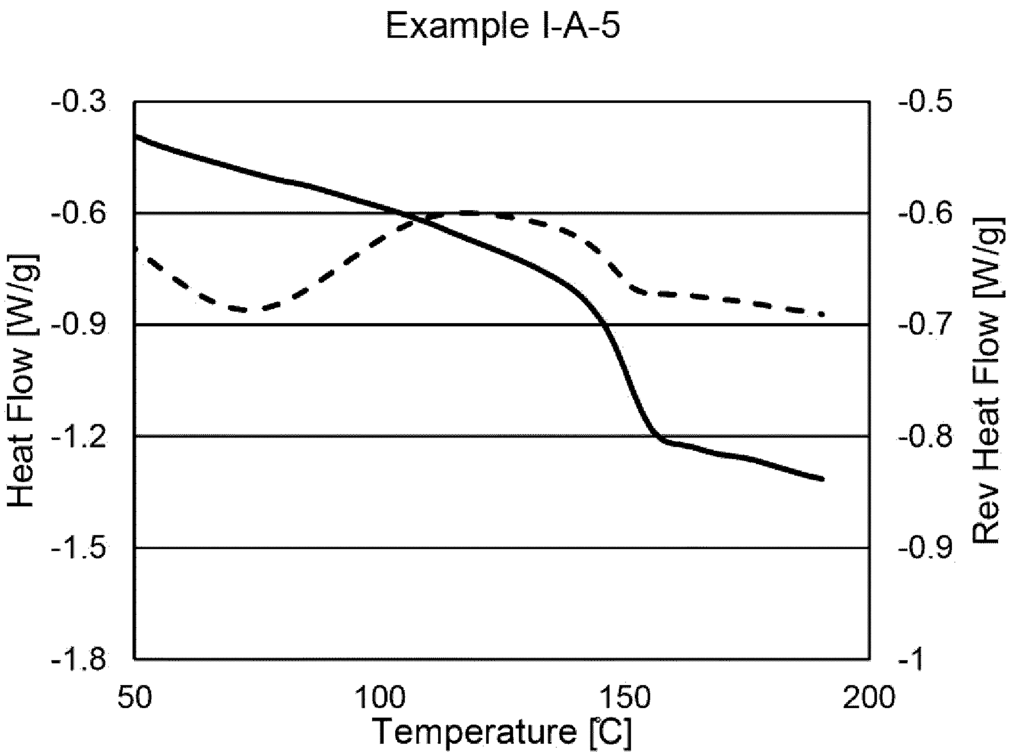
Example I-A-5
-0.3
-0.6
-0.9
-1.2
-1.5
-1.8
Heat Flow [W/g]
-0.5
-0.6
-0.7
-0.8
-0.9
-1
Rev Heat Flow [W/g]
50
100
150
200
Temperature [℃]

# Fig.13

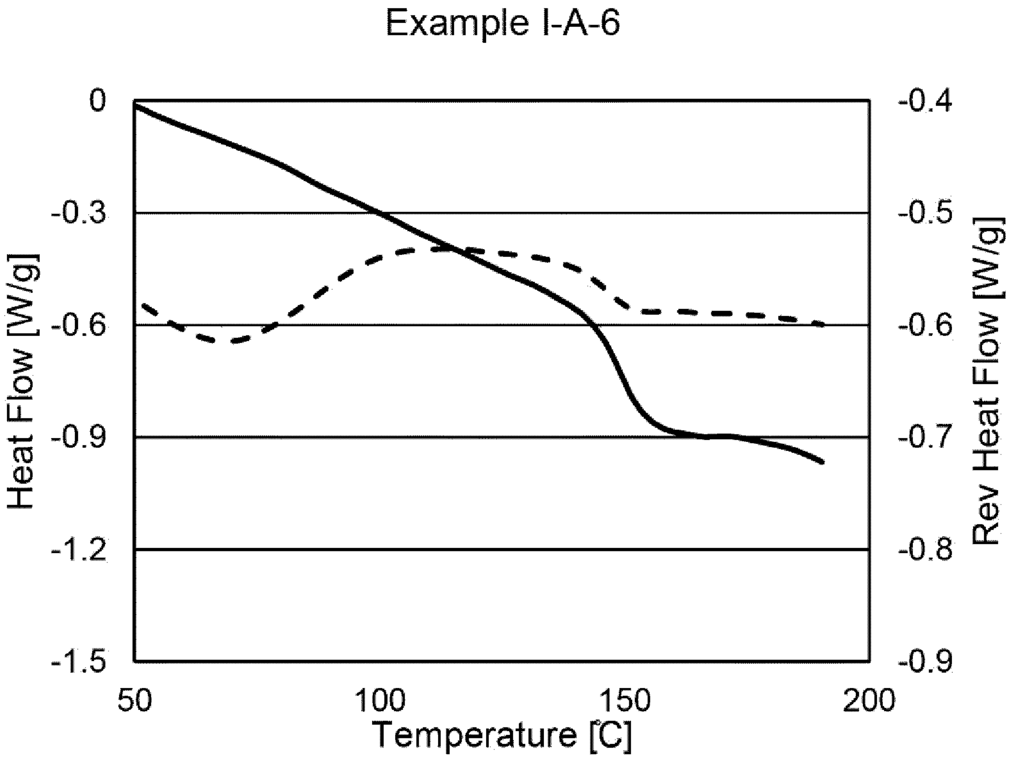
Example I-A-6
0
-0.3
-0.6
-0.9
-1.2
-1.5
Heat Flow [W/g]
-0.4
-0.5
-0.6
-0.7
-0.8
-0.9
Rev Heat Flow [W/g]
50
100
150
200
Temperature [℃]

# Fig.14

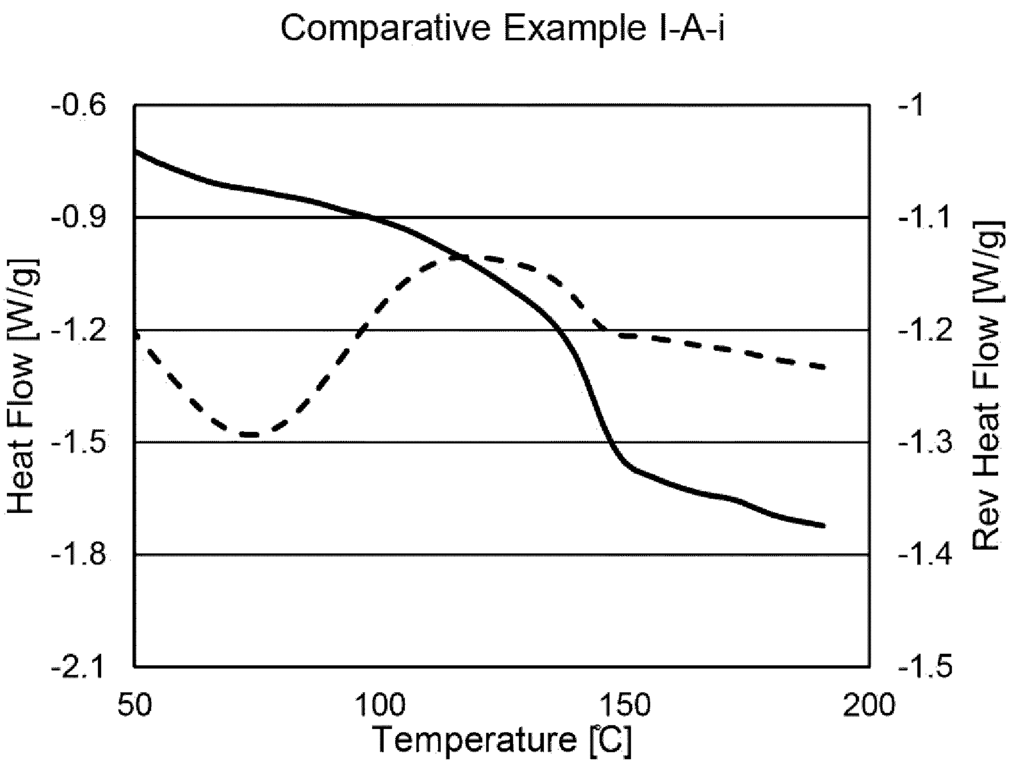
Comparative Example I-A-i
-0.6
-0.9
-1.2
-1.5
-1.8
-2.1
Heat Flow [W/g]
-1
-1.1
-1.2
-1.3
-1.4
-1.5
Rev Heat Flow [W/g]
50
100
150
200
Temperature [℃]

*Fig.15*

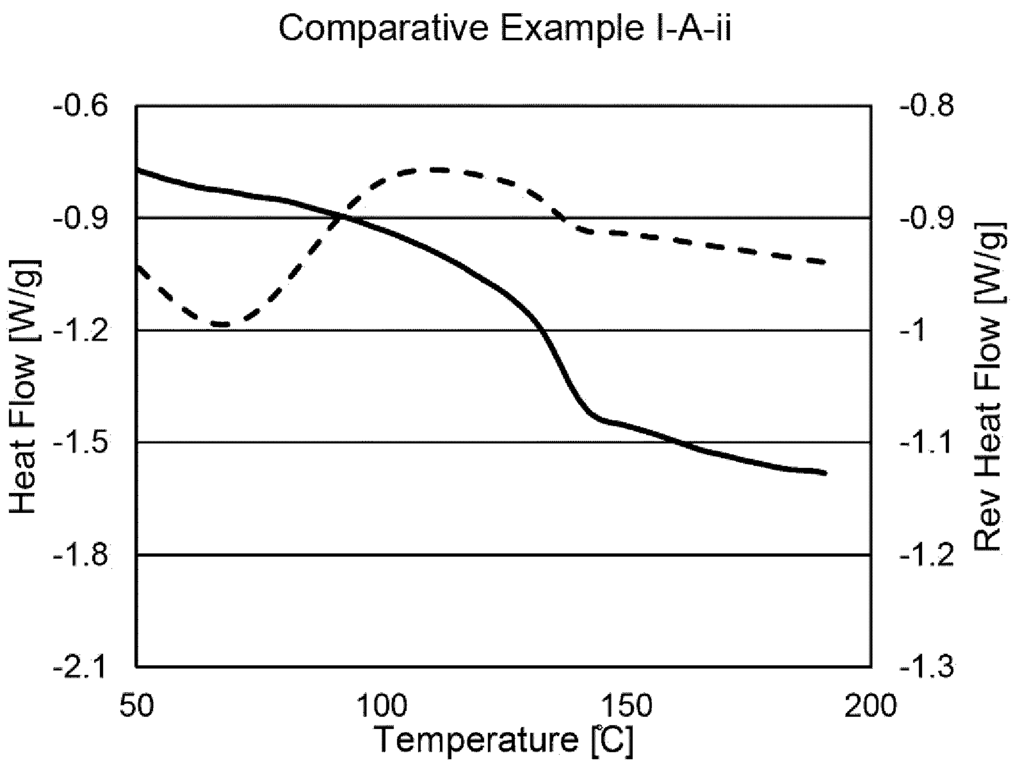
Comparative Example I-A-ii
-0.6
-0.9
-1.2
-1.5
-1.8
-2.1
Heat Flow [W/g]
-0.8
-0.9
-1
-1.1
-1.2
-1.3
Rev Heat Flow [W/g]
50
100
150
200
Temperature [℃]

# Fig.16

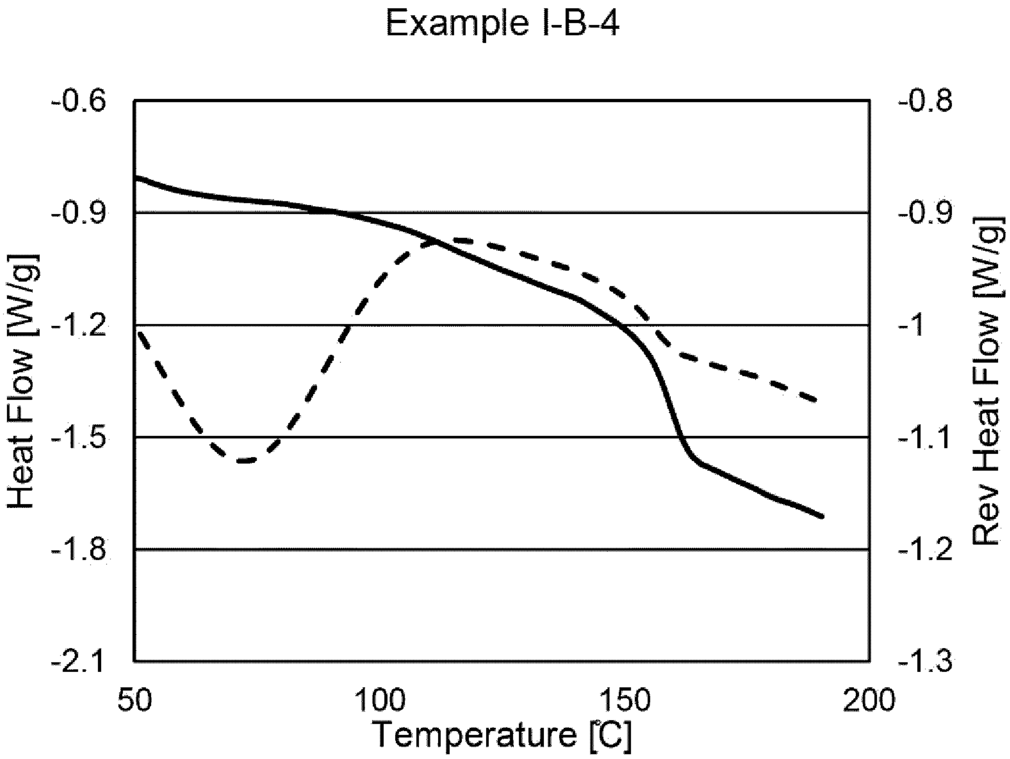
Example I-B-4
Heat Flow [W/g]
Rev Heat Flow [W/g]
Temperature [°C]
-0.6
-0.9
-1.2
-1.5
-1.8
-2.1
-0.8
-0.9
-1
-1.1
-1.2
-1.3
50
100
150
200

# Fig.17

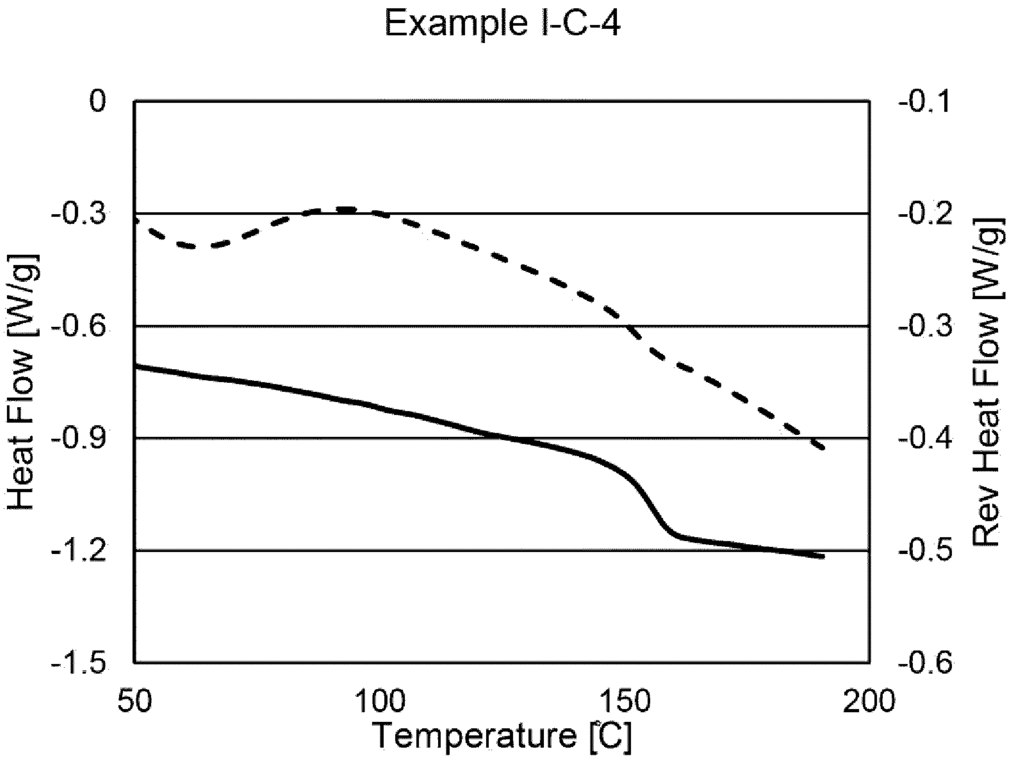
Example I-C-4
0
-0.3
-0.6
-0.9
-1.2
-1.5
Heat Flow [W/g]
-0.1
-0.2
-0.3
-0.4
-0.5
-0.6
Rev Heat Flow [W/g]
50
100
150
200
Temperature [℃]

# Fig.18

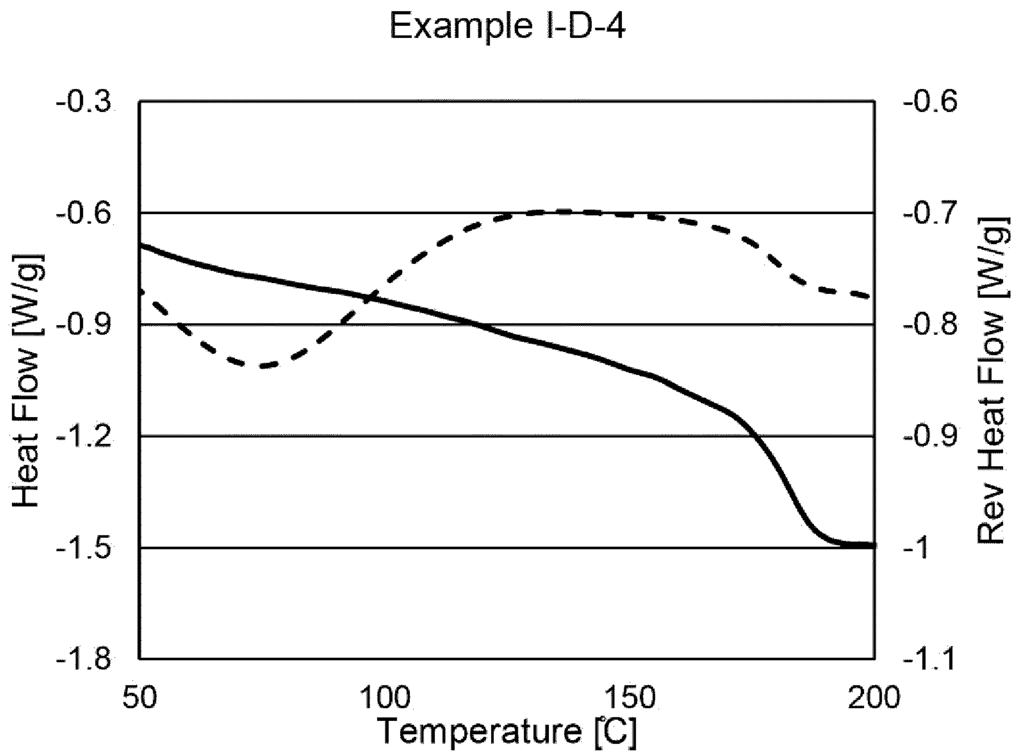
Example I-D-4
-0.3
-0.6
-0.9
-1.2
-1.5
-1.8
Heat Flow [W/g]
-0.6
-0.7
-0.8
-0.9
-1
-1.1
Rev Heat Flow [W/g]
50
100
150
200
Temperature [℃]

*Fig.19*

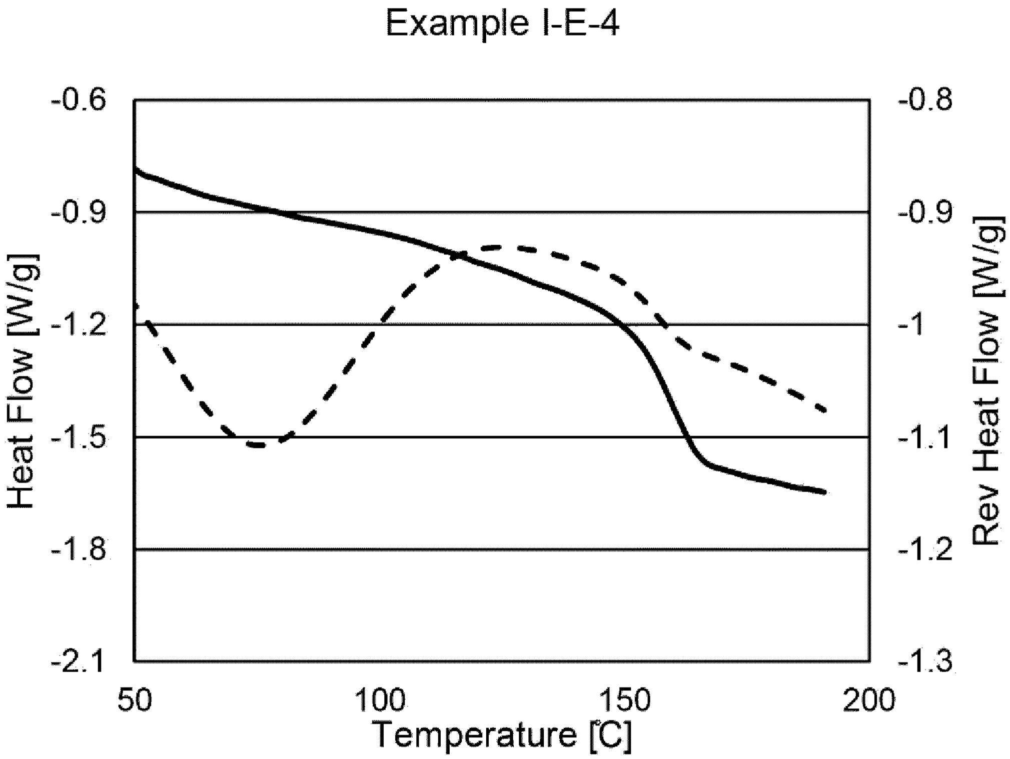
Example I-E-4
-0.6
-0.9
-1.2
-1.5
-1.8
-2.1
Heat Flow [W/g]
-0.8
-0.9
-1
-1.1
-1.2
-1.3
Rev Heat Flow [W/g]
50
100
150
200
Temperature [℃]

*Fig.20*

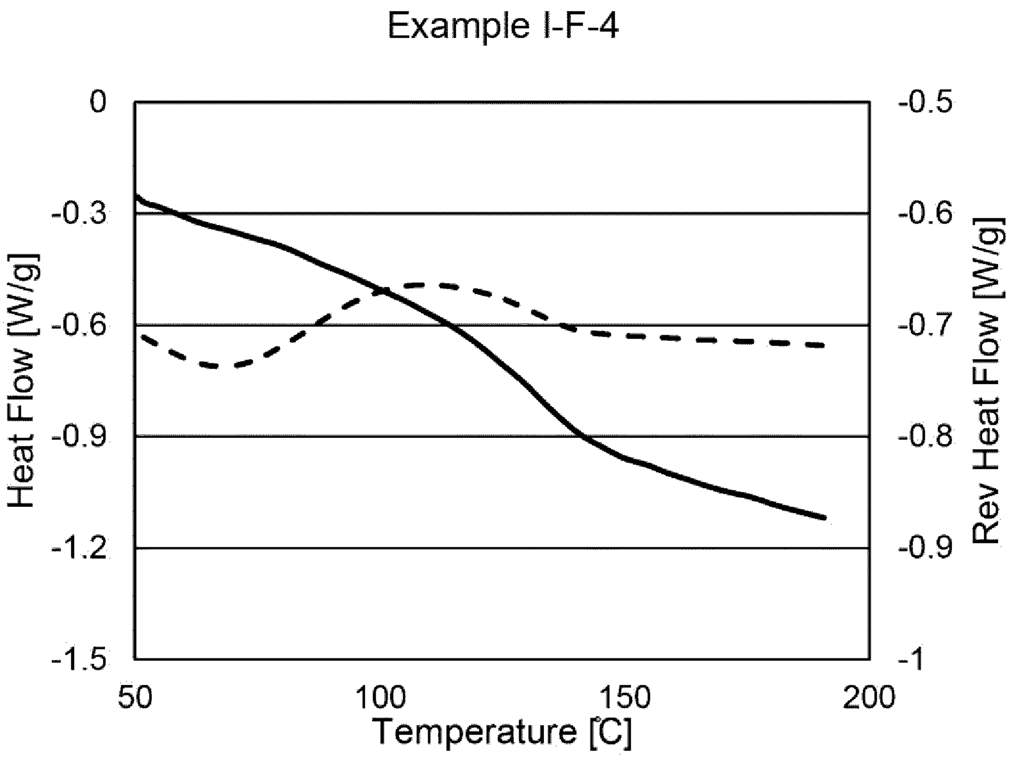
Example I-F-4
0
-0.3
-0.6
-0.9
-1.2
-1.5
Heat Flow [W/g]
-0.5
-0.6
-0.7
-0.8
-0.9
-1
Rev Heat Flow [W/g]
50
100
150
200
Temperature [℃]

*Fig.21*

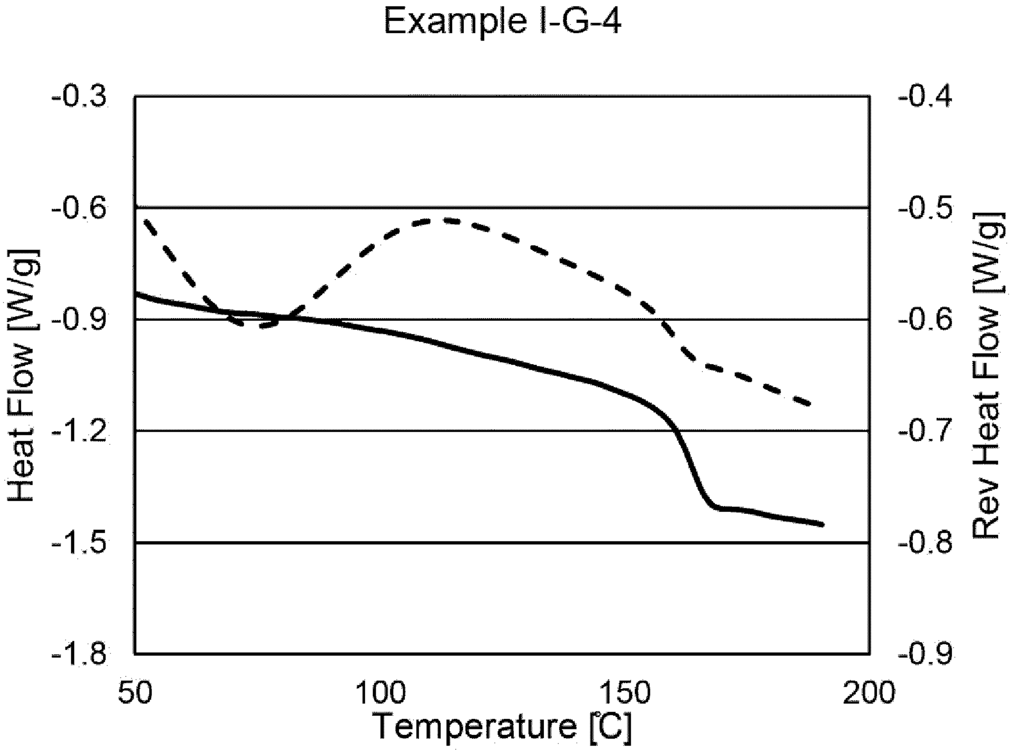
Example I-G-4
-0.3
-0.6
-0.9
-1.2
-1.5
-1.8
Heat Flow [W/g]
-0.4
-0.5
-0.6
-0.7
-0.8
-0.9
Rev Heat Flow [W/g]
50
100
150
200
Temperature [℃]

Fig.22

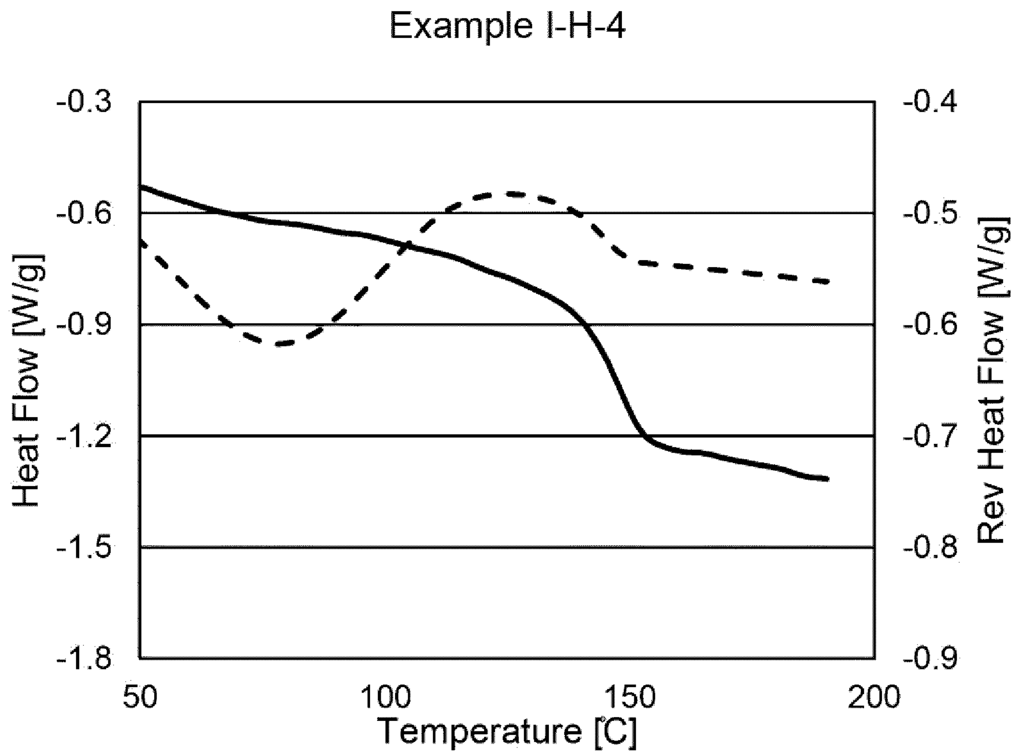
Example I-H-4
-0.3
-0.6
-0.9
-1.2
-1.5
-1.8
Heat Flow [W/g]
-0.4
-0.5
-0.6
-0.7
-0.8
-0.9
Rev Heat Flow [W/g]
50
100
150
200
Temperature [℃]

*Fig.23*

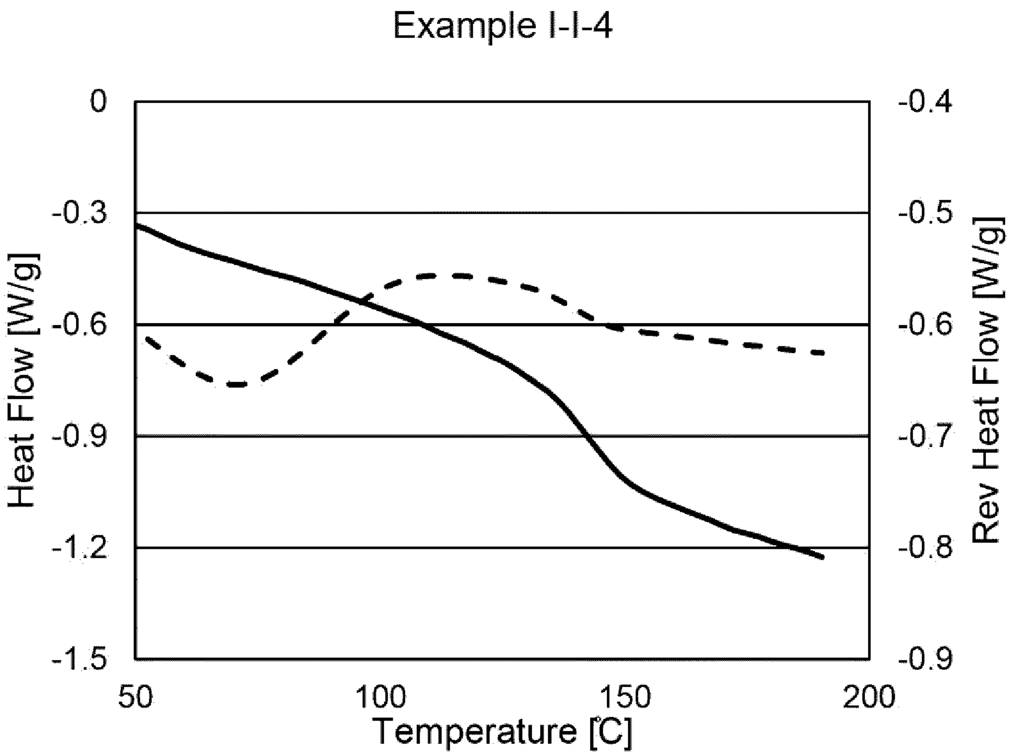
Example I-I-4
0
-0.3
-0.6
-0.9
-1.2
-1.5
Heat Flow [W/g]
-0.4
-0.5
-0.6
-0.7
-0.8
-0.9
Rev Heat Flow [W/g]
50
100
150
200
Temperature [℃]

# Fig.24

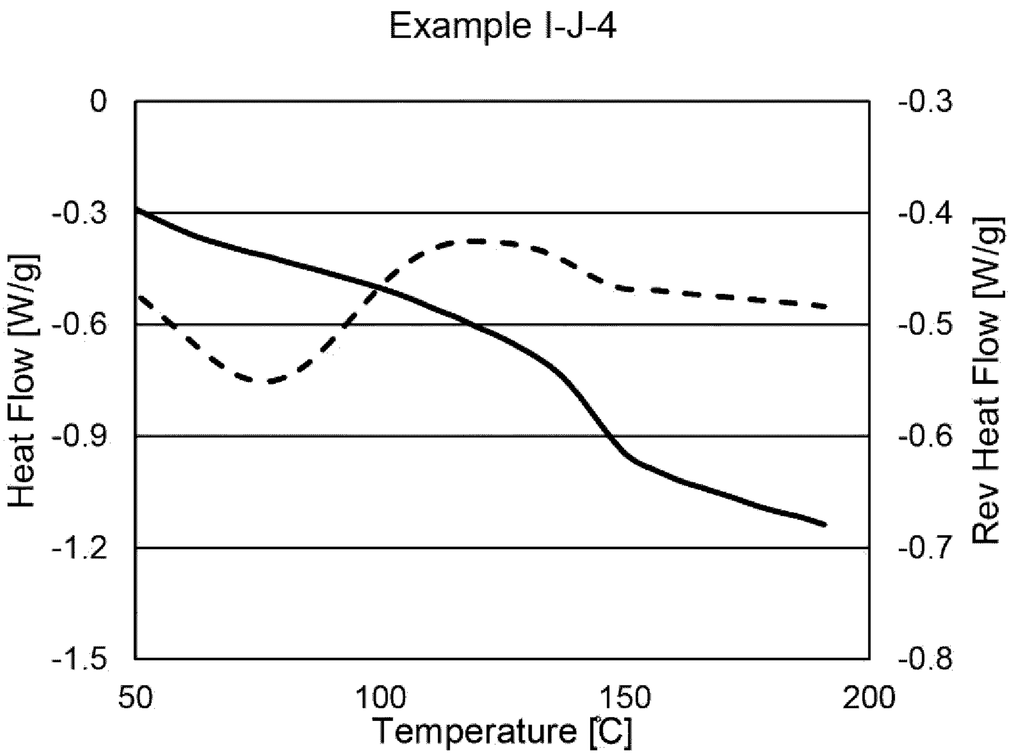
Example I-J-4
0
-0.3
-0.6
-0.9
-1.2
-1.5
Heat Flow [W/g]
-0.3
-0.4
-0.5
-0.6
-0.7
-0.8
Rev Heat Flow [W/g]
50
100
150
200
Temperature [℃]

**Fig.25**

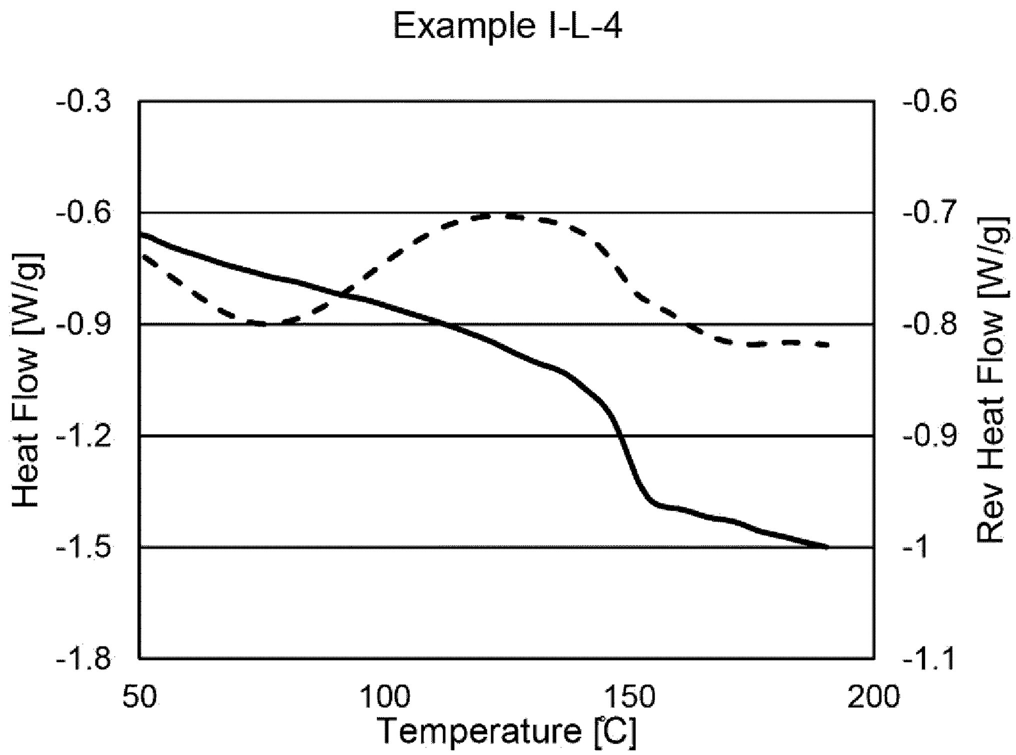
Example I-L-4
-0.3
-0.6
-0.9
-1.2
-1.5
-1.8
Heat Flow [W/g]
-0.6
-0.7
-0.8
-0.9
-1
-1.1
Rev Heat Flow [W/g]
50
100
150
200
Temperature [℃]

*Fig.26*

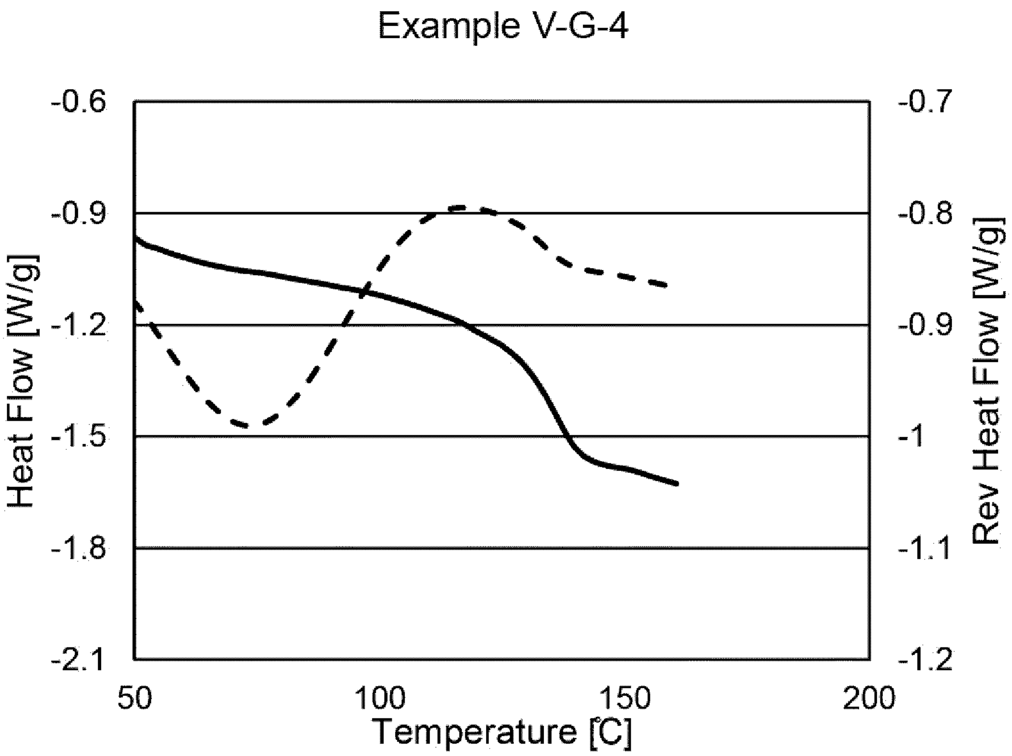
Example V-G-4
-0.6
-0.9
-1.2
-1.5
-1.8
-2.1
Heat Flow [W/g]
-0.7
-0.8
-0.9
-1
-1.1
-1.2
Rev Heat Flow [W/g]
50
100
150
200
Temperature [℃]

***Fig.27***

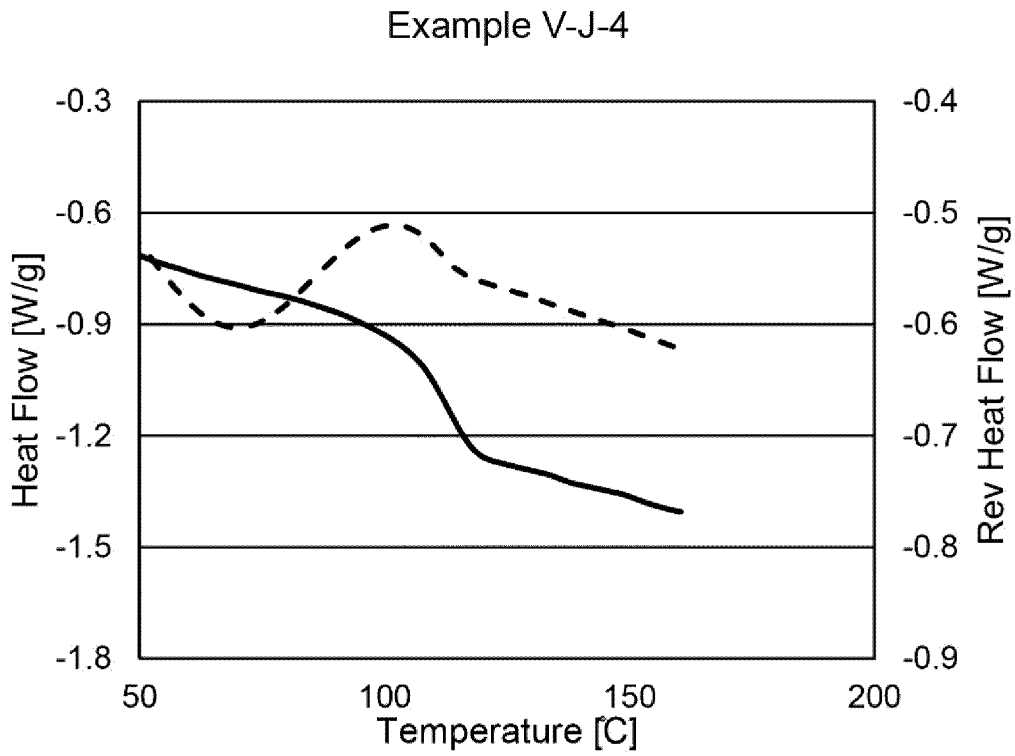
Example V-J-4
-0.3
-0.6
-0.9
-1.2
-1.5
-1.8
Heat Flow [W/g]
-0.4
-0.5
-0.6
-0.7
-0.8
-0.9
Rev Heat Flow [W/g]
50
100
150
200
Temperature [℃]

# Fig.28

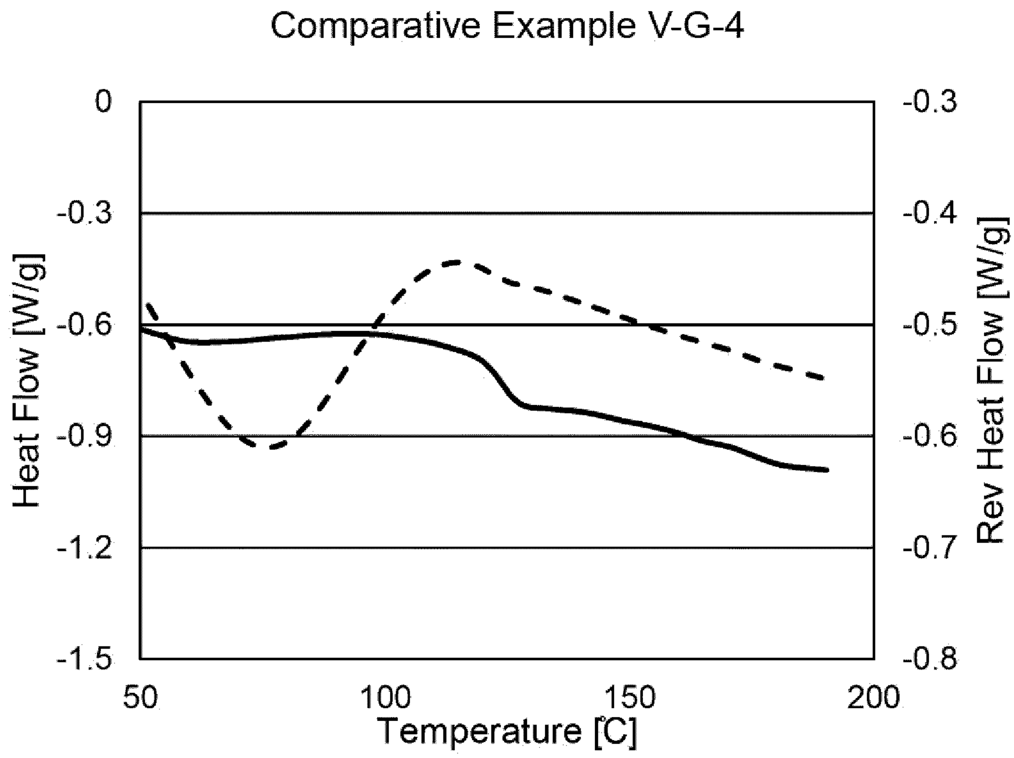
Comparative Example V-G-4
Heat Flow [W/g]
0
-0.3
-0.6
-0.9
-1.2
-1.5
Rev Heat Flow [W/g]
-0.3
-0.4
-0.5
-0.6
-0.7
-0.8
50
100
150
200
Temperature [℃]

*Fig.29*

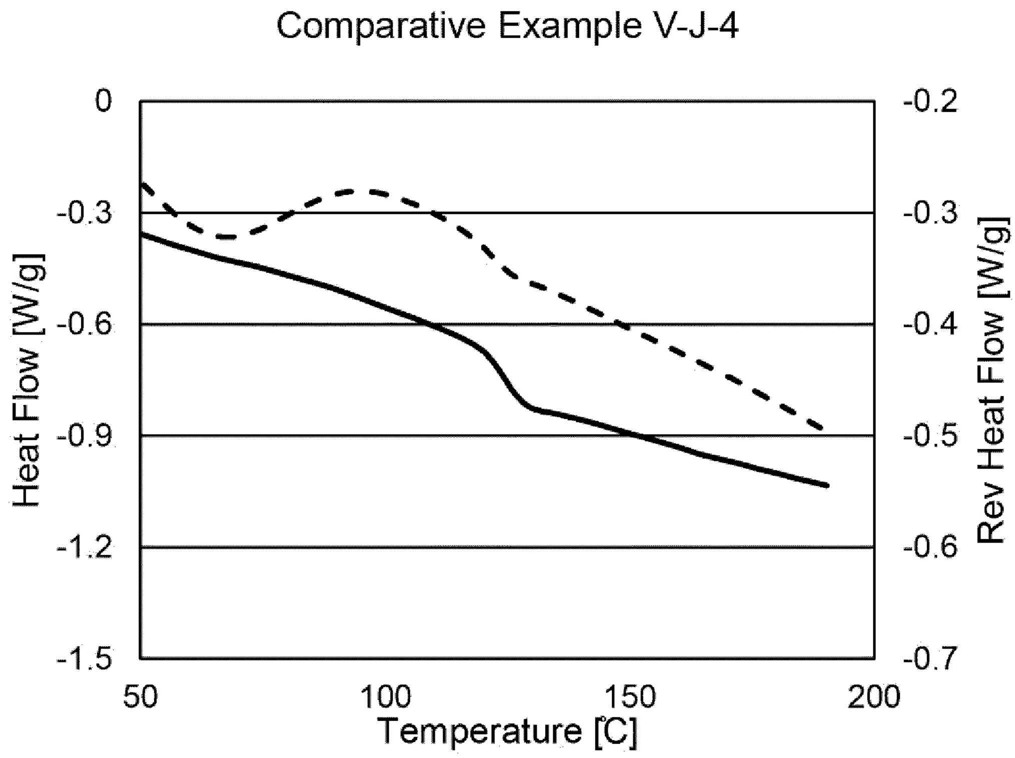
Comparative Example V-J-4
0
-0.3
-0.6
-0.9
-1.2
-1.5
Heat Flow [W/g]
-0.2
-0.3
-0.4
-0.5
-0.6
-0.7
Rev Heat Flow [W/g]
50
100
150
200
Temperature [℃]

# Fig.30

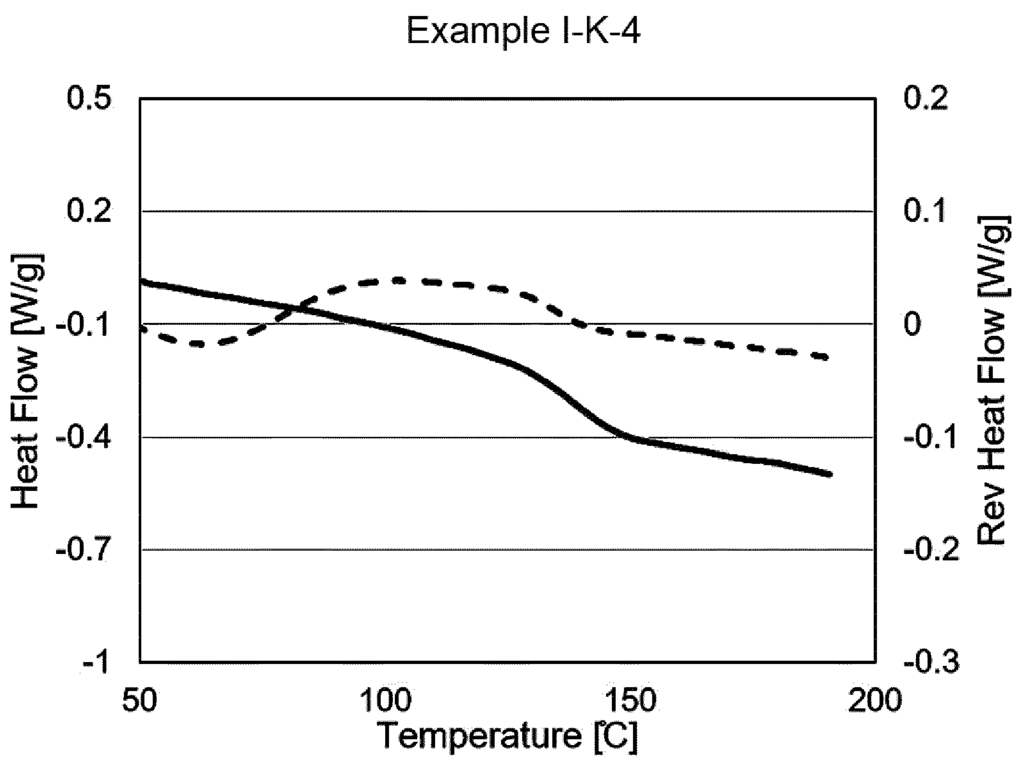
Example I-K-4
0.5
0.2
-0.1
-0.4
-0.7
-1
Heat Flow [W/g]
0.2
0.1
0
-0.1
-0.2
-0.3
Rev Heat Flow [W/g]
50
100
150
200
Temperature [̊C]

# Fig.31

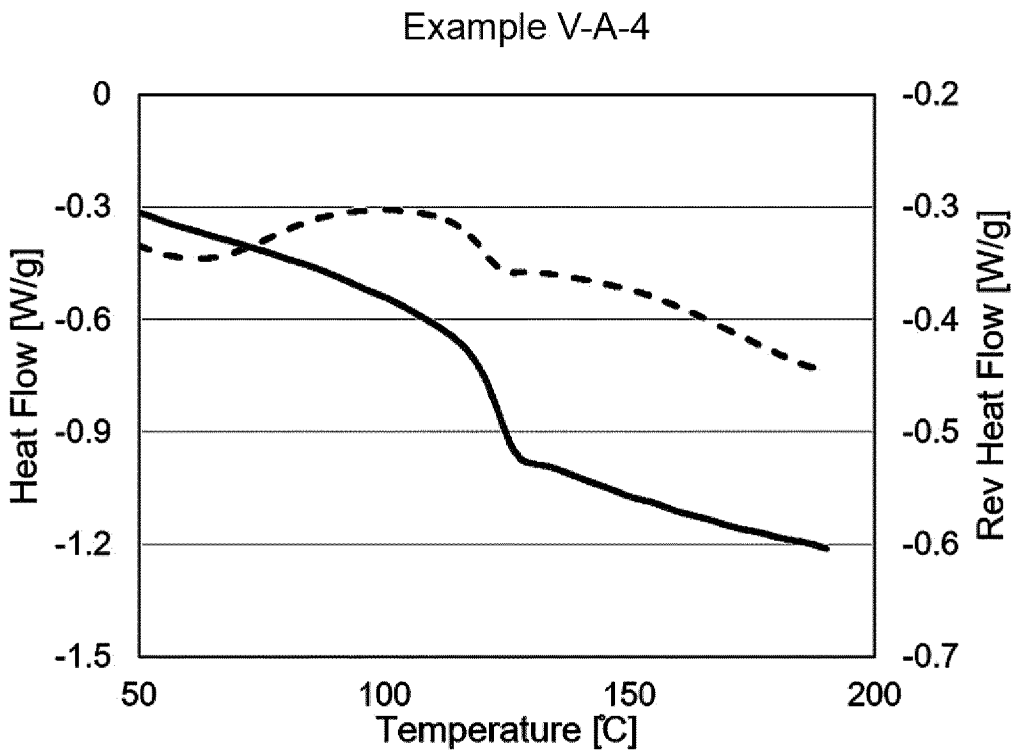
Example V-A-4
Heat Flow [W/g]
Rev Heat Flow [W/g]
Temperature [°C]
0
-0.3
-0.6
-0.9
-1.2
-1.5
-0.2
-0.3
-0.4
-0.5
-0.6
-0.7
50
100
150
200

*Fig.32*

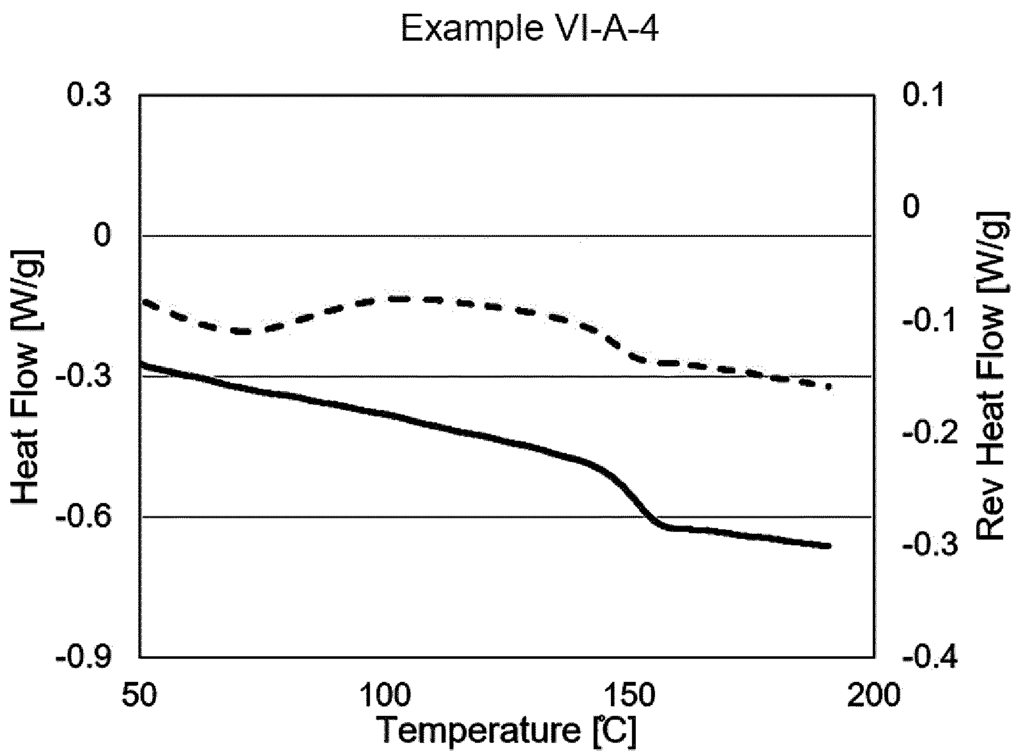
Example VI-A-4
0.3
0
-0.3
-0.6
-0.9
Heat Flow [W/g]
0.1
0
-0.1
-0.2
-0.3
-0.4
Rev Heat Flow [W/g]
50
100
150
200
Temperature [℃]

**INTERNATIONAL SEARCH REPORT**

International application No. **PCT/JP2023/047077**

**A. CLASSIFICATION OF SUBJECT MATTER**

***C08L 101/00***(2006.01)i; ***A61K 9/16***(2006.01)i; ***A61K 38/08***(2019.01)i; ***A61K 38/10***(2006.01)i; ***A61K 38/12***(2006.01)i; ***A61K 38/16***(2006.01)i; ***C08L 89/00***(2006.01)i
FI: C08L101/00; A61K9/16; A61K38/08; A61K38/10; A61K38/16; A61K38/12; C08L89/00

According to International Patent Classification (IPC) or to both national classification and IPC

**B. FIELDS SEARCHED**

Minimum documentation searched (classification system followed by classification symbols)

C08L101/00; A61K9/16; A61K38/08; A61K38/10; A61K38/12; A61K38/16; C08L89/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2016-525123 A (MANNKIND CORPORATION) 22 August 2016 (2016-08-22) table 2, sample 13, etc. | 1-2, 4-15 |
| X | JP 2002-537321 A (ELAN CORPORATION, PLC) 05 November 2002 (2002-11-05) example 6, etc. | 1-5, 9-14 |
| A | JP 2021-523931 A (PALATIN TECHNOLOGIES, INC.) 09 September 2021 (2021-09-09) | 1-15 |
| A | JP 2007-503380 A (BOEHRINGER INGELHEIM PHARMA GMBH & CO. KG) 22 February 2007 (2007-02-22) | 1-15 |
| A | JP 2016-049105 A (KAO CORPORATION) 11 April 2016 (2016-04-11) | 1-15 |
| A | JP 2006-502972 A (FERRING BV) 26 January 2006 (2006-01-26) | 1-15 |
| A | JP 2017-504590 A (IRONWOOD PHARMACEUTICALS, INC.) 09 February 2017 (2017-02-09) | 1-15 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **06 February 2024** | **27 February 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**

International application No.
**PCT/JP2023/047077**

**C. DOCUMENTS CONSIDERED TO BE RELEVANT**

| Category* | Citation of document, with indication, where appropriate, of the relevant passages | Relevant to claim No. |
|---|---|---|
| A | WO 2021/090855 A1 (CHUGAI SEIYAKU KABUSHIKI KAISHA) 14 May 2021 (2021-05-14) | 1-15 |

Form PCT/ISA/210 (second sheet) (July 2022)

**INTERNATIONAL SEARCH REPORT**
**Information on patent family members**

International application No.
**PCT/JP2023/047077**

| Patent document cited in search report | Publication date (day/month/year) | Patent family member(s) | Publication date (day/month/year) |
|---|---|---|---|
| JP 2016-525123 A | 22 August 2016 | US 2016/0158156 A1<br>table 2, sample 13, etc.<br>US 2018/0221280 A1<br>US 2019/0358163 A1<br>US 2021/0244667 A1<br>EP 3021834 A1<br>CN 105451716 A<br>KR 10-2016-0032139 A<br>KR 10-2021-0134805 A<br>CN 114848614 A | |
| JP 2002-537321 A | 05 November 2002 | US 2003/0091623 A1<br>example 6, etc.<br>US 2007/0148228 A1<br>US 2007/0196464 A1<br>US 2008/0275001 A1<br>US 2010/0028421 A1<br>US 2010/0209499 A1<br>US 2013/0089604 A1<br>EP 1154761 A1 | |
| JP 2021-523931 A | 09 September 2021 | US 2022/0088146 A1<br>EP 3768245 A1<br>CN 112188888 A<br>KR 10-2021-0003759 A | |
| JP 2007-503380 A | 22 February 2007 | US 2007/0298116 A1<br>EP 1658047 A1<br>KR 10-2007-0054590 A | |
| JP 2016-049105 A | 11 April 2016 | US 2017/0273999 A1<br>CN 106793821 A | |
| JP 2006-502972 A | 26 January 2006 | US 2004/0138098 A1<br>US 2005/0232997 A1<br>US 2007/0265207 A1<br>US 2008/0274951 A1<br>US 2009/0005432 A1<br>US 2009/0318665 A1<br>US 2010/0056436 A1<br>US 2012/0071538 A1<br>US 2012/0322734 A1<br>US 2015/0150938 A1<br>US 2016/0175246 A1<br>US 2017/0112895 A1<br>US 2018/0280469 A1<br>US 2021/0161810 A1<br>CN 1649615 A<br>CN 1981745 A<br>KR 10-0632455 B1 | |
| JP 2017-504590 A | 09 February 2017 | US 2016/0310559 A1<br>US 2016/0310560 A1<br>US 2017/0157200 A1<br>US 2018/0015139 A1<br>US 2020/0038476 A1 | |

Form PCT/ISA/210 (patent family annex) (July 2022)

**INTERNATIONAL SEARCH REPORT**
**Information on patent family members**

International application No.
**PCT/JP2023/047077**

| Patent document cited in search report | Publication date (day/month/year) | Patent family member(s) | Publication date (day/month/year) |
|---|---|---|---|
| | | US 2022/0031802 A1 | |
| | | US 2023/0142270 A1 | |
| | | EP 3079669 A1 | |
| | | EP 3821881 A1 | |
| | | KR 10-2016-0132001 A | |
| | | CN 106659687 A | |
| | | CN 112569199 A | |
| | | KR 10-2021-0152588 A | |
| WO 2021/090855 A1 | 14 May 2021 | US 2023/0151060 A1 | |
| | | EP 4043478 A1 | |
| | | KR 10-2021-0064234 A | |
| | | KR 10-2022-0081327 A | |
| | | CN 114729006 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010126030 A
- WO 2014092061 A
- WO 2015189901 A
- WO 2008047201 A
- US 10004719 B
- US 20030054038
- US 20030185893
- WO 2021090855 A


### Non-patent literature cited in the description


- *International Journal of Pharmaceutics*, 2010, vol. 399, 94-101
- Perry's Chemical Engineers' Handbook. 1984, 20-54, 20-57
- **MARSHALL**. Atomization and Spray-Drying. *50 Chem. Eng. Prog. Monogr*, 1954
- **MASTERS**. Spray-Drying Handbook. 1985